# EUROPEAN PATENT APPLICATION

(11) **EP 4 692 118 A1**
(43) Date of publication of application: **11.02.2026**
(21) Application number: 24777900.2
(22) Date of filing: 22.03.2024
(51) Int. Cl.: C07K 16/28, C07K 16/40, C07K 16/30, A61P 35/00, A61K 47/68

(54) **ADAM9-TARGETING HUMANIZED ANTIBODY AND ANTIBODY-DRUG CONJUGATE COMPRISING SAME AND USE THEREOF**

(30) Priority: 24.03.2023 CN 202310302436; 07.03.2024 CN 202410263320
(71) Applicant: Duality Biologics (Shanghai) Co., Ltd., Shanghai 201204 (CN)
(72) Inventor: LIN, Shengchao, Suzhou, Jiangsu 215000 (CN); HUA, Haiqing, Suzhou, Jiangsu 215000 (CN); ZHU, Zhongyuan, Suzhou, Jiangsu 215000 (CN)
(74) Representative: Greaves Brewster LLP
(86) International application number: PCT/CN2024/083218
(87) International publication number: WO 2024/199111

(57) **Abstract**

An ADAM9-targeting humanized antibody and an antibody-drug conjugate comprising same and a use thereof. The ADAM9-targeting humanized antibody comprises a heavy chain variable region and a light chain variable region, the heavy chain variable region comprises heavy chain complementarity determining regions HCDR1, HCDR2 and HCDR3, and the light chain variable region comprises light chain complementarity determining regions LCDR1, LCDR2 and LCDR3. The antibody-drug conjugate comprises the humanized antibody, a linker unit L and a cytotoxic drug. The humanized antibody has good affinity, hydrophilicity and solubility and high stability, and has a high internalization rate for cells expressing ADAM9. The antibody-drug conjugate has a good proliferation inhibition effect on cells having positive expression of ADAM9, and can show a remarkable anti-tumor drug effect in vivo.

## Description

The present application claims priority to Chinese patent application 2023103024365 filed on March 24, 2023 and Chinese patent application 202410263320X filed on March 7, 2024. The contents of the Chinese patent applications are incorporated herein by reference in their entirety.

### TECHNICAL FIELD

The present disclosure belongs to the field of biomedical technology, and specifically relates to an ADAM9-targeting humanized antibody, an antibody-drug conjugate comprising the same, and a use thereof.

### BACKGROUND

ADAM (A disintegrin and metalloproteinase domain) is a family of proteins involved in various physiological and pathological processes. At least 40 gene members of the family have been identified, and at least 21 of these members are considered to be functional in humans. ADAM family members have a well-conserved structure with eight domains, among which are a metalloproteinase domain and an integrin-binding (disintegrin) domain. The ADAM metalloproteinase domain acts as a sheddase and has been reported to modulate a series of biological processes by cleaving transmembrane proteins, which then can act as soluble ligands and regulate cellular signaling.

ADAM9 is a member of the ADAM family of molecules. It is synthesized as an inactive form, which is proteolytically cleaved to produce an active enzyme. Processing at the upstream site is particularly important for activation of the proenzyme. ADAM9 is expressed in fibroblasts, activated vascular smooth muscle cells, monocytes, and activated macrophages.

The metalloproteinase activity of ADAM9 is involved in the degradation of matrix components, thereby enabling migration of tumor cells. The disintegrin domain of ADAM9, which is highly homologous to many snake venom disintegrins, allows the interaction between ADAM9 and integrins, and enables ADAM9 to modulate, either positively or negatively, cell adhesion events. The disintegrin domain of ADAM9 has been shown to interact with α6β1, α6β4, αvβ5, and α9β1 integrins.

The expression of ADAM9 has been found to be associated with diseases, especially cancer. ADAM9 has been found to cleave and release a number of molecules that play important roles in tumorigenesis and angiogenesis, such as TEK, KDR, EPHB4, CD40, VCAM1, and CDH5. ADAM9 is expressed by many types of tumor cells, including those of breast cancer, colon cancer, gastric cancer, glioma, liver cancer, non-small cell lung cancer, melanoma, myeloma, pancreatic cancer, and prostate cancer.

Significantly, increased ADAM9 expression has been found to be positively correlated with tumor malignancy and metastatic potential. Additionally, ADAM9 and its secreted soluble isoforms seem to be critical for the dissemination of cancer cells. Consequently, a number of studies have identified ADAM9 as a potential target for anticancer therapy. Patent WO2018119196A1 disclosed that based on the murine anti-human ADAM9 monoclonal antibody "MAB-A" (whose heavy chain variable region comprising the amino acid sequence shown in SEQ ID NO: 7 and whose light chain variable region comprising the amino acid sequence shown in SEQ ID NO: 11 in the patent), the VH and VL domains of MAB-A were humanized, and the CDRs were optimized to improve affinity and/or to remove potential amino acid liabilities. However, in the patent, the humanized version of MAB-A, hMAB-A (2I.2) (whose heavy chain comprising the amino acid sequence shown in SEQ ID NO: 52 and whose light chain comprising the amino acid sequence shown in SEQ ID NO: 68 in the patent), which entered clinical research, exhibited issues with high hydrophobicity.

Therefore, there remains a need to develop humanized anti-ADAM9 antibodies with lower hydrophobicity, higher solubility, and better conjugation yield and druggability as ADC (antibody-drug conjugate) drugs.

### SUMMARY

To overcome the technical problems in the prior art that humanized anti-ADAM9 antibodies exhibit high hydrophobicity, low solubility, and poor conjugation yield and druggability as ADC drugs, the present disclosure provides an ADAM9-targeting humanized antibody, an antibody-drug conjugate comprising the same, and a use thereof by re-humanizing the murine monoclonal antibody MAB-A disclosed in patent WO2018119196A1 to improve the solubility and reduce the hydrophobicity of the humanized version, thereby enhancing the conjugation yield and druggability as an ADC drug.

The present disclosure addresses the above technical problems through the following technical solutions.

A first aspect of the present disclosure provides an ADAM9-targeting antibody or an antigen-binding fragment thereof, wherein the antibody or the antigen-binding fragment thereof comprises a heavy chain variable region and a light chain variable region, wherein the heavy chain variable region comprises heavy chain complementarity-determining regions HCDR1, HCDR2, and HCDR3, and the light chain variable region comprises light chain complementarity-determining regions LCDR1, LCDR2, and LCDR3;
the HCDR1 comprises the amino acid sequence shown in LYWMX₁, the HCDR2 comprises the amino acid sequence shown in X₂IIPIFGHTX₃YX₄EKFX₃X₆, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19;
wherein X₁ is N, D, H, or E; X₂ is R or D; X₃ is D or K; X₄ is N or E; X₃ is K or R; X₆ is D or N.

In some embodiments of the present disclosure, in the HCDR1, X₁ is N or H.

In some embodiments of the present disclosure, in the HCDR2, X₂ is R; X₃ is K; X₄ is N; X₃ is K; X₆ is D or N.

In some specific embodiments of the present disclosure:
when X₁ is N, X₂ is R; X₃ is K; X₄ is N; X₃ is K; X₆ is D;
when X₁ is H, X₂ is R; X₃ is K; X₄ is N; X₃ is K; X₆ is N.

In some embodiments of the present disclosure, the HCDR1 comprises the amino acid sequence shown in SEQ ID NO: 3, the HCDR2 comprises the amino acid sequence shown in SEQ ID NO: 8, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19.

In some other embodiments of the present disclosure, the HCDR1 comprises the amino acid sequence shown in SEQ ID NO: 1, the HCDR2 comprises the amino acid sequence shown in SEQ ID NO: 7, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19.

In some other embodiments of the present disclosure, the HCDR1 comprises the amino acid sequence shown in SEQ ID NO: 1, the HCDR2 comprises the amino acid sequence shown in SEQ ID NO: 5, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19.

In some other embodiments of the present disclosure, the HCDR1 comprises the amino acid sequence shown in SEQ ID NO: 2, the HCDR2 comprises the amino acid sequence shown in SEQ ID NO: 6, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19.

In some other embodiments of the present disclosure, the HCDR1 comprises the amino acid sequence shown in SEQ ID NO: 3, the HCDR2 comprises the amino acid sequence shown in SEQ ID NO: 7, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19.

In some other embodiments of the present disclosure, the HCDR1 comprises the amino acid sequence shown in SEQ ID NO: 2, the HCDR2 comprises the amino acid sequence shown in SEQ ID NO: 9, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19.

In some other embodiments of the present disclosure, the HCDR1 comprises the amino acid sequence shown in SEQ ID NO: 4, the HCDR2 comprises the amino acid sequence shown in SEQ ID NO: 10, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19.

In some specific embodiments of the present disclosure, the amino acid sequence of the HCDR1 is shown in SEQ ID NO: 3, the amino acid sequence of the HCDR2 is shown in SEQ ID NO: 8, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19.

In some other specific embodiments of the present disclosure, the amino acid sequence of the HCDR1 is shown in SEQ ID NO: 1, the amino acid sequence of the HCDR2 is shown in SEQ ID NO: 5, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19.

In some other specific embodiments of the present disclosure, the amino acid sequence of the HCDR1 is shown in SEQ ID NO: 2, the amino acid sequence of the HCDR2 is shown in SEQ ID NO: 6, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19.

In some other specific embodiments of the present disclosure, the amino acid sequence of the HCDR1 is shown in SEQ ID NO: 3, the amino acid sequence of the HCDR2 is shown in SEQ ID NO: 7, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19.

In some other specific embodiments of the present disclosure, the amino acid sequence of the HCDR1 is shown in SEQ ID NO: 1, the amino acid sequence of the HCDR2 is shown in SEQ ID NO: 7, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19.

In some other specific embodiments of the present disclosure, the amino acid sequence of the HCDR1 is shown in SEQ ID NO: 2, the amino acid sequence of the HCDR2 is shown in SEQ ID NO: 9, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19.

In some other specific embodiments of the present disclosure, the amino acid sequence of the HCDR1 is shown in SEQ ID NO: 4, the amino acid sequence of the HCDR2 is shown in SEQ ID NO: 10, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19.

In the present disclosure, the heavy chain variable region and/or the light chain variable region further comprises a framework region, wherein the framework region is a humanized framework region.

In some embodiments of the present disclosure, the heavy chain variable region comprises an amino acid sequence having at least 90%, at least 95%, or at least 99% sequence identity with SEQ ID NO: 31, and/or the light chain variable region comprises an amino acid sequence having at least 90%, at least 95%, or at least 99% sequence identity with SEQ ID NO: 23.

In some other embodiments of the present disclosure, the heavy chain variable region comprises an amino acid sequence having at least 90%, at least 95%, or at least 99% sequence identity with SEQ ID NO: 42, and/or the light chain variable region comprises an amino acid sequence having at least 90%, at least 95%, or at least 99% sequence identity with SEQ ID NO: 43.

In some other embodiments of the present disclosure, the heavy chain variable region comprises an amino acid sequence having at least 90%, at least 95%, or at least 99% sequence identity with SEQ ID NO: 22, and/or the light chain variable region comprises an amino acid sequence having at least 90%, at least 95%, or at least 99% sequence identity with SEQ ID NO: 23.

In some other embodiments of the present disclosure, the heavy chain variable region comprises an amino acid sequence having at least 90%, at least 95%, or at least 99% sequence identity with SEQ ID NO: 24, and/or the light chain variable region comprises an amino acid sequence having at least 90%, at least 95%, or at least 99% sequence identity with SEQ ID NO: 25.

In some other embodiments of the present disclosure, the heavy chain variable region comprises an amino acid sequence having at least 90%, at least 95%, or at least 99% sequence identity with SEQ ID NO: 26, and/or the light chain variable region comprises an amino acid sequence having at least 90%, at least 95%, or at least 99% sequence identity with SEQ ID NO: 27.

In some other embodiments of the present disclosure, the heavy chain variable region comprises an amino acid sequence having at least 90%, at least 95%, or at least 99% sequence identity with SEQ ID NO: 28, and/or the light chain variable region comprises an amino acid sequence having at least 90%, at least 95%, or at least 99% sequence identity with SEQ ID NO: 30.

In some other embodiments of the present disclosure, the heavy chain variable region comprises an amino acid sequence having at least 90%, at least 95%, or at least 99% sequence identity with SEQ ID NO: 34, and/or the light chain variable region comprises an amino acid sequence having at least 90%, at least 95%, or at least 99% sequence identity with SEQ ID NO: 35.

In some other embodiments of the present disclosure, the heavy chain variable region comprises an amino acid sequence having at least 90%, at least 95%, or at least 99% sequence identity with SEQ ID NO: 36, and/or the light chain variable region comprises an amino acid sequence having at least 90%, at least 95%, or at least 99% sequence identity with SEQ ID NO: 30.

In some other embodiments of the present disclosure, the heavy chain variable region comprises an amino acid sequence having at least 90%, at least 95%, or at least 99% sequence identity with SEQ ID NO: 37, and/or the light chain variable region comprises an amino acid sequence having at least 90%, at least 95%, or at least 99% sequence identity with SEQ ID NO: 38.

In some other embodiments of the present disclosure, the heavy chain variable region comprises an amino acid sequence having at least 90%, at least 95%, or at least 99% sequence identity with SEQ ID NO: 36, and/or the light chain variable region comprises an amino acid sequence having at least 90%, at least 95%, or at least 99% sequence identity with SEQ ID NO: 27.

In some other embodiments of the present disclosure, the heavy chain variable region comprises an amino acid sequence having at least 90%, at least 95%, or at least 99% sequence identity with SEQ ID NO: 39, and/or the light chain variable region comprises an amino acid sequence having at least 90%, at least 95%, or at least 99% sequence identity with SEQ ID NO: 27.

In some other embodiments of the present disclosure, the heavy chain variable region comprises an amino acid sequence having at least 90%, at least 95%, or at least 99% sequence identity with SEQ ID NO: 44, and/or the light chain variable region comprises an amino acid sequence having at least 90%, at least 95%, or at least 99% sequence identity with SEQ ID NO: 45.

In the present disclosure, the variable region comprising an amino acid sequence having at least 90%, at least 95%, or at least 99% sequence identity maintains antigen-binding functionality at least equivalent to that of the original sequence.

In some specific embodiments of the present disclosure, the amino acid sequence of the heavy chain variable region is shown in SEQ ID NO: 31; and/or, the amino acid sequence of the light chain variable region is shown in SEQ ID NO: 23.

In some other specific embodiments of the present disclosure, the amino acid sequence of the heavy chain variable region is shown in SEQ ID NO: 42; and/or, the amino acid sequence of the light chain variable region is shown in SEQ ID NO: 43.

In some other specific embodiments of the present disclosure, the amino acid sequence of the heavy chain variable region is shown in SEQ ID NO: 22; and/or, the amino acid sequence of the light chain variable region is shown in SEQ ID NO: 23.

In some other specific embodiments of the present disclosure, the amino acid sequence of the heavy chain variable region is shown in SEQ ID NO: 24; and/or, the amino acid sequence of the light chain variable region is shown in SEQ ID NO: 25.

In some other specific embodiments of the present disclosure, the amino acid sequence of the heavy chain variable region is shown in SEQ ID NO: 26; and/or, the amino acid sequence of the light chain variable region is shown in SEQ ID NO: 27.

In some other specific embodiments of the present disclosure, the amino acid sequence of the heavy chain variable region is shown in SEQ ID NO: 28; and/or, the amino acid sequence of the light chain variable region is shown in SEQ ID NO: 30.

In some other specific embodiments of the present disclosure, the amino acid sequence of the heavy chain variable region is shown in SEQ ID NO: 34; and/or, the amino acid sequence of the light chain variable region is shown in SEQ ID NO: 35.

In some other specific embodiments of the present disclosure, the amino acid sequence of the heavy chain variable region is shown in SEQ ID NO: 36; and/or, the amino acid sequence of the light chain variable region is shown in SEQ ID NO: 30.

In some other specific embodiments of the present disclosure, the amino acid sequence of the heavy chain variable region is shown in SEQ ID NO: 37; and/or, the amino acid sequence of the light chain variable region is shown in SEQ ID NO: 38.

In some other specific embodiments of the present disclosure, the amino acid sequence of the heavy chain variable region is shown in SEQ ID NO: 36; and/or, the amino acid sequence of the light chain variable region is shown in SEQ ID NO: 27.

In some other specific embodiments of the present disclosure, the amino acid sequence of the heavy chain variable region is shown in SEQ ID NO: 39; and/or, the amino acid sequence of the light chain variable region is shown in SEQ ID NO: 27.

In some other specific embodiments of the present disclosure, the amino acid sequence of the heavy chain variable region is shown in SEQ ID NO: 44; and/or, the amino acid sequence of the light chain variable region is shown in SEQ ID NO: 45.

In the present disclosure, the ADAM9 is preferably human or monkey ADAM9; in some specific embodiments of the present disclosure, the ADAM9 is human ADAM9.

In some embodiments of the present disclosure, the antibody or the antigen-binding fragment thereof is: (1) a full-length antibody, Fab, Fab', F(ab')₂, Fv, sdAb, or scFv; and/or, (2) a monoclonal antibody, bispecific antibody, or multispecific antibody.

In the present disclosure, when the antibody or the antigen-binding fragment thereof is a full-length antibody, it comprises a heavy chain constant region of a heavy chain of a human antibody, preferably a heavy chain constant region of a human antibody IgG1; and/or, it comprises a light chain constant region of a light chain of a human antibody, preferably a light chain constant region of a human antibody κ chain.

In some embodiments of the present disclosure, the amino acid sequence of the heavy chain constant region of the human antibody IgG1 is shown in SEQ ID NO: 48, or has at least 90%, at least 95%, or at least 99% sequence identity with SEQ ID NO: 48; and/or, the amino acid sequence of the light chain constant region of the human antibody κ chain is shown in SEQ ID NO: 49, or has at least 90%, at least 95%, or at least 99% sequence identity with SEQ ID NO: 49.

In some embodiments of the present disclosure, the amino acid sequence of the heavy chain constant region of the human antibody IgG1 is shown in SEQ ID NO: 48, and the amino acid sequence of the light chain constant region of the human antibody κ chain is shown in SEQ ID NO: 49.

In some embodiments of the present disclosure, the heavy chain of the antibody or the antigen-binding fragment thereof comprises an amino acid sequence having at least 90%, at least 95%, or at least 99% sequence identity with SEQ ID NO: 50; and/or, the light chain of the antibody or the antigen-binding fragment thereof comprises an amino acid sequence having at least 90%, at least 95%, or at least 99% sequence identity with SEQ ID NO: 51.

In some embodiments of the present disclosure, the heavy chain of the antibody or the antigen-binding fragment thereof comprises an amino acid sequence having at least 90%, at least 95%, or at least 99% sequence identity with SEQ ID NO: 52; and/or, the light chain of the antibody or the antigen-binding fragment thereof comprises an amino acid sequence having at least 90%, at least 95%, or at least 99% sequence identity with SEQ ID NO: 53.

In the present disclosure, the amino acid sequence having at least 90%, at least 95%, or at least 99% sequence identity maintains antigen-binding functionality at least equivalent to that of the original sequence.

In some specific embodiments of the present disclosure, the amino acid sequence of the heavy chain of the antibody or the antigen-binding fragment thereof is shown in SEQ ID NO: 50; and/or, the amino acid sequence of the light chain of the antibody or the antigen-binding fragment thereof is shown in SEQ ID NO: 51.

In some specific embodiments of the present disclosure, the amino acid sequence of the heavy chain of the antibody or the antigen-binding fragment thereof is shown in SEQ ID NO: 52; and/or, the amino acid sequence of the light chain of the antibody or the antigen-binding fragment thereof is shown in SEQ ID NO: 53.

A second aspect of the present disclosure provides an isolated nucleic acid encoding the antibody or the antigen-binding fragment thereof according to the first aspect.

A third aspect of the present disclosure provides a recombinant expression vector comprising the isolated nucleic acid according to the second aspect.

In some specific embodiments of the present disclosure, the backbone plasmid of the recombinant expression vector is PTT5.

A fourth aspect of the present disclosure provides a transformant comprising the recombinant expression vector according to the third aspect.

In some embodiments of the present disclosure, the host cell of the transformant is a eukaryotic cell.

In some specific embodiments of the present disclosure, the eukaryotic cell is a CHO cell.

A fifth aspect of the present disclosure provides a method for preparing an ADAM9-targeting antibody or an antigen-binding fragment thereof, comprising the steps of culturing the transformant according to the fourth aspect, and isolating the ADAM9-targeting antibody or the antigen-binding fragment thereof from the culture.

A sixth aspect of the present disclosure provides a method for detecting ADAM9, comprising the step of contacting a test sample with the antibody or the antigen-binding fragment thereof according to the first aspect.

In some embodiments of the present disclosure, the detection is for non-diagnostic and/or non-therapeutic purposes.

A seventh aspect of the present disclosure provides an antibody-drug conjugate comprising the antibody or the antigen-binding fragment thereof as described above, a linker unit L, and a cytotoxic drug.

In some embodiments of the present disclosure, the cytotoxic drug is a structure represented by formula (A-1), a stereoisomer thereof, a pharmaceutically acceptable salt thereof, a solvate thereof, or a solvate of the salt thereof, 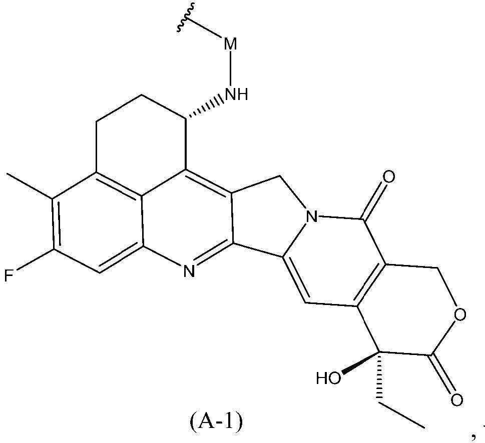 wherein
M is -L²-L¹-C(O)-;
L² is -O- or -S-, and L² is connected to the linker unit L;
L¹ is -(C(R^{1a})(R^{1b}))ₘ-CH₂-, C₃-C₆ saturated cycloalkyl, or 3- to 6-membered saturated heterocyclyl, wherein the C₃-C₆ saturated cycloalkyl and 3- to 6-membered saturated heterocyclyl are each independently and optionally substituted by one or more R^{2a};
m is 1, 2, 3, or 4; heteroatoms in the 3- to 6-membered saturated heterocyclyl are each independently N, O, or S, and the number of heteroatoms is 1, 2, or 3;
R^{1b}, R^{1b}, and R^{2a} are each independently hydrogen, halogen, hydroxyl, amino, or C₁-C₆ alkyl, wherein the C₁-C₆ alkyl is optionally substituted by one or more R;
each R is independently hydrogen or halogen.

In some embodiments of the present disclosure, L¹ is -(C(R¹³)(R^{1b}))ₘ-CH₂-; each R^{1a} is independently hydrogen, halogen, or C₁-C₆ alkyl; each R^{1b} is independently hydrogen, halogen, or C₁-C₆ alkyl.

In some embodiments of the present disclosure, L¹ is

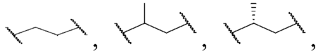

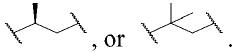

In some embodiments of the present disclosure, L¹ is C₃-C₆ saturated cycloalkyl, wherein the C₃-C₆ saturated cycloalkyl is optionally substituted by one or more R^{2a}, and each R^{2a} is independently hydrogen, halogen, or C₁-C₆ alkyl.

In some embodiments of the present disclosure, L¹ is

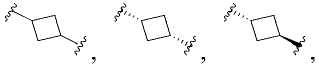

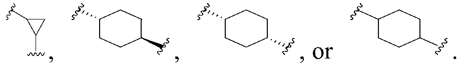

In some embodiments of the present disclosure, the cytotoxic drug is any one of the following structures:

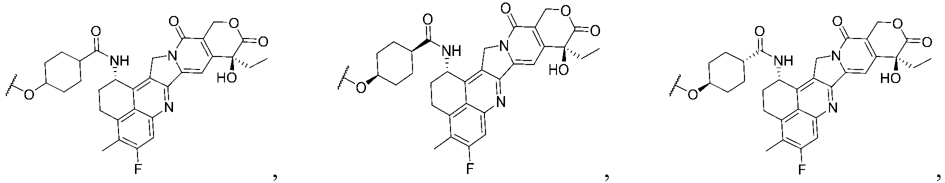

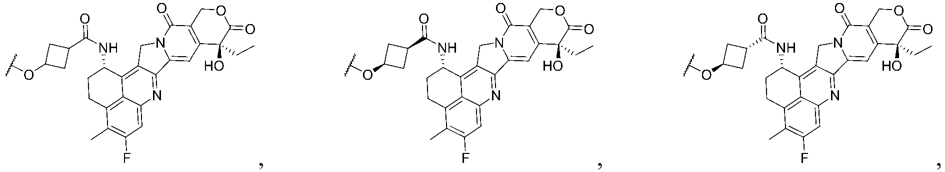

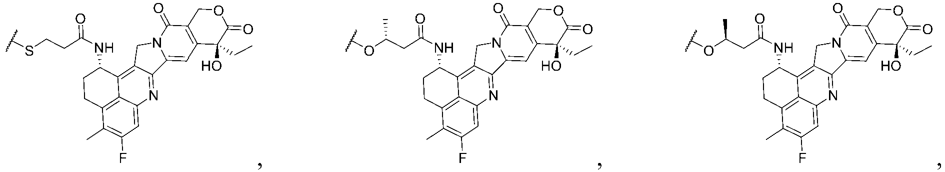

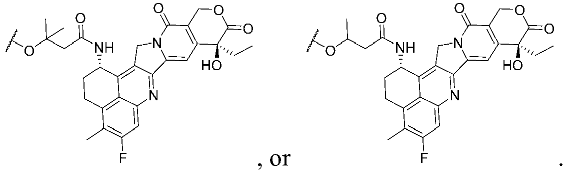

In some embodiments of the present disclosure, -Lₐ- is 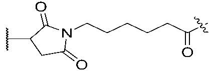 or 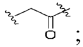 preferably 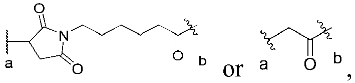 wherein the a-terminus is connected to Ab, and the b-terminus is connected to L_{b}.

In some embodiments of the present disclosure, -L_{b}- is any one of the following structures: 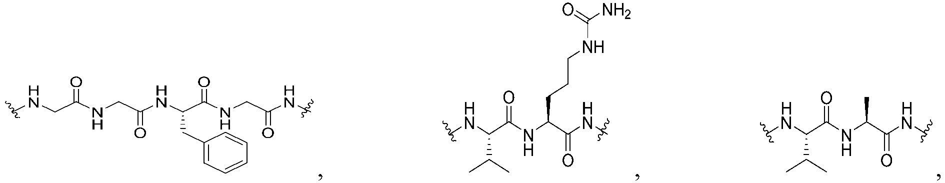 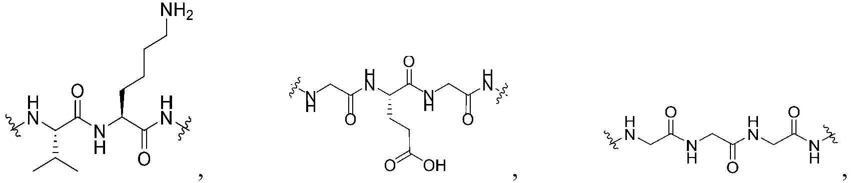 or 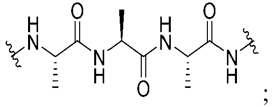 preferably 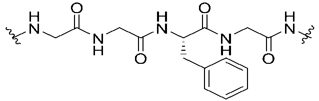 or 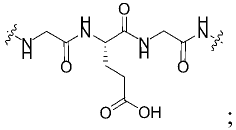 more preferably 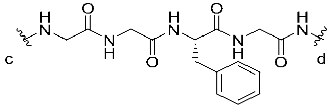 or 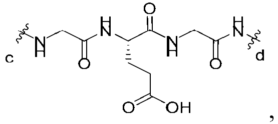 wherein the c-terminus is connected to Lₐ, and the d-terminus is connected to L_{c}.

In some embodiments of the present disclosure, -L_{c}- is

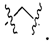

In some embodiments of the present disclosure, the linker unit L is 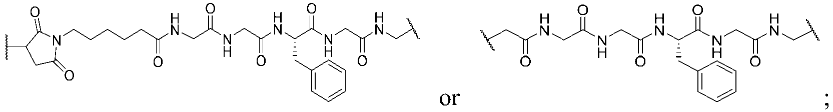 preferably

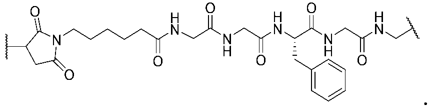

In some embodiments of the present disclosure, the antibody-drug conjugate has a structure represented by formula (A-2): 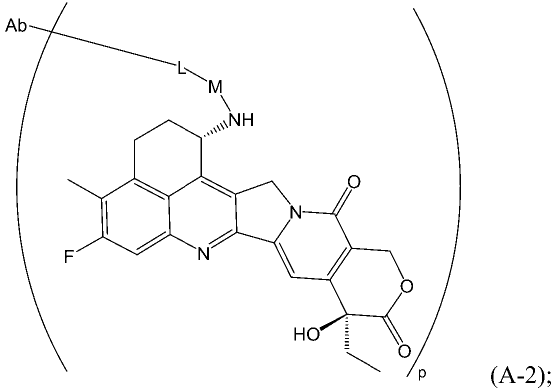 p represents the average number of connections or the number of connections, and p is any integer or decimal from 1 to 10;
Ab is the antibody or the antigen-binding fragment thereof according to the first aspect;
M is as described in the seventh aspect;
L is the linker unit as described above.

In some embodiments of the present disclosure, p is an integer or decimal of 3 to 9.

In some embodiments of the present disclosure, p is an integer or decimal of 7 to 8, such as 7.8 or 7.9.

In some embodiments of the present disclosure, the antibody-drug conjugate is selected from the group consisting of the following structural formulas: 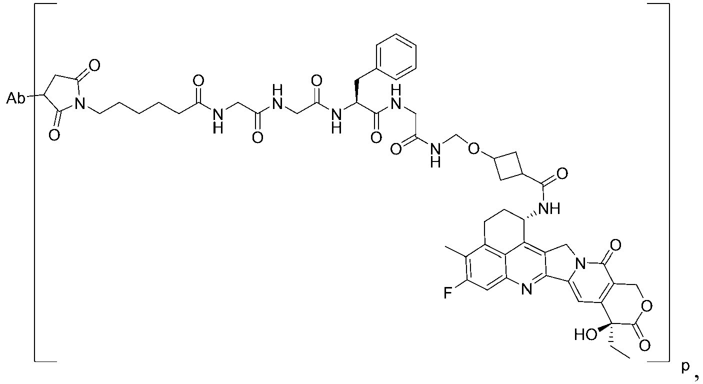 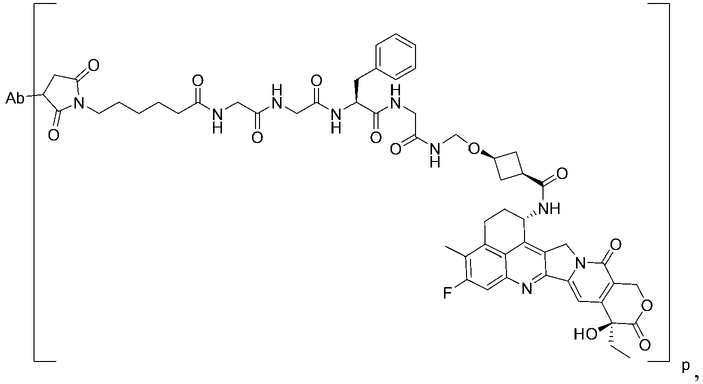 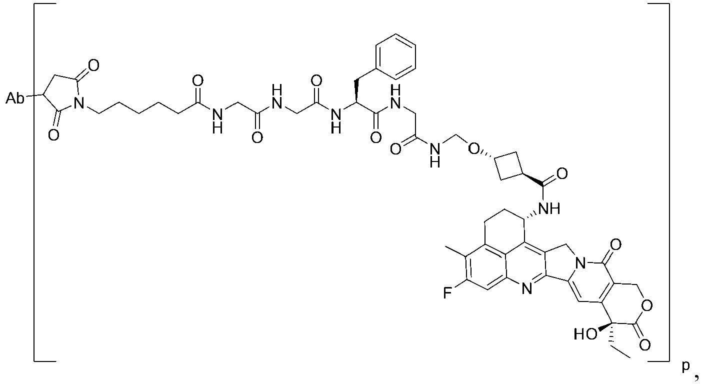 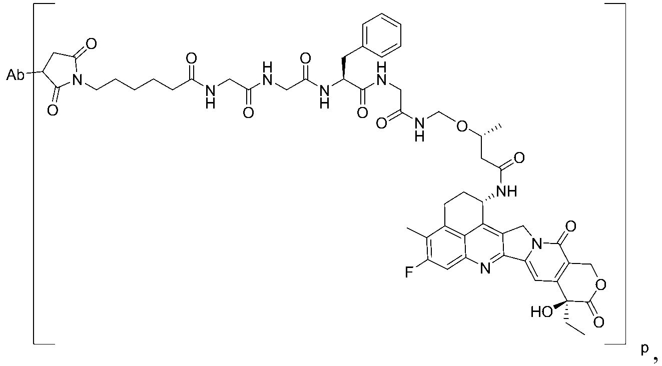 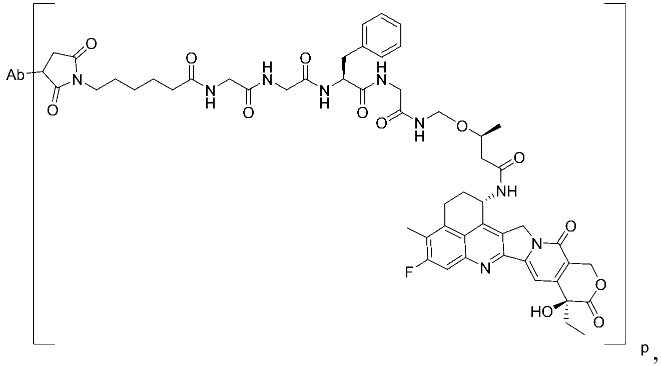 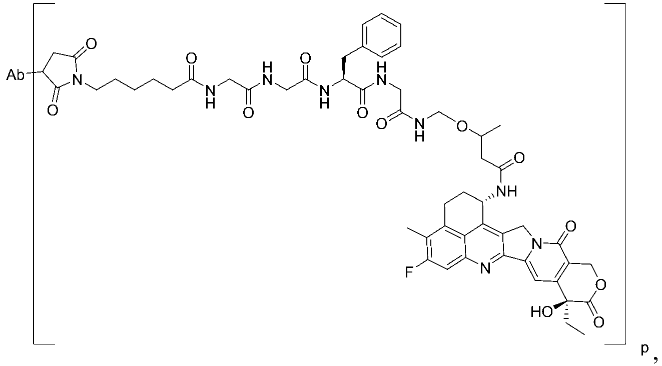 wherein
p is any integer or decimal from 1 to 10, preferably any integer or decimal from 3 to 9; Ab is the antibody or the antigen-binding fragment thereof as described above.

In some embodiments of the present disclosure, the antibody-drug conjugate is any one of the following conjugates: 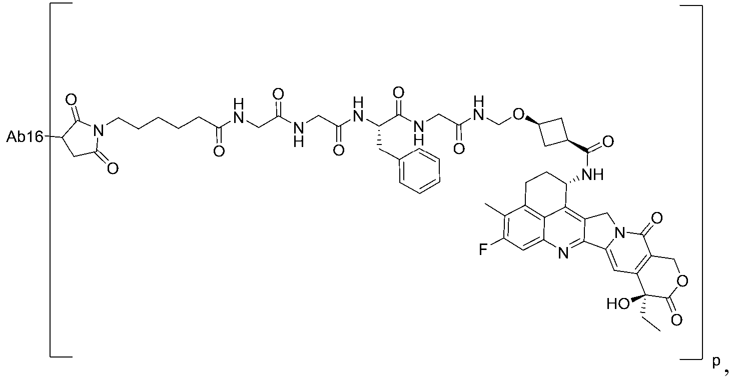
p is any integer or decimal from 1 to 10, preferably any integer or decimal from 3 to 9, more preferably any integer or decimal from 6 to 8; more preferably, p is 7.8; 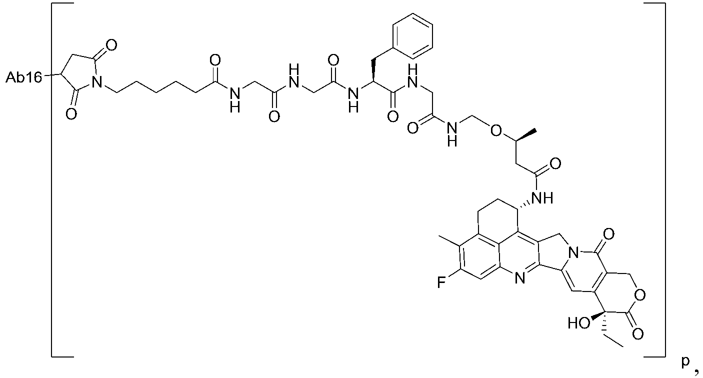
p is any integer or decimal from 1 to 10, preferably any integer or decimal from 3 to 9, more preferably any integer or decimal from 6 to 8; for example, p is 7.9; 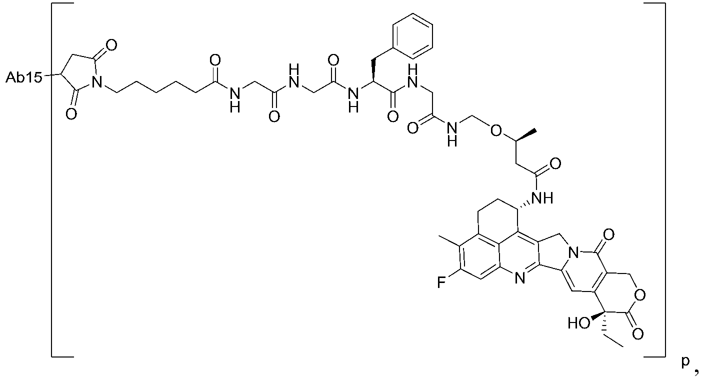
p is any integer or decimal from 1 to 10, preferably any integer or decimal from 3 to 9, more preferably any integer or decimal from 6 to 8; for example, p is 7.8;
Ab16 is an ADAM9-targeting antibody or an antigen-binding fragment thereof, wherein the amino acid sequence of the heavy chain of the Ab16 is shown in SEQ ID NO: 50, and the amino acid sequence of the light chain of the Ab16 is shown in SEQ ID NO: 51;
Ab15 is an ADAM9-targeting antibody or an antigen-binding fragment thereof, wherein the amino acid sequence of the heavy chain of the Ab 15 is shown in SEQ ID NO: 52, and the amino acid sequence of the light chain of the Ab15 is shown in SEQ ID NO: 53.

In some embodiments of the present disclosure, the antibody-drug conjugate is the following conjugate: 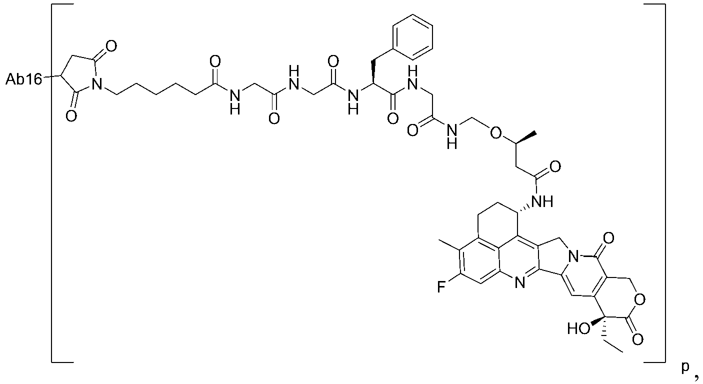 p represents the number of connections, and p is any integer from 1 to 10, preferably any integer from 3 to 9, more preferably any integer from 4 to 8; for example, p is 4, 5, 6, 7, or 8;
Ab16 is an ADAM9-targeting antibody or an antigen-binding fragment thereof, wherein the amino acid sequence of the heavy chain of the Ab16 is shown in SEQ ID NO: 50, and the amino acid sequence of the light chain of the Ab16 is shown in SEQ ID NO: 51.

An eighth aspect of the present disclosure provides a pharmaceutical composition comprising the antibody or the antigen-binding fragment thereof according to the first aspect and/or the antibody-drug conjugate according to the seventh aspect, and a pharmaceutically acceptable carrier.

A ninth aspect of the present disclosure provides the use of the antibody or the antigen-binding fragment thereof according to the first aspect, the antibody-drug conjugate according to the seventh aspect, and/or the pharmaceutical composition according to the eighth aspect in the manufacture of a medicament for diagnosing, preventing, and/or treating cancer with high expression of ADAM9.

In some embodiments of the present disclosure, the cancer is selected from the group consisting of lung cancer, prostate cancer, liver cancer, breast cancer, thyroid cancer, esophageal cancer, pancreatic cancer, gastric cancer, ovarian cancer, and intestinal cancer.

In some specific embodiments of the present disclosure, the cancer is non-small cell lung cancer, such as lung adenocarcinoma.

A tenth aspect of the present disclosure provides a method for diagnosing, preventing, and/or treating cancer with high expression of ADAM9, comprising administering to a patient in need thereof a therapeutically effective amount of the antibody or the antigen-binding fragment thereof according to the first aspect, the antibody-drug conjugate according to the seventh aspect, and/or the pharmaceutical composition according to the eighth aspect.

In some embodiments of the present disclosure, the cancer is selected from the group consisting of lung cancer, prostate cancer, liver cancer, breast cancer, thyroid cancer, esophageal cancer, pancreatic cancer, gastric cancer, ovarian cancer, and intestinal cancer.

In some specific embodiments of the present disclosure, the cancer is non-small cell lung cancer, such as lung adenocarcinoma.

An eleventh aspect of the present disclosure provides the antibody or the antigen-binding fragment thereof according to the first aspect, the antibody-drug conjugate according to the seventh aspect, and/or the pharmaceutical composition according to the eighth aspect for use in diagnosing, preventing, and/or treating cancer with high expression of ADAM9.

In some embodiments of the present disclosure, the cancer is selected from the group consisting of lung cancer, prostate cancer, liver cancer, breast cancer, thyroid cancer, esophageal cancer, pancreatic cancer, gastric cancer, ovarian cancer, and intestinal cancer.

In some specific embodiments of the present disclosure, the cancer is non-small cell lung cancer, such as lung adenocarcinoma.

A twelfth aspect of the present disclosure provides a combination therapy, comprising administering to a patient in need thereof the antibody or the antigen-binding fragment thereof according to the first aspect, the antibody-drug conjugate according to the seventh aspect, and/or the pharmaceutical composition according to the eighth aspect, and a second therapeutic agent.

In some embodiments of the present disclosure, the second therapeutic agent comprises another anti-ADAM9 antibody or an antigen-binding fragment thereof, or an antibody-drug conjugate or pharmaceutical composition comprising the another anti-ADAM9 antibody or the antigen-binding fragment thereof, and/or another medicament for treating cancer with high expression of ADAM9.

In some embodiments of the present disclosure, the cancer is selected from the group consisting of lung cancer, prostate cancer, liver cancer, breast cancer, thyroid cancer, esophageal cancer, pancreatic cancer, gastric cancer, ovarian cancer, and intestinal cancer.

In some specific embodiments of the present disclosure, the cancer is non-small cell lung cancer, such as lung adenocarcinoma.

A thirteenth aspect of the present disclosure provides a method for diagnosing, preventing, and/or treating cancer, comprising administering to a patient in need thereof a therapeutically effective amount of the antibody or the antigen-binding fragment thereof according to the first aspect, the antibody-drug conjugate according to the seventh aspect, and/or the pharmaceutical composition according to the eighth aspect.

In some embodiments of the present disclosure, the cancer is selected from the group consisting of lung cancer, prostate cancer, liver cancer, breast cancer, thyroid cancer, esophageal cancer, pancreatic cancer, gastric cancer, ovarian cancer, and intestinal cancer.

In some specific embodiments of the present disclosure, the cancer is non-small cell lung cancer, such as lung adenocarcinoma.

A fourteenth aspect of the present disclosure provides the antibody or the antigen-binding fragment thereof according to the first aspect, the antibody-drug conjugate according to the seventh aspect, and/or the pharmaceutical composition according to the eighth aspect for use in diagnosing, preventing, and/or treating cancer.

In some embodiments of the present disclosure, the cancer is selected from the group consisting of lung cancer, prostate cancer, liver cancer, breast cancer, thyroid cancer, esophageal cancer, pancreatic cancer, gastric cancer, ovarian cancer, and intestinal cancer.

In some specific embodiments of the present disclosure, the cancer is non-small cell lung cancer, such as lung adenocarcinoma.

A fifteenth aspect of the present disclosure provides a combination therapy, comprising administering to a patient in need thereof the antibody or the antigen-binding fragment thereof according to the first aspect, the antibody-drug conjugate according to the seventh aspect, and/or the pharmaceutical composition according to the eighth aspect, and a second therapeutic agent.

In some embodiments of the present disclosure, the second therapeutic agent comprises another anti-ADAM9 antibody or an antigen-binding fragment thereof, or an antibody-drug conjugate or pharmaceutical composition comprising the another anti-ADAM9 antibody or the antigen-binding fragment thereof, and/or another medicament for treating cancer.

In some embodiments of the present disclosure, the cancer is selected from the group consisting of lung cancer, prostate cancer, liver cancer, breast cancer, thyroid cancer, esophageal cancer, pancreatic cancer, gastric cancer, ovarian cancer, and intestinal cancer.

In some specific embodiments of the present disclosure, the cancer is non-small cell lung cancer, such as lung adenocarcinoma.

A sixteenth aspect of the present disclosure provides a method for preparing the antibody-drug conjugate according to the seventh aspect, comprising reacting the antibody or the antigen-binding fragment thereof according to the first aspect with a compound represented by formula II to obtain the antibody-drug conjugate,

L'-cytotoxic drug II ;

L' forms the linker unit L according to the seventh aspect with the antibody or the antigen-binding fragment thereof;
the cytotoxic drug is as described in the seventh aspect.

In some embodiments of the present disclosure, the antibody-drug conjugate satisfies one or more of the following conditions:
(1) the compound represented by formula II is 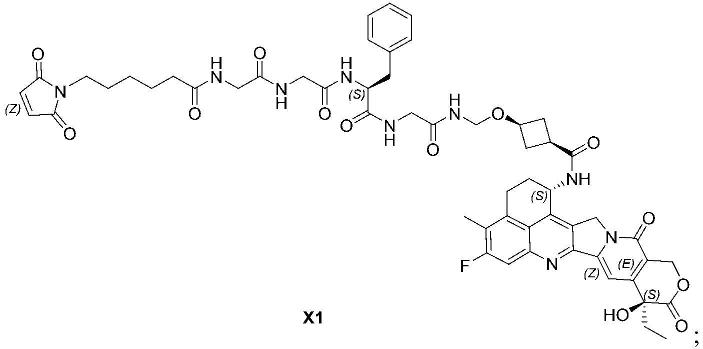 or 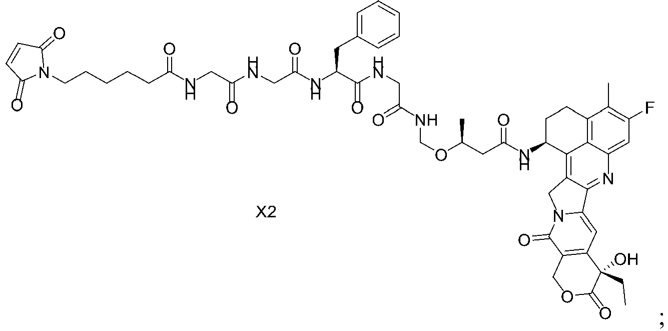
(2) the antibody or the antigen-binding fragment thereof is Ab16 or Ab15;
   the amino acid sequence of the heavy chain of the Ab16 is preferably shown in SEQ ID NO: 50, and the amino acid sequence of the light chain of the Ab16 is preferably shown in SEQ ID NO: 51;
   the amino acid sequence of the heavy chain of the Ab15 is preferably shown in SEQ ID NO: 52, and the amino acid sequence of the light chain of the Ab15 is preferably shown in SEQ ID NO: 53.

A seventeenth aspect of the present disclosure provides the use of the antibody or the antigen-binding fragment thereof according to the first aspect, the antibody-drug conjugate according to the seventh aspect, and/or the pharmaceutical composition according to the eighth aspect in the manufacture of a medicament for diagnosing, preventing, and/or treating cancer.

In some embodiments of the present disclosure, the cancer is selected from the group consisting of lung cancer, prostate cancer, liver cancer, breast cancer, thyroid cancer, esophageal cancer, pancreatic cancer, gastric cancer, ovarian cancer, and intestinal cancer.

In some specific embodiments of the present disclosure, the cancer is non-small cell lung cancer, such as lung adenocarcinoma.

On the basis of common knowledge in the art, the above preferred conditions can be arbitrarily combined to obtain various preferred examples of the present disclosure.

The reagents and starting materials used in the present disclosure are commercially available.

The positive and progressive effects of the present disclosure are as follows:

The ADAM9-targeting antibody of the present disclosure has one or more of the following advantages:
1. Compared to the humanized anti-ADAM9 antibody hMAB-A (2I.2) in the prior art, the antibody of the present disclosure exhibits higher hydrophilicity, and better stability and druggability of antibody molecules;
2. Compared to the humanized anti-ADAM9 antibody hMAB-A (2I.2) in the prior art, the antibody of the present disclosure exhibits significantly improved expression levels, a lower proportion of high molecular weight polymers, and better druggability;
3. The antibody of the present disclosure exhibits good affinity for both human and monkey ADAM9 antigens;
4. The antibody of the present disclosure exhibits a high internalization rate for cells expressing ADAM9.

The antibody-drug conjugate of the present disclosure has one or both of the following advantages:
1. The antibody-drug conjugate of the present disclosure exhibits good inhibitory activity against the proliferation of ADAM9-positive human colorectal cancer cells LS174T or Colo205 and human lung adenocarcinoma cells Calu-3;
2. The antibody-drug conjugate of the present disclosure exhibits significant antitumor activity against DLD-1 human colorectal cancer xenograft mice, LS174T colorectal cancer xenograft mice, and Calu-3 human lung cancer xenograft mice.

### BRIEF DESCRIPTION OF THE DRAWINGS

FIG. 1 shows the results of *in vivo* tumor inhibition assay of ADAM9-ADC in DLD-1 human colorectal cancer xenograft mice.
FIG. 2 shows the results of *in vivo* tumor inhibition assay of ADAM9-ADC in LS174T human colorectal cancer xenograft mice.
FIG. 3 shows the results of *in vivo* tumor inhibition assay of ADAM9-ADC in Calu-3 human lung cancer xenograft mice.
FIG. 4a shows the results of FACS binding assay on LS174T cells.
FIG. 4b shows the results of proliferation inhibition assay of LS174T-ADC.
FIG. 5a shows the results of FACS binding assay on HepG2 cells.
FIG. 5b shows the results of proliferation inhibition assay of HepG2-ADC.

### DETAILED DESCRIPTION OF THE PREFERRED EMBODIMENT

### Definitions

In the present disclosure, the letters in the amino acid sequence denote the single-letter abbreviations of amino acids well known in the art, *e.g.,* as described in J. Biol. Chem, 243, p3558 (1968):
Alanine: Ala-A; Arginine: Arg-R; Aspartic acid: Asp-D; Cysteine: Cys-C; Glutamine: Gln-Q; Glutamic acid: Glu-E; Histidine: His-H; Glycine: Gly-G; Asparagine: Asn-N; Tyrosine: Tyr-Y; Proline: Pro-P; Serine: Ser-S; Methionine: Met-M; Lysine: Lys-K; Valine: Val-V; Isoleucine: Ile-I; Phenylalanine: Phe-F; Leucine: Leu-L; Tryptophan: Trp-W; Threonine: Thr-T.

In the present disclosure, the term "and/or" should be understood to mean any one of the options or a combination of any two or more of the options.

In the present disclosure, the term "or" should be understood to have the same meaning as "and/or" as defined above. For example, when separating items in a list, "or" or "and/or" should be construed as being inclusive, that is, including at least one, but also more than one, of the quantities or elements in the list, and optionally, additional unlisted items. Only terms expressly indicated to the contrary, such as "only one" or "exactly one", or "consisting of" used in the claims, will refer to only one number or only one element in the list.

In the present disclosure, the term "antibody-drug conjugate" generally refers to an antibody connected to a biologically active cytotoxic drug via a stable linker unit. In the present disclosure, an "antibody-drug conjugate" may refer to an antibody or an antigen-binding fragment thereof connected to a biologically active cytotoxic drug fragment via a stable linker unit.

In the present disclosure, the term "cytotoxic drug" generally refers to a toxic drug, which may have chemical molecules capable of significantly disrupting the normal growth of tumor cells. Cytotoxic drugs can kill tumor cells at a sufficiently high concentration. The "cytotoxic drug" may include payloads, such as small molecule payloads or enzymatically active payloads derived from bacterial, fungal, plant, or animal sources, radioactive isotopes *(e.g.,* At²¹¹, I¹³¹, I¹²⁵, Y⁹⁰, Re¹⁸⁶, Re¹⁸⁸, Sm¹⁵³, Bi²¹², P³², or radioactive isotopes of Lu), toxic drugs, chemotherapeutic drugs, antibiotics, or nucleolytic enzymes, or their derivatives. For example, cytotoxic drugs may be toxic drugs, including, but not limited to, camptothecin derivatives, such as Exatecan (chemical name: (1S,9S)-1-amino-9-ethyl-5-fluoro-2,3-dihydro-9-hydroxy-4-methyl-1H,12H-benzo[de]pyrano[3',4':6,7]imidazo[1,2-b]quinoline-10,13(9H,15H)-dione).

In the present disclosure, the term "antibody" generally refers to an immunoglobulin that is reactive to a specified protein or peptide or a fragment thereof. The antibody may be antibodies from any class, including, but not limited to, IgG, IgA, IgM, IgD, and IgE, as well as antibodies from any subclass *(e.g.,* IgG1, IgG2, IgG3, and IgG4). The antibody may have a heavy chain constant region selected from, for example, IgG1, IgG2, IgG3, or IgG4. The antibody may also have a light chain selected from, for example, kappa (κ) or lambda (λ). The antibody of the present disclosure may be derived from any species. The term "antibody" may include intact polyclonal antibodies, intact monoclonal antibodies, multispecific antibodies (*e.g*., bispecific antibodies), chimeric antibodies, humanized antibodies, human antibodies, fusion proteins comprising antibodies, and any other modified immunoglobulin molecules, provided that such antibodies exhibit the desired biological activity.

In the present disclosure, the term "antigen-binding fragment" generally refers to a portion of an antibody molecule that contains amino acids responsible for the specific binding between an antibody and an antigen. The portion of an antigen that is specifically recognized and bound by an antibody is referred to as an "epitope". As described above, the antigen-binding domain may typically comprise an antibody light chain variable region (VL) and an antibody heavy chain variable region (VH); however, it is not necessary to comprise both. An Fd fragment, for example, comprises two VH regions and generally retains some antigen-binding functionality of the intact antigen-binding domain. Examples of antigen-binding fragments of an antibody include (1) an Fab fragment, a monovalent fragment having VL, VH, constant light chain (CL), and CH1 domains; (2) an F(ab')₂ fragment, a bivalent fragment comprising two Fab fragments linked by a disulfide bridge at the hinge region; (3) an Fd fragment having two VH and CH1 domains; (4) an Fv fragment having VL and VH domains of a single arm of an antibody; (5) a dAb fragment (Ward et al., "Binding Activities of a Repertoire of Single Immunoglobulin Variable Domains Secreted From Escherichia coli", Nature 341: 544-546 (1989), the contents of which are incorporated herein by reference in their entirety) having a VH domain; (6) an isolated complementarity-determining region (CDR); (7) a single-chain Fv (scFv), such as one derived from an scFv library. Although the two domains of the Fv fragment, VL and VH, are encoded by separate genes, they can be joined using recombinant methods via a synthetic linker, enabling their preparation as a single protein chain (referred to as single-chain Fv (scFv)) in which the VL and VH regions are paired to form a monovalent molecule (see, *e.g.,* Huston et al., "Protein Engineering of Antibody Binding Sites: Recovery of Specific Activity in an Anti-Digoxin Single-Chain Fv Analogue Produced in Escherichia coli", Proc. Natl. Acad. Sci. USA 85: 5879-5883 (1988)); (8) "VHH" refers to the variable antigen-binding domain derived from a camelid (camel, dromedary, llama, alpaca, *etc*.) heavy-chain antibody (see Nguyen V.K. et al., 2000, The EMBO Journal, 19, 921-930; Muyldermans S., 2001, J Biotechnol., 74, 277-302; and for review Vanlandschoot P. et al., 2011, Antiviral Research 92, 389-407). VHH may also be referred to as a nanobody (Nb).

In the present disclosure, the term "variable region" or "variable domain" generally refers to the domain of an antibody heavy or light chain that is involved in binding of the antibody to an antigen. In the present disclosure, the term "variable" generally refers to certain portions of the sequence of the variable domains of an antibody that exhibit substantial variation, resulting in the binding and specificity of various specific antibodies to their respective antigens. Variability is not uniformly distributed throughout the variable region of an antibody. It is concentrated in three segments of the light chain variable region and heavy chain variable region, referred to as complementarity-determining regions (CDRs) or hypervariable regions (HVRs), namely LCDR1, LCDR2, LCDR3, HCDR1, HCDR2, and HCDR3. The more highly conserved portions within the variable domains are referred to as framework regions (FRs). The natural heavy and light chain variable domains each comprise four FRs (H-FR1, H-FR2, H-FR3, H-FR4, L-FR1, L-FR2, L-FR3, L-FR4), predominantly adopting a β-sheet conformation, connected by three CDR structural loop regions. The CDRs in each chain are closely juxtaposed via the FRs and, together with the CDRs from the other chain, form the antigen-binding site of the antibody.

In the present disclosure, the amino acid sequences of the listed CDRs are defined according to Kabat definition. However, it is well known to those skilled in the art that the CDRs of antibodies can be defined by various methods in the art, *e.g.,* Chothia based on the three-dimensional structure of antibodies and the topology of CDR loops (Chothia et al. (1989) Nature 342: 877-883, Al-Lazikani et al., "Standard conformations for the canonical structures of immunoglobulins", Journal of Molecular Biology, 273: 927-948 (1997)); Kabat based on the variability of antibody sequences (Kabat et al., Sequences of Proteins of Immunological Interest, 4th Edition, U.S. Department of Health and Human Services, National Institutes of Health (1987)); AbM (University of Bath), Contact (University College London), international ImMunoGeneTics database (IMGT) (www.imgt.cines.fr/ on the World Wide Web), and North CDR definition based on the affinity propagation clustering using a large number of crystal structures. It should be understood by those skilled in the art that, unless otherwise specified, the terms "CDR" and "complementarity-determining region" of a given antibody or region thereof (*e.g.,* a variable region) should be understood to encompass the complementarity-determining regions as defined according to any one of the known schemes described in the present disclosure. Although the scope of protection claimed in the present disclosure is based on the sequences defined according to Kabat definition, the corresponding amino acid sequences defined according to other CDR definitions should also fall within the scope of protection of the present disclosure. Therefore, when an antibody is defined by the specific CDR sequences as defined in the present disclosure, the scope of the antibody also encompasses antibodies whose variable region sequences contain the specific CDR sequences but whose claimed CDR boundaries differ from the specific CDR boundaries defined in the present disclosure due to the application of different schemes (*e.g*., different assignment system rules or their combinations).

The calculation of sequence identity between sequences is performed as follows. To determine the percent identity between two amino acid sequences, the sequences are aligned for optimal comparison purposes (*e.g*., gaps may be introduced in the first and second amino acid sequences for optimal alignment, or non-homologous sequences may be discarded for comparison purposes). In one preferred embodiment, for comparison purposes, the length of the aligned reference sequence is at least 30%, preferably at least 40%, more preferably at least 50%, 60%, and even more preferably at least 70%, 80%, 90%, 100% of the length of the reference sequence. Subsequently, the amino acid residues at corresponding amino acid positions are compared. When a position in the first sequence is occupied by the same amino acid residue as the corresponding position in the second sequence, the molecules are identical at the position. A mathematical algorithm may be used to achieve the sequence comparison and calculation of percent identity between two sequences. In one preferred embodiment, the percent identity between two amino acid sequences is determined using the Needleman and Wunsch ((1970) J. Mol. Biol. 48: 444-453) algorithm (available at http://www.gcg.com) which has been integrated into the GAP program of the GCG software package, using a Blossum 62 matrix or a PAM250 matrix, with a gap weight of 16, 14, 12, 10, 8, 6, or 4 and a length weight of 1, 2, 3, 4, 5, or 6. A particularly preferred set of parameters (and one that should be used unless otherwise specified) is a Blossum 62 scoring matrix with a gap penalty of 12, a gap extension penalty of 4, and a frameshift gap penalty of 5. The percent identity between two amino acid sequences can also be determined using the E. Meyers and W. Miller algorithm ((1989) CABIOS, 4: 11-17) which has been incorporated into the ALIGN program (version 2.0), with a PAM120 weighted remainder table, a gap length penalty of 12, and a gap penalty of 4. Additionally or alternatively, the protein sequences described in the present disclosure may be further used as "query sequences" to perform searches against public databases, *e.g.,* to identify other family member sequences or related sequences.

In the present disclosure, the term "full-length antibody" is used interchangeably to refer to a glycoprotein comprising at least two heavy chains (HC) and two light chains (LC) interconnected by disulfide bonds. Each heavy chain consists of a heavy chain variable region (abbreviated as VH in the present disclosure) and a heavy chain constant region. The heavy chain constant region consists of three domains, namely CH1, CH2, and CH3. Each light chain consists of a light chain variable region (abbreviated as VL in the present disclosure) and a light chain constant region (abbreviated as CL in the present disclosure). The light chain constant region consists of one domain, namely CL. Mammalian heavy chains are classified as α, δ, ε, γ, and µ. Mammalian light chains are classified as λ or κ. Immunoglobulins comprising heavy chains α, δ, ε, γ, and µ are classified as immunoglobulin (Ig) A, IgD, IgE, IgG, and IgM. The intact antibody forms a "Y" shape. The stem of the Y consists of the second and third constant regions (and for IgE and IgM, the fourth constant region) of the two heavy chains bound together via a disulfide bond (interchain) formed in the hinge. Heavy chains γ, α, and δ have a constant region consisting of three tandem (in a row) Ig domains and a hinge region for increased flexibility. Heavy chains µ and ε have a constant region consisting of four immunoglobulin domains. The second and third constant regions are referred to as the "CH2 domain" and "CH3 domain", respectively. Each arm of the Y comprises the variable region and the first constant region of a single heavy chain bound to the variable and constant regions of a single light chain. The variable regions of the light and heavy chains are responsible for antigen binding.

In the present disclosure, a "Fab" fragment consists of one light chain and the CH1 and variable region of one heavy chain. The heavy chain of a Fab molecule cannot form a disulfide bond with another heavy chain molecule. An "Fc" region contains two heavy chain fragments comprising the CH2 and CH3 domains of an antibody. The two heavy chain fragments are held together by two or more disulfide bonds and by hydrophobic interactions of the CH3 domains. A "Fab‴ fragment contains one light chain and a portion of one heavy chain comprising the VH domain and the CH1 domain as well as the region between the CH1 and CH2 domains, such that an interchain disulfide bond can be formed between the two heavy chains of two Fab' fragments to form a F(ab')₂ molecule. A "F(ab')₂" fragment contains two light chains and two heavy chains comprising a portion of the constant region between the CH1 and CH2 domains, such that an interchain disulfide bond is formed between the two heavy chains. Thus, the F(ab')₂ fragment consists of two Fab' fragments held together by a disulfide bond between the two heavy chains. The term "Fv" refers to an antibody fragment consisting of the VL and VH domains of a single arm of an antibody, but lacking a constant region.

In the present disclosure, the scFv (single-chain antibody fragment) may be a conventional single-chain antibody in the art, which comprises a heavy chain variable region, a light chain variable region, and a short peptide of 15 to 20 amino acids. The VL and VH domains form a monovalent molecule via a linker which enables them to be paired as a single polypeptide chain [see, *e.g.,* Bird et al., Science 242: 423-426 (1988) and Huston et al., Proc. Natl. Acad. Sci. USA 85: 5879-5883 (1988)]. Such scFv molecules may have a general structure: NH₂-VL-linker-VH-COOH or NH₂-VH-linker-VL-COOH. Suitable linkers in the prior art consist of repetitive G₄S amino acid sequences or variants thereof. For example, a linker with the amino acid sequence (G₄S)₄ or (G₄S)₃ may be used, but variants thereof may also be used.

In the present disclosure, the term "monoclonal antibody" refers to an antibody derived from a substantially homogeneous population of antibodies, *i.e.,* the individual antibodies comprising the population are identical, except for possible naturally occurring mutations that may be present in minor quantities. Monoclonal antibodies are highly specific, being directed against a single antigenic epitope. In contrast, conventional (polyclonal) antibody preparations typically include a multitude of antibodies directed against (or specific for) different epitopes. The modifier "monoclonal" indicates the characteristics of an antibody derived from a substantially homogeneous population of antibodies and should not be construed as requiring the antibody to be produced by any particular method.

The term "multispecific antibody" is used in its broadest sense to encompass antibodies with multiple epitope specificities. These multispecific antibodies include, but are not limited to, antibodies comprising a heavy chain variable region (VH) and a light chain variable region (VL), wherein the VH-VL unit has multiple epitope specificities; antibodies having two or more VL and VH regions, wherein each VH-VL unit binds to different targets or different epitopes of the same target; antibodies having two or more single variable regions, wherein each single variable region binds to different targets or different epitopes of the same target; full-length antibodies, antibody fragments, diabodies, triabodies, antibody fragments covalently or non-covalently linked together, *etc.*

In the present disclosure, the term "humanized antibody" refers to an antibody form containing sequences derived from human and non-human (*e.g*., mouse, rat) antibodies. Generally, a humanized antibody comprises substantially all of at least one, typically two, variable domains, wherein all or substantially all of the hypervariable loops correspond to those of a non-human immunoglobulin, while all or substantially all of the framework regions (FRs) are those of a human immunoglobulin sequence. The humanized antibody may optionally comprise at least a portion of a human immunoglobulin constant region (Fc).

In the present disclosure, "affinity" or "binding affinity" refers to the inherent binding affinity that reflects the interaction between members of a binding pair. The affinity of molecule X for its partner Y can be typically represented by the equilibrium dissociation constant (K_{D}), which is the ratio of the dissociation rate constant to the binding rate constant (k_{dis} and kₒₙ, respectively). Affinity may be measured by common methods known in the art. In some embodiments of the present disclosure, surface plasmon resonance (SPR) technology is used to measure affinity, such as the affinity between an antibody and an antigen of the present disclosure. In some preferred embodiments of the present disclosure, one specific method for measuring affinity is the Biacore method disclosed herein.

In the present disclosure, the term "halogen" generally refers to fluorine, chlorine, bromine, or iodine, for example, fluorine or chlorine.

In the present disclosure, the term "alkyl" generally refers to a residue derived from an alkane by removal of a hydrogen atom. The alkyl group may be substituted or unsubstituted, or replaced or unreplaced. The term "alkyl" generally refers to a saturated linear or branched aliphatic hydrocarbon group having a residue derived from the parent alkane by removal of hydrogen atoms from the same carbon atom or two different carbon atoms, which may be a linear or branched group containing 1 to 20 carbon atoms, for example, a chain alkyl group containing 1 to 12 carbon atoms, such as 1 to 6 carbon atoms. Non-limiting examples of alkyl groups include, but are not limited to, methyl, ethyl, propyl, butyl, *etc.*

In the present disclosure, the term "cycloalkyl" refers to a saturated or partially unsaturated monocyclic or polycyclic hydrocarbon substituent containing 3 to 20 carbon atoms, preferably 3 to 12 carbon atoms, preferably 3 to 10 carbon atoms, preferably 3 to 8 carbon atoms, more preferably 3 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, *etc.* Polycyclic cycloalkyl groups include spiro, fused, and bridged cycloalkyl groups.

In the present disclosure, the term "heterocyclyl" refers to a saturated or partially unsaturated monocyclic or polycyclic hydrocarbon substituent containing 3 to 20 ring atoms, wherein one or more of the ring atoms are heteroatoms selected from nitrogen, oxygen, or sulfur, and the remaining ring atoms are carbon; preferably comprising 3 to 12 ring atoms, 1 to 4 of which are heteroatoms; more preferably comprising 3 to 8 ring atoms, 1 to 3 of which are heteroatoms; still more preferably comprising 3 to 6 ring atoms, 1 to 3 of which are heteroatoms; most preferably comprising 5 or 6 ring atoms, 1 to 3 of which are heteroatoms. Non-limiting examples of monocyclic heterocyclyl groups include pyrrolidinyl, tetrahydropyranyl, piperidinyl, morpholinyl, thiomorpholinyl, homopiperazinyl, *etc.* Polycyclic heterocyclyl groups include spiro, fused, and bridged heterocyclyl groups. The heterocyclyl ring may be fused to an aryl, heteroaryl, or cycloalkyl ring, wherein the ring attached to the parent structure is a heterocyclyl ring.

In the present disclosure, the term "each independently" generally refers to a variable applicable to any circumstance, regardless of the presence or absence of variables with identical or different definitions in the same compound. For example, the variables may refer to the type or number of substituents of a compound, or the type of atoms in the compound. For example, when R appears twice in a compound and R is defined as "independently carbon or nitrogen", both R may be carbon, both R may be nitrogen, or one R may be carbon while the other R may be nitrogen.

In the present disclosure, the term "optional" or "optionally" generally means that the subsequently described event or circumstance may but does not necessarily occur, and that the description includes both cases where the event or circumstance does or does not occur. For example, "heterocyclyl optionally substituted by an alkyl group" means that the alkyl group may but does not necessarily have to be present, and that the description includes the case where the heterocyclyl group is substituted by an alkyl group and the case where the heterocyclyl group is not substituted by an alkyl group.

In the present disclosure, the term "substituted" generally means that one or more hydrogen atoms (*e.g.,* up to 5 hydrogen atoms, such as 1 to 3 hydrogen atoms) are independently substituted by a corresponding number of substituents in a group. Substituents are only located at their possible chemical positions, and those skilled in the art are able to determine (experimentally or theoretically) possible or impossible substitutions without undue effort. For example, an amino or hydroxyl group with a free hydrogen may be unstable when bonded to a carbon atom with an unsaturated (*e.g*., olefinic) bond.

In the present disclosure, as known to those skilled in the art, terms such as "alkyl", "alkenyl", and "cycloalkyl" may be preceded by an identifier indicating the number of atoms present in the group under specific circumstances, for example, C₁-C₄ alkyl, C₃-C₇ cycloalkoxy, and C₁-C₄ alkylcarbonylamino, where the subscript number following "C" indicates the number of carbon atoms present in the group. For example, C₃ alkyl refers to an alkyl group having three carbon atoms (*e.g*., n-propyl, isopropyl); for C₁₋₁₀, the members of the group may have any number of carbon atoms falling within the range of 1 to 10.

In the present disclosure, the compound or antibody-drug conjugate of the present disclosure includes a tautomer, mesomer, racemate, enantiomer, and/or diastereomer thereof. In the present disclosure, the term "diastereomer" generally refers to a stereoisomer having two or more chiral centers and whose molecules are not mirror images of each other. Diastereomers may exhibit distinct physical properties, such as melting point, boiling point, spectral properties, and reactivity. In the present disclosure, the terms "tautomer" or "tautomeric form" are used interchangeably and generally refer to structural isomers of different energy levels that can be interconverted via a low energy barrier. For example, proton tautomers (also known as prototropic tautomers) include interconversions via proton migration, such as keto-enol isomerization and imine-enamine isomerization. Valence tautomers include interconversions through the reorganization of some bonding electrons. In the present disclosure, the term "mesomer" generally refers to a molecule containing asymmetric atoms, but having symmetric factors that result in a total optical rotation of zero within the molecule. The term "racemate" or "racemic mixture" refers to a composition consisting of two enantiomeric substances in equimolar amounts.

In the present disclosure, the term "linker unit" or "linker structure" generally refers to a chemical structural fragment or bond that is linked to a ligand at one end and to a cytotoxic drug at the other end, and may also be linked to other linkers before being linked to the cytotoxic drug. The direct or indirect linking of a ligand may mean that the group is directly linked to the ligand via a covalent bond, or may be linked to the ligand via a linker structure. For example, a chemical structural fragment or bond comprising an acid-labile linker structure (*e.g.,* hydrazone), a protease-sensitive (*e.g.,* peptidase-sensitive) linker structure, a photolabile linker structure, a dimethyl linker structure, or a disulfide-containing linker structure may be used as a linker structure.

In certain examples of the present disclosure, an antibody-drug conjugate refers to a composition with different DAR distributions. The term "drug loading" generally refers to the average amount of cytotoxic drug loaded per ligand, which may be referred to as the average number of connections, or may be expressed as the ratio of the amount of cytotoxic drug to the amount of antibody. The cytotoxic drug loading may range from 0 to 12 (*e.g*., 1 to 10) cytotoxic drugs per ligand (Ab). The drug loading per ADC molecule after the coupling reaction may be characterized by conventional methods such as UV/visible spectroscopy, mass spectrometry, ELISA, and HPLC. The average number of connections p may be an integer or decimal from 1 to 10. For example, the average number of connections p may be an integer or decimal from 2 to 8. For example, the average number of connections p may be an integer or decimal from 3 to 8. For example, the average number of connections p may be an integer or decimal from 1 to 2, 2 to 3, 3 to 4, 4 to 5, 5 to 6, 6 to 7, 7 to 8, 8 to 9, or 9 to 10. For example, the average number of connections p is 7.8 or 7.9.

In certain examples of the present disclosure, an antibody-drug conjugate refers to a compound with the same DAR distribution. The term "drug loading" refers to the amount of cytotoxic drug loaded per ligand, which may be referred to as the number of connections, or may be expressed as the ratio of the amount of cytotoxic drug to the amount of antibody. The cytotoxic drug loading may range from 0 to 12 (*e.g.,* 1 to 10) cytotoxic drugs per ligand (Ab). The number of connections p may be any integer from 1 to 10. For example, the number of connections p may be any integer from 3 to 9. For example, the number of connections p may be any integer from 4 to 8. For example, the number of connections p may be 4, 5, 6, 7, or 8.

In the present disclosure, certain atoms of the compounds or antibody-drug conjugates of the present disclosure may be present in more than one isotopic form. For example, hydrogen may occur as protium (¹H), deuterium (²H), and tritium (³H), while carbon may naturally occur as three different isotopes (¹²C, ¹³C, and ¹⁴C). Examples of isotopes that may be incorporated into the compounds of the present disclosure also include, but are not limited to, ¹⁵N, ¹⁸O, ¹⁷O, ¹⁸F, ³²P, ³³P, ¹²⁹I, ¹³¹I, ¹²³I, ¹²⁴I, ¹²⁵I, or similar isotopes. Therefore, relative to the natural abundance of these isotopes, the compounds or antibody-drug conjugates of the present disclosure may be enriched in one or more of these isotopes. As known to those skilled in the art, such isotopically enriched compounds may be used for a variety of purposes. For example, substitution with heavy isotopes such as deuterium (²H) may provide certain therapeutic advantages, possibly due to higher metabolic stability. For example, the natural abundance of deuterium (²H) is approximately 0.015%. Accordingly, one out of approximately 6,500 hydrogen atoms in nature is a deuterium atom. Therefore, the deuterium-containing compound or antibody-drug conjugate of the present disclosure has a deuterium abundance of greater than 0.015% at one or more sites, as the case may be. Unless otherwise indicated, the structures described in the present disclosure may also include compounds or antibody-drug conjugates that differ only in the presence or absence of one or more isotopically enriched atoms. For example, except for the substitution of hydrogen atoms with deuterium or tritium or the substitution of carbon atoms with carbon-13 or carbon-14, compounds or antibody-drug conjugates whose structures are consistent with the present disclosure are within the scope of the present disclosure.

As known in the art, in the present disclosure, the term "nucleic acid" refers to a nucleotide chain of any length, and includes DNA and RNA. The nucleotide may be a deoxyribonucleotide, a ribonucleotide, a modified nucleotide or base, and/or analogs thereof, or any substrate capable of being incorporated into a chain by a DNA or RNA polymerase.

The recombinant expression vector of the present disclosure may be any suitable recombinant expression vector that can be used to transform or transfect one or more genes or sequences of interest into any suitable host cell and preferably express the genes or sequences in the host cell. Suitable vectors include those designed for expansion and amplification or for expression or both. Examples of vectors include, but are not limited to, viral vectors, naked DNA or RNA expression vectors, plasmids, cosmids, or phage vectors.

In the present disclosure, the term "host cell" refers to any type of cell that may contain the nucleic acid or vector described herein. In exemplary aspects, the host cell is a eukaryotic cell, such as a plant, animal, fungal, or algae; or may be a prokaryotic cell, such as a bacterium or protozoa. In exemplary aspects, the host cell is a cell derived from or obtained from an individual, as described herein. In exemplary aspects, the host cell is derived from or obtained from a mammal.

In the present disclosure, methods and conditions for culturing the resulting transformants and for recovering the resulting antibody molecules are known to those skilled in the art and may be varied or optimized based on the specific expression vector and mammalian host cell used, in accordance with the present specification and methods known in the art.

In the present disclosure, application scenarios of detection for non-diagnostic and/or non-therapeutic purposes include, e.g., detecting the presence or absence of ADAM9 protein in a laboratory; or using it as a positive antibody to screen other ADAM9-targeting antibodies; or competing with other anti-ADAM9 antibodies for binding to determine whether there is competition between antibodies, *i.e.,* whether the antigenic epitopes are identical or similar, or the like.

In the present disclosure, the term "pharmaceutical composition" generally refers to a mixture containing one or more of the compounds described in the present disclosure or a physiologically/pharmaceutically acceptable salt or prodrug thereof, with other chemical components, as well as additional components such as physiologically/pharmaceutically acceptable carriers and excipients. The pharmaceutical composition may facilitate administration to an organism, promote absorption of active ingredients, and thus exert biological activity. The preparation of conventional pharmaceutical compositions can be found in the Chinese Pharmacopoeia. The pharmaceutical composition may be in the form of a sterile injectable aqueous or oily suspension for intramuscular and subcutaneous administration. The suspension may be formulated according to known techniques using the suitable dispersing or wetting agents and suspending agents as described above. The sterile injectable preparation may also be a sterile injectable solution or suspension prepared in a parenterally acceptable non-toxic diluent or solvent, such as a solution prepared in 1,3-butanediol. In addition, a sterile fixed oil may be conveniently used as a solvent or suspending medium. For example, any fixed oil including synthetic mono- or diglycerides may be used. In addition, fatty acids such as oleic acid may also be used in the preparation of injections.

In the present disclosure, the term "pharmaceutically acceptable salt" or "medicinal salt" generally refers to a salt of the compound or antibody-drug conjugate of the present disclosure, or a salt of the compound or antibody-drug conjugate described in the present disclosure. Such salts may be safe and/or effective when used in mammals and may have the desired biological activity. The compound or antibody-drug conjugate of the present disclosure may form a salt with an acid. Non-limiting examples of pharmaceutically acceptable salts include hydrochloride, hydrobromide, hydroiodide, sulfate, bisulfate, citrate, acetate, succinate, ascorbate, oxalate, nitrate, sorbate, hydrogen phosphate, dihydrogen phosphate, salicylate, hydrogen citrate, tartrate, maleate, fumarate, formate, benzoate, methanesulfonate, ethanesulfonate, benzenesulfonate, and*p*-toluenesulfonate.

In the present disclosure, the pharmaceutically acceptable carrier is any one of those conventionally used, and is limited only by physico-chemical considerations such as solubility and lack of reactivity with the ADAM9-targeting antibody, as well as by the route of administration. The pharmaceutically acceptable carriers described herein, such as vehicles, adjuvants, excipients, and diluents, are well known to those skilled in the art and are readily available to the public. In one aspect, the pharmaceutically acceptable carrier is one that is chemically inert to the active ingredient of the pharmaceutical composition and one which has no detrimental side effects or toxicity under the conditions of use. In some examples, the carrier does not produce adverse, allergic, or other untoward reactions when administered to an animal or a human. In some aspects, the pharmaceutical composition is free of pyrogens as well as other impurities that may be harmful to humans or animals. Pharmaceutically acceptable carriers include any and all solvents, dispersion media, coatings, antibacterial and antifungal agents, isotonic and absorption delaying agents, and the like; the use of which are well known in the art.

As used herein, the term "effective amount" refers to the amount of a drug or agent that elicits a biological or pharmaceutical response in a tissue, system, animal, or human that is being sought, for example, by a researcher or clinician. Furthermore, the term "therapeutically effective amount" refers to an amount that results in improved treatment, cure, prevention, or amelioration of a disease, disorder, or side effect, or a reduction in the rate of progression of a disease or condition, compared to a corresponding subject who has not received such amount. The term also includes within its scope amounts effective to enhance normal physiological function.

In the present disclosure, the term "cancer" refers to a malignant tumor, which is a disease caused by a malfunction in the mechanisms controlling cell growth and proliferation. The term "intestinal cancer" generally refers to carcinoma of large intestine, also known as "colorectal cancer", which denotes cancer originating from the epithelial tissue of the large intestine, including colon cancer and rectal cancer. The term "lung adenocarcinoma" refers to a malignant tumor originating from the epithelial tissue of the lung, which falls under the category of non-small cell lung cancer. The term "cancer with high expression of ADAM9" refers to a malignant tumor caused by abnormally high expression of ADAM9 in the body.

The present disclosure is further described below by way of examples, but the present disclosure is not thereby limited to the scope of the described examples. Experimental methods for which specific conditions are not indicated in the following examples are selected according to conventional methods and conditions, or according to the commodity instructions.

### Example 1: Re-humanization of MAB-A antibody

The purpose of re-humanizing the murine anti-ADAM9 monoclonal antibody MAB-A (disclosed in patent WO2018119196A1, whose heavy chain variable region comprising the sequence shown in SEQ ID NO: 7 and whose light chain variable region comprising the sequence shown in SEQ ID NO: 11) is to improve the solubility and reduce the hydrophobicity of the humanized version, thereby enhancing the conjugation yield and druggability as an ADC drug. The humanized version of MAB-A, hMAB-A (2I.2) (disclosed in patent WO2018119196A1, whose heavy chain comprising the sequence shown in SEQ ID NO: 52 and whose light chain comprising the sequence shown in SEQ ID NO: 68), which entered clinical research, exhibited issues with high hydrophobicity.

Re-humanization of the murine antibody was performed according to methods disclosed in literatures in the art, *i.e.,* replacing the murine constant domain with human constant domain and modifying the CDRs for affinity and druggability optimization. Human antibody sequences were selected based on the homology between the parental murine antibody MAB-A and the human antibody, and MAB-A was subjected to humanization. The specific method was as follows. The heavy and light chain variable region sequences of the murine antibody MAB-A were compared with the human antibody germline database to obtain a human germline template with high homology, based on the canonical structures of the VH/VL CDRs of MAB-A. The CDRs of the murine antibody were grafted onto the selected corresponding humanized template. Subsequently, based on the three-dimensional structure of the murine antibody, back mutations were made to embedded residues, residues directly interacting with the CDRs, and residues critical for the conformation of VL and VH. Additionally, chemically unstable amino acid residues in the CDRs were optimized. After expression testing and comparison of the number of back mutations, antibodies comprising the designed humanized heavy chain variable region (HCVR) and light chain variable region (LCVR) sequences were selected. The sequence numbering of the CDRs of the humanized antibody is shown in Table 1, the amino acid sequences of the CDRs are shown in Table 2, and the heavy and light chain variable region sequences of the humanized antibody are shown in Table 3.

**Table 1: Sequence numbering of CDRs of humanized antibodies**

| Humanized antibody | HCDR1 | HCDR2 | HCDR3 | LCDR1 | LCDR2 | LCDR3 |
|---|---|---|---|---|---|---|
| Ab1 | SEQ ID NO: 1 | SEQ ID NO: 5 | SEQ ID NO: 11 | SEQ ID NO: 12 | SEQ ID NO: 16 | SEQ ID NO: 19 |
| Ab2 | SEQ ID NO: 2 | SEQ ID NO: 6 | SEQ ID NO: 11 | SEQ ID NO: 12 | SEQ ID NO: 16 | SEQ ID NO: 19 |
| Ab3 | SEQ ID NO: 3 | SEQ ID NO: 7 | SEQ ID NO: 11 | SEQ ID NO: 12 | SEQ ID NO: 16 | SEQ ID NO: 19 |
| Ab4 | SEQ ID NO: 1 | SEQ ID NO: 7 | SEQ ID NO: 11 | SEQ ID NO: 12 | SEQ ID NO: 17 | SEQ ID NO: 19 |
| Ab5 | SEQ ID NO: 1 | SEQ ID NO: 7 | SEQ ID NO: 11 | SEQ ID NO: 12 | SEQ ID NO: 16 | SEQ ID NO: 19 |
| Ab6 | SEQ ID NO: 3 | SEQ ID NO: 8 | SEQ ID NO: 11 | SEQ ID NO: 12 | SEQ ID NO: 18 | SEQ ID NO: 19 |
| Ab7 | SEQ ID NO: 1 | SEQ ID NO: 5 | SEQ ID NO: 11 | SEQ ID NO: 13 | SEQ ID NO: 16 | SEQ ID NO: 19 |
| Ab8 | SEQ ID NO: 3 | SEQ ID NO: 7 | SEQ ID NO: 11 | SEQ ID NO: 12 | SEQ ID NO: 16 | SEQ ID NO: 19 |
| Ab9 | SEQ ID NO: 2 | SEQ ID NO: 9 | SEQ ID NO: 11 | SEQ ID NO: 12 | SEQ ID NO: 16 | SEQ ID NO: 19 |
| Ab10 | SEQ ID NO: 1 | SEQ ID NO: 7 | SEQ ID NO: 11 | SEQ ID NO: 12 | SEQ ID NO: 16 | SEQ ID NO: 19 |
| Ab11 | SEQ ID NO: 1 | SEQ ID NO: 7 | SEQ ID NO: 11 | SEQ ID NO: 12 | SEQ ID NO: 18 | SEQ ID NO: 19 |
| Ab12 | SEQ ID NO: 2 | SEQ ID NO: 9 | SEQ ID NO: 11 | SEQ ID NO: 12 | SEQ ID NO: 16 | SEQ ID NO: 19 |
| Ab13 | SEQ ID NO: 1 | SEQ ID NO: 7 | SEQ ID NO: 11 | SEQ ID NO: 13 | SEQ ID NO: 16 | SEQ ID NO: 19 |
| Ab14 | SEQ ID NO: 3 | SEQ ID NO: 8 | SEQ ID NO: 11 | SEQ ID NO: 14 | SEQ ID NO: 18 | SEQ ID NO: 19 |
| Ab15 | SEQ ID NO: 1 | SEQ ID NO: 7 | SEQ ID NO: 11 | SEQ ID NO: 12 | SEQ ID NO: 16 | SEQ ID NO: 19 |
| Ab16 | SEQ ID NO: 3 | SEQ ID NO: 8 | SEQ ID NO: 11 | SEQ ID NO: 12 | SEQ ID NO: 16 | SEQ ID NO: 19 |
| Ab17 | SEQ ID NO: 1 | SEQ ID NO: 7 | SEQ ID NO: 11 | SEQ ID NO: 12 | SEQ ID NO: 16 | SEQ ID NO: 19 |
| Ab18 | SEQ ID NO: 4 | SEQ ID NO: 10 | SEQ ID NO: 11 | SEQ ID NO: 12 | SEQ ID NO: 16 | SEQ ID NO: 19 |
| Ab19 | SEQ ID NO: 2 | SEQ ID NO: 6 | SEQ ID NO: 11 | SEQ ID NO: 15 | SEQ ID NO: 16 | SEQ ID NO: 19 |
| Ab20 | SEQ ID NO: 1 | SEQ ID NO: 7 | SEQ ID NO: 11 | SEQ ID NO: 12 | SEQ ID NO: 17 | SEQ ID NO: 19 |

**Table 2: Amino acid sequences of CDRs of humanized antibodies**

| No. | Sequence |
|---|---|
| VH CDR1 SEQ ID NO: 1 | LYWMN |
| VH CDR1 SEQ ID NO: 2 | LYWMD |
| VH CDR1 SEQ ID NO: 3 | LYWMH |
| VH CDR1 SEQ ID NO: 4 | LYWME |
| VH CDR1 shared sequence shown in SEQ ID NO: 20 | LYWMX₁ |
| VH CDR2 SEQ ID NO: 5 | RIIPIFGHTDYNEKFKD |
| VH CDR2 SEQ ID NO: 6 | DIIPIFGHTKYEEKFRD |
| VH CDR2 SEQ ID NO: 7 | RIIPIFGHTKYNEKFKD |
| VH CDR2 SEQ ID NO: 8 | RIIPIFGHTKYNEKFKN |
| VH CDR2 SEQ ID NO: 9 | DIIPIFGHTKYNEKFKD |
| VH CDR2 SEQ ID NO: 10 | DIIPIFGHTKYNEKFRD |
| VH CDR2 shared sequence shown in SEQ ID NO: 21 | X₂IIPIFGHTX₃YX₄EKFX₅X₆ |
| VH CDR3 SEQ ID NO: 11 | GGYYYYPREGFLDY |
| VL CDR1 SEQ ID NO: 12 | RSSQSVDYSGDSYMN |
| VL CDR1 SEQ ID NO: 13 | RSDQSVDYSGDSYMN |
| VL CDR1 SEQ ID NO: 14 | RASQSVDYSGDSYMN |
| VL CDR1 SEQ ID NO: 15 | RASQSVDYSGDSYMD |
| VL CDR2 SEQ ID NO: 16 | AASDRES |
| VL CDR2 SEQ ID NO: 17 | AASDLES |
| VL CDR2 SEQ ID NO: 18 | AASDRDS |
| VL CDR3 SEQ ID NO: 19 | QQSREDPFT |

In Table 2, the CDRs of the antibodies are defined according to the Kabat numbering system, wherein X₁ may be amino acid N, D, H, or E; X₂ may be amino acid D or R; X₃ may be amino acid K or D; X₄ may be amino acid N or E; X₅ may be amino acid R or K; X₆ may be amino acid D or N.

**Table 3: Heavy and light chain variable region sequences of humanized antibodies**

| Humanized antibody variable regions | Name | Sequence | SEQ ID NO: |
|---|---|---|---|
| Ab1-V | HCVR | QVQLQESGAEVKRPGSSVKVSCKASGFTFPLYWMNWV RQRPGQGLEWMGRIIPIFGHTDYNEKFKDRATITRDESS NTAYMELSRLRSEDSAVYYCARGGYYYYPREGFLDYW GQGTSVTVSS | 22 |
| | LCVR | DIVMTQSPASLSVSPGERASISCRSSQSVDYSGDSYMNW YQQKPGQSPRLLIYAASDRESGVPDRFSGSGSGTDFTLK ISRVEEEDAGTYYCQQSREDPFTFGQGTKVDIK | 23 |
| Ab2-V | HCVR | EVQLQESGSEVRRPSESVKMSCKDSGFTFPLYWMDWV RQRQGKSLEWMGDIIPIFGHTKYEEKFRDRATMDRDES SDTAYMELRRLRSDDSAVYYCARGGYYYYPREGFLDY WGQGTSVTVSS | 24 |
| | LCVR | DIVMTQSPDSLSVSPGERATINCRSSQSVDYSGDSYMN WYQQKPGQSPRLLIYAASDRESGVPDRFSGSGSGTDFTL KISRVEEEDAATYYCQQSREDPFTFGQGTRVEIK | 25 |
| Ab3-V | HCVR | QVQLQESGAELKKPGASVKVSCKASGFTFPLYWMHWV RQRTGRGLEWMGRIIPIFGHTKYNEKFKDRATITRDESS NTAYMELSSLTSEDSAVYYCARGGYYYYPREGFLDYW GQGTSVTVSS | 26 |
| | LCVR | DIVMTQSPDSLSASPGERATLSCRSSQSVDYSGDSYMN WYQQKPGQSPKLLLYAASDRESGVPDRFSGSGSGTDFT LNINRVEEEDEATYYCQQSREDPFTFGQGTRVEIK | 27 |
| Ab4-V | HCVR | QVQLQESGAEVKRPGSSVKVSCKASGFTFPLYWMNWV RQRPGRGLEWMGRIIPIFGHTKYNEKFKDRATMTRDKS SSTAYMELSSLTSDDSAVYYCARGGYYYYPREGFLDY WGQGTSVTVSS | 28 |
| | LCVR | DIVMTQSPSSLSVSPGERATINCRSSQSVDYSGDSYMNW YQQKPGQAPRLLIYAASDLESGVPDRFSGSGSGTDFTLN INGVEEEDAATYYCQQSREDPFTFGQGTKVEIK | 29 |
| Ab5-V | HCVR | QVQLQESGAEVKRPGSSVKVSCKASGFTFPLYWMNWV RQRPGRGLEWMGRIIPIFGHTKYNEKFKDRATMTRDKS SSTAYMELSSLTSDDSAVYYCARGGYYYYPREGFLDY WGQGTSVTVSS | 28 |
| | LCVR | DIVMTQSPDSLSVSPGERATINCRSSQSVDYSGDSYMN WYQQKPGQSPKLLIYAASDRESGVPDRFSGSGSGTDFT LNISRVEEEDAATYYCQQSREDPFTFGQGTRVEIK | 30 |
| Ab6-V | HCVR | QVQLQQPGTEVVKPGASVKLSCKASGFTFPLYWMHWV RQRPGRGLEWIGRIIPIFGHTKYNEKFKNKATLTADESSS TAYMELRSLTSDDSAVYYCARGGYYYYPREGFLDYWG QGTSVTVSS | 31 |
| | LCVR | DIVMTQSPASLSVSPGERASISCRSSQSVDYSGDSYMNW YQQKPGQSPRLLIYAASDRDSGVPDRFSGSGSGTDFTLK ISRVEEEDAATYYCQQSREDPFTFGQGTRVEVK | 32 |
| Ab7-V | HCVR | QVQLQESGAEVKRPGSSVKVSCKASGFTFPLYWMNWV RQRPGQGLEWMGRIIPIFGHTDYNEKFKDRATITRDESS NTAYMELSRLRSEDSAVYYCARGGYYYYPREGFLDYW GQGTSVTVSS | 22 |
| | LCVR | DIVMTQSPASLSVSPGERASINCRSDQSVDYSGDSYMN WYQQKPGKSPRLLIYAASDRESGVPDRFSGSGSGTDFTL NINSVEEEDAGTYYCQQSREDPFTFGQGTRVDIK | 33 |
| Ab8-V | HCVR | QVQLQQPGAELKKPGASVKVSCKTSGFTFPLYWMHWV KQRPGRGLEWMGRIIPIFGHTKYNEKFKDKATLTRDESS STAYMELSSLTSEDSAVYYCARGGYYYYPREGFLDYW GQGTSVTVSS | 34 |
| | LCVR | DIVMTQSPSSLSASPGERASISCRSSQSVDYSGDSYMNW YQQRPGQSPRLLIYAASDRESGVPDRFSGSGSGTDFTLK ISDVEEEDAATYYCQQSREDPFTFGQGTRVEIK | 35 |
| Ab9-V | HCVR | QVQLQQSGTEVKRPGSSVKMSCKDSGFTFPLYWMDWV KQRHGRRLEWMGDIIPIFGHTKYNEKFKDRATMTTDES SSTAYMELRSLRSDDSAVYYCARGGYYYYPREGFLDY WGQGTSVTVSS | 36 |
| | LCVR | DIVMTQSPDSLSVSPGERATINCRSSQSVDYSGDSYMN WYQQKPGQSPKLLIYAASDRESGVPDRFSGSGSGTDFT LNISRVEEEDAATYYCQQSREDPFTFGQGTRVEIK | 30 |
| Ab10-V | HCVR | QVQLQESGAELKRPGSSVKVSCKASGFTFPLYWMNWV RQRPGRGLEWMGRIIPIFGHTKYNEKFKDRATLTADESS STAYMELSSLRSDDSAVYYCARGGYYYYPREGFLDYW GQGTSVTVSS | 37 |
| | LCVR | DIVMTQSPASLSVSPGERASISCRSSQSVDYSGDSYMNW YQQKPGQSPRLLIYAASDRESGVPARFSGSKSGTDFTLK INRVEEEDAGTYYCQQSREDPFTFGQGTRVEIK | 38 |
| Ab11-V | HCVR | QVQLQQSGAEVKRPGASVKMSCKASGFTFPLYWMNW VRQRPGRGLEWIGRIIPIFGHTKYNEKFKDRATITADESS DTAYMELSSLRSDDSAVYYCARGGYYYYPREGFLDYW GQGTSVTVSS | 39 |
| | LCVR | DIVMTQSPASLSVSPGERASISCRSSQSVDYSGDSYMNW YQQKPGQSPRLLIYAASDRDSGVPDRFSGSGSGTDFTLK ISRVEEEDAATYYCQQSREDPFTFGQGTRVEVK | 32 |
| Ab12-V | HCVR | QVQLQQSGTEVKRPGSSVKMSCKDSGFTFPLYWMDWV KQRHGRRLEWMGDIIPIFGHTKYNEKFKDRATMTTDES SSTAYMELRSLRSDDSAVYYCARGGYYYYPREGFLDY WGQGTSVTVSS | 36 |
| | LCVR | DIVMTQSPDSLSASPGERATLSCRSSQSVDYSGDSYMN WYQQKPGQSPKLLLYAASDRESGVPDRFSGSGSGTDFT LNINRVEEEDEATYYCQQSREDPFTFGQGTRVEIK | 27 |
| Ab13-V | HCVR | QVQLQESGAEVKRPGSSVKVSCKASGFTFPLYWMNWV RQRPGRGLEWMGRIIPIFGHTKYNEKFKDRATMTRDKS SSTAYMELSSLTSDDSAVYYCARGGYYYYPREGFLDY WGQGTSVTVSS | 28 |
| | LCVR | DIVMTQSPDSLSVSPGERATINCRSDQSVDYSGDSYMN WYQQRPGQAPRLLIYAASDRESGTPDRFSGSGSGTDFTL NISRVEEEDAATYYCQQSREDPFTFGQGTRVEIK | 40 |
| Ab14-V | HCVR | QVQLQQPGTEVVKPGASVKLSCKASGFTFPLYWMHWV RQRPGRGLEWIGRIIPIFGHTKYNEKFKNKATLTADESSS TAYMELRSLTSDDSAVYYCARGGYYYYPREGFLDYWG QGTSVTVSS | 31 |
| | LCVR | DIVMTQSPASLSVSPGERATLSCRASQSVDYSGDSYMN WYQQKPGQAPRLLIYAASDRDSGVPDRFSGSGSGTDFT LKINRVEEEDAATYYCQQSREDPFTFGQGTRVEIK | 41 |
| Ab15-V | HCVR | QVQLQQPGAELKKPGASVKVSCKASGFTFPLYWMNW VRQRPGRGLEWIGRIIPIFGHTKYNEKFKDRATMTADES SSTAYMELSSLTSEDSAVYYCARGGYYYYPREGFLDY WGQGTSVTVSS | 42 |
| | LCVR | DIVMTQSPDSLSVSPGERATINCRSSQSVDYSGDSYMN WYQQKPGQSPRLLIYAASDRESGVPDRFSGSGSGTDFTL KISNVEEEDAATYYCQQSREDPFTFGQGTRVEIK | 43 |
| Ab16-V | HCVR | QVQLQQPGTEVVKPGASVKLSCKASGFTFPLYWMHWV RQRPGRGLEWIGRIIPIFGHTKYNEKFKNKATLTADESSS TAYMELRSLTSDDSAVYYCARGGYYYYPREGFLDYWG QGTSVTVSS | 31 |
| | LCVR | DIVMTQSPASLSVSPGERASISCRSSQSVDYSGDSYMNW YQQKPGQSPRLLIYAASDRESGVPDRFSGSGSGTDFTLK ISRVEEEDAGTYYCQQSREDPFTFGQGTKVDIK | 23 |
| Ab17-V | HCVR | QVQLQQSGAEVKRPGASVKMSCKASGFTFPLYWMNW VRQRPGRGLEWIGRIIPIFGHTKYNEKFKDRATITADESS DTAYMELSSLRSDDSAVYYCARGGYYYYPREGFLDYW GQGTSVTVSS | 39 |
| | LCVR | DIVMTQSPDSLSASPGERATLSCRSSQSVDYSGDSYMN WYQQKPGQSPKLLLYAASDRESGVPDRFSGSGSGTDFT LNINRVEEEDEATYYCQQSREDPFTFGQGTRVEIK | 27 |
| Ab18-V | HCVR | QMQLQESGAEVKKPSRSVRMSCKGSGFTFPLYWMEWV RQRPGKGLEWMGDIIPIFGHTKYNEKFRDRATMTRDES SNTAYMELRSLRSDDSAVYYCARGGYYYYPREGFLDY WGQGTSVTVSS | 44 |
| | LCVR | DIVMTQSPDSLSVSPGQRASINCRSSQSVDYSGDSYMN WYQQRPGQAPRLLIYAASDRESGVPDRFSGSGSGTDFT LRINRVEEEDAGTYYCQQSREDPFTFGQGTRVDIK | 45 |
| Ab19-V | HCVR | EVQLQESGSEVRRPSESVKMSCKDSGFTFPLYWMDWV RQRQGKSLEWMGDIIPIFGHTKYEEKFRDRATMDRDES SDTAYMELRRLRSDDSAVYYCARGGYYYYPREGFLDY WGQGTSVTVSS | 24 |
| | LCVR | DIVMTQSPDSLSASVGDRASISCRASQSVDYSGDSYMD WYQQKPGQSPRLLIYAASDRESGVPDRFSGSGSGTDFTL KINRVEEEDAATYYCQQSREDPFTFGQGTRVEIK | 46 |
| Ab20-V | HCVR | QVQLQQSGAEVRRPGSSVKMSCKDSGFTFPLYWMNW VRQRPGRGLEWMGRIIPIFGHTKYNEKFKDRATMTADE SSSTAYMELSSLRSDDSAVYYCARGGYYYYPREGFLDY WGQGTSVTVSS | 47 |
| | LCVR | DIVMTQSPSSLSVSPGERATINCRSSQSVDYSGDSYMNW YQQKPGQAPRLLIYAASDLESGVPDRFSGSGSGTDFTLN INGVEEEDAATYYCQQSREDPFTFGQGTKVEIK | 29 |

The underlined amino acid sequences in Table 3 indicate the CDRs defined according to the Kabat numbering system.

The designed heavy and light chain variable region sequences were connected to the heavy and light chain constant region sequences of human antibodies, respectively. Exemplarily, the antibody heavy chain constant region was selected from the human IgG1 heavy chain constant region whose sequence is shown in SEQ ID NO: 48; the antibody light chain constant region was selected from the human κ chain constant region whose sequence is shown in SEQ ID NO: 49. The antibody constant region sequences are shown in Table 4.

**Table 4: Sequence numbering of constant regions**

| Name | Sequence | No. |
|---|---|---|
| Human IgG1 heavy chain constant region | | SEQ ID NO: 48 |
| Human light chain (K chain) constant region | | SEQ ID NO: 49 |

The amino acid sequence of the heavy chain of Ab16 is (SEQ ID NO: 50):

QVQLQQPGTEVVKPGASVKLSCKASGFTFPLYWMHWVRQRPGRGLEWIGR
IIPIFGHTKYNEKFKNKATLTADESSSTAYMELRSLTSDDSAVYYCARGGYYYYPRE
GFLDYWGQGTSVTVSSASTKGPSVFPLAPSSKSTSGGTAALGCLVKDYFPEPVTVSW

NSGALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSL
SLSPGK

The amino acid sequence of the light chain of Ab16 is (SEQ ID NO: 51):

DIVMTQSPASLSVSPGERASISCRSSQSVDYSGDSYMNWYQQKPGQSPRLLI
YAASDRESGVPDRFSGSGSGTDFTLKISRVEEEDAGTYYCQQSREDPFTFGQGTKVDI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC

The amino acid sequence of the heavy chain of Ab15 is (SEQ ID NO: 52):

QVQLQQPGAELKKPGASVKVSCKASGFTFPLYWMNWVRQRPGRGLEWIGR
IIPIFGHTKYNEKFKDRATMTADESSSTAYMELSSLTSEDSAVYYCARGGYYYYPRE
GFLDYWGQGTSVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSL
SLSPGK

The amino acid sequence of the light chain of Ab15 is (SEQ ID NO: 53):

DIVMTQSPDSLSVSPGERATINCRSSQSVDYSGDSYMNWYQQKPGQSPRLLI
YAASDRESGVPDRFSGSGSGTDFTLKISNVEEEDAATYYCQQSREDPFTFGQGTRVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC

### Example 2: Hydrophobicity assay of humanized anti-ADAM9 antibody

Experimental procedure: The hydrophilicity/hydrophobicity of different antibodies was evaluated using HIC-HPLC analysis. The stronger the hydrophilicity of the antibody, the weaker the hydrophobic interaction with the HIC ligand (butyl group), resulting in a relatively shorter retention time. All antibodies were exchanged into 200 mM Arg. SSC pH 5.5 buffer and adjusted to a concentration of 5 mg/mL. The hydrophobicity of each re-humanized anti-ADAM9 antibody and hMAB-A (21.2) antibody was tested using the following chromatographic method (Table 5). The experimental results are as shown in Table 6.

**Table 5: Chromatographic method**

| Chromatographic column | MabPac^{™} HIC-Butyl 5 µm 4.6 × 100 mm | | | |
|---|---|---|---|---|
| Column temperature (°C) | 30 | | | |
| Sample chamber temperature (°C) | 8 | | | |
| Detection wavelength (nm) | 280 | | | |
| Injection volume (µL) | 10 | | | |
| Mobile phase | MPA: 1.5 M (NH₄)₂SO₄ + 50 mM potassium phosphate (pH 7.0) | | | |
| | MPB: 50 mM sodium phosphate (pH 7.0) + 25% isopropanol (v/v) | | | |
| Elution conditions | Time (min) | %A | %B | Flow rate (mL/min) |
| | 0.0 | 100.0 | 0.0 | 1.0 |
| | 2.0 | 95.0 | 5.0 | 1.0 |
| | 22.0 | 5.0 | 95.0 | 1.0 |
| | 24.0 | 0.0 | 100.0 | 1.0 |
| | 26.0 | 100.0 | 0.0 | 1.0 |
| | 30.0 | 100.0 | 0.0 | 1.0 |

**Table 6: Chromatographic experimental results**

| No. | HIC retention time (min) | Trend of retention time (vs. control group) |
|---|---|---|
| hMAB-A (21.2) | 14.372 | / |
| Ab1 | 12.380 | -10.73% |
| Ab4 | 12.714 | -11.54% |
| Ab6 | 12.478 | -13.18% |
| Ab7 | 12.865 | -10.49% |
| Ab 10 | 12.534 | -12.79% |
| Ab11 | 12.449 | -13.38% |
| Ab14 | 12.654 | -11.95% |
| Ab15 | 12.43 | -13.51% |
| Ab16 | 12.623 | -12.17% |
| Ab17 | 12.901 | -10.24% |

Experimental conclusion: Based on the above experimental results, it can be observed that after structural design and modification, the hydrophilicity of the modified antibodies was significantly improved, enhancing the stability and druggability of the antibody molecules.

### Example 3: Production and expression of humanized anti-ADAM9 antibody

The light chain variable region amino acid sequences of the 20 antibodies from Example 1, Ab1 to Ab20, and hMAB-A (2I.2) were each connected to the light chain κ constant region amino acid sequence (SEQ ID NO: 49), while the heavy chain variable region amino acid sequences were each connected to the IgG1 heavy chain constant region amino acid sequence (SEQ ID NO: 48). cDNA was synthesized through codon optimization and ligated into PTT5 plasmid (outsourced to GenScript Biotech Corporation, Nanjing). All 21 antibodies used the same signal peptide sequence. The PTT5 plasmids corresponding to the heavy and light chains of each antibody were co-transfected into CHO cells using PEI MAX 40K. After 5 days of culture, the cell culture supernatant was collected, and the antibody components were purified by Protein A (MabSelect^{™} PrismA) chromatography, followed by antibody quantification by OD280nm (outsourced to GenScript Biotech Corporation, Nanjing).

The 20 antibodies from Example 1 were expressed in a 4 mL system, with the expression levels of each antibody shown in Table 7 below.

**Table 7: Antibody yield expressed in 4 mL system**

| Antibody | Expression level in 4 mL system (mg) |
|---|---|
| Ab01 | 0.136 |
| Ab02 | 0.016 |
| Ab03 | 0.04 |
| Ab04 | 0.152 |
| Ab05 | 0.096 |
| Ab06 | 0.328 |
| Ab07 | 0.192 |
| Ab08 | 0.056 |
| Ab09 | 0.008 |
| Ab10 | 0.392 |
| Ab11 | 0.272 |
| Ab12 | 0.008 |
| Ab13 | 0.048 |
| Ab14 | 0.266 |
| Ab15 | 0.344 |
| Ab16 | 0.936 |
| Ab17 | 0.144 |
| Ab18 | 0.008 |
| Ab19 | 0.008 |
| Ab20 | 0.064 |
| hMAB-A (2I.2) | 0.132 |

Experimental conclusion: The expression levels of Ab06, Ab10, Ab15, and Ab16 were approximately 3-fold higher than that of hMAB-A (2I.2).

The four antibodies, Ab10, Ab15, Ab16, and hMAB-A (2I.2), were further expressed in a 500 mL system, with the expression levels of each antibody shown in Table 8 below.

**Table 8: Antibody yield expressed in 500 mL system**

| Antibody | Expression level in 4 mL system (mg) | SEC purity | Proportion of high molecular weight polymers |
|---|---|---|---|
| Ab10 | 33 mg | 96% | 2% |
| Ab15 | 61 mg | 97% | 2% |
| Ab16 | 112 mg | 98% | 1% |
| hMAB-A (21.2) | 20 mg | 97% | 2% |

Experimental conclusion: The expression levels of Ab15 and Ab16 were significantly higher than that of hMAB-A (2I.2), and SEC-HPLC analysis showed a lower proportion of high molecular weight polymers, indicating better druggability compared to hMAB-A (2I.2).

### Example 4: In vitro binding activity assay of humanized anti-ADAM9 antibody

### 4.1 In vitro indirect ELISA binding assay

Human ADAM-His protein (ACROBiosystems, Cat. No. AD9-H52H7) was diluted to a concentration of 1 µg/mL in PBS (pH 7.4) and added to a 96-well high-affinity ELISA plate at 100 µL/well, followed by incubation in a 4°C freezer overnight (16 to 20 hours). The plate was washed three times with PBST (PBS (pH 7.4) containing 0.05% Tween-20), followed by addition of 200 µL/well of blocking buffer containing 1% bovine serum albumin (BSA) diluted in PBST, and incubated at 37°C for 1 hour for blocking. After blocking, the blocking buffer was discarded, and the plate was washed once with PBST buffer. The test antibody was diluted in PBST containing 1% BSA with a 3-fold serial dilution starting at 15 µg/mL across 11 doses, and added to the ELISA plate at 100 µL/well, followed by incubation at 37°C for 1 hour. After incubation, the plate was washed three times with PBST, followed by addition of 100 µL/well of HRP-labeled goat anti-human secondary antibody (Rockland, Cat. No. 609-103-123) diluted in PBST containing 1% BSA, and incubated at room temperature for 45 minutes. The plate was washed six times with PBST, followed by addition of 100 µL/well of TMB chromogenic substrate (Suzhou Yacoo Chemical Reagent Co., Ltd., Cat. No. S0025), and incubated at room temperature in the dark for 10 minutes. The reaction was terminated by adding 50 µL/well of 1 M HCl, and the absorbance was measured at 450 nm using a microplate reader (Thermo, Ascent) for data analysis. A concentration-OD curve was generated to analyze the results, as shown in Table 9 below. The results demonstrated that the antibody of the present disclosure exhibited good affinity for human ADAM9 antigen.

**Table 9: Affinity (EC₅₀) of re-humanized antibodies for human ADAM9 antigen**

| **Antibody** | **Top (OD450)** | **EC₅₀ (nM)** |
|---|---|---|
| Ab1 | 3.119 | 0.052 |
| Ab4 | 3.060 | 0.055 |
| Ab7 | 3.308 | 0.060 |
| Ab10 | 3.399 | 0.059 |
| Ab15 | 3.276 | 0.049 |
| Ab16 | 3.361 | 0.042 |

### 4.2 Biacore affinity assay

**Chip preparation:** The mouse anti-human IgG (Fc) antibody (Cat. No. 29234600, Cytiva) was diluted to 25 µg/mL in immobilization buffer (10 mM sodium acetate, pH 5.0), specifically by adding 950 µL of the immobilization buffer to 50 µL of the mouse anti-human IgG (Fc) antibody. The dilution was used for immobilization in eight channels. First, the surface of the CM5 chip was activated with 400 mM EDC and 100 mM NHS at a flow rate of 10 µL/min for 420 seconds. Then, 25 µg/mL of the mouse anti-human IgG (Fc) antibody was injected into the test channels at a flow rate of 10 µL/min for approximately 360 seconds, with the level of immobilization at approximately 7000 to 14000 RU. Finally, the chip was blocked with 1 M ethanolamine at a flow rate of 10 µL/min for 420 seconds. The reference channels underwent the same procedures as the test channels.

**Ligand capture:** Antibodies Ab10, Ab15, Ab16, and hMAB-A (21.2) were diluted to 4 µg/mL in running buffer (containing 10 mM N-(2-hydroxyethyl)piperazine-N-2-ethanesulfonic acid (HEPES), 150 mM sodium chloride (NaCl), 3 mM ethylenediaminetetraacetic acid (EDTA), 0.005% Tween-20, pH adjusted to 7.4) and injected into the test channels for human IgG (Fc) capture at a flow rate of 10 µL/min at approximately 200 RU. No ligand capture was performed in the reference channels.

**Analyte multi-cycle analysis:** Human and cynomolgus ADAM9 proteins (human ADAM9 protein purchased from ACRO, Cat. No. AD9-H52H7; monkey ADAM9 protein purchased from ACRO, Cat. No. AD9-C52H7) were diluted 2-fold in running buffer (Table 10). The diluted human or cynomolgus ADAM9 protein was sequentially injected into the test channels and the reference channels at a flow rate of 30 µL/min, with corresponding association and dissociation times. Both the association and dissociation steps were performed in the running buffer. After each concentration analysis, the chip was regenerated with 3 M magnesium chloride at a flow rate of 20 µL/min for 30 seconds to wash away the ligand and any undissociated analyte. For the next concentration analysis, the test channels required re-capture of the same amount of ligand.

**Table 10: Concentration gradients and association/dissociation times for affinity testing of anti-ADAM9 antibody with human and cynomolgus ADAM9 proteins**

| **Ligand** | **Analyte** | **Analyte concentration (nM)** | **Association time (s)** | **Dissociation time (s)** |
|---|---|---|---|---|
| Human ADAM9 | Anti-ADAM9 antibody | 1.563-200 | 120 | 300 |
| Cynomolgus ADAM9 | Anti-ADAM9 antibody | 3.125-200 | 120 | 300 |

**Data analysis:** The KD values were calculated for each sample using Biacore 8K analysis software Biacore Insight Evaluation Software. The reference channels were used for background subtraction.

The results of *in vitro* indirect ELISA binding assay and Biacore method are shown in Table 11 and Table 12:

**Table 11: Affinity of antibodies to human ADAM9 antigen**

| **Antibody** | | **Biacore** | |
|---|---|---|---|
| | **ka (1/Ms)** | **kd (1/s)** | **KD (M)** |
| Ab10 | 1.46E+05 | 2.55E-03 | 1.75E-08 |
| Ab15 | 1.73E+05 | 6.97E-04 | 4.03E-09 |
| Ab16 | 1.63E+05 | 8.16E-04 | 5.01E-09 |

**Table 12: Affinity of antibodies to monkey ADAM9 antigen**

| **Antibody** | | **Biacore** | |
|---|---|---|---|
| | **ka (1/Ms)** | **kd (1/s)** | **KD (M)** |
| Ab15 | 4.87E+04 | 4.61E-04 | 9.47E-09 |
| Ab16 | 6.13E+04 | 5.28E-04 | 8.61E-09 |

ELISA and Biacore results demonstrated that the antibodies Ab15 and Ab16 exhibited good affinity to human and monkey ADAM9 antigens.

### Example 5: Internalization capacity of humanized anti-ADAM9 antibody

A binding plate was set up. Calu-3 human lung cancer cells with high expression of ADAM9 (purchased from the Cell Bank of the Chinese Academy of Sciences, Cat. No. TCHu157) were digested with trypsin and collected by centrifugation, and the cell density was adjusted with FACS buffer (1× PBS containing 2% FBS). The cells were then distributed into a 96-well U-bottom plate at 1 × 10⁵ cells per well. The plate was centrifuged at 1200 g for 5 minutes, and the supernatant was discarded. 100 µL of antibody solution serially diluted in FACS buffer (with 10-fold dilutions starting at a concentration of 100 nM across 8 concentration points, including a 0 nM point) was added to each well, followed by incubation at 4°C for 1 hour. The plate was centrifuged at 1200 g for 5 minutes, and the supernatant was discarded. The cells were washed twice with PBS, then resuspended in 100 µL/well of FITC-conjugated goat anti-human IgG H&L antibody (Abcam, Cat. No. ab6866) prepared in FACS buffer, followed by incubation at 4°C for 1 hour. The plate was centrifuged at 1200 g for 5 minutes, and the supernatant was discarded. The cells were washed twice with PBS and resuspended in PBS, and the mean fluorescence intensity (MFI_{binding well}) of the cells under each antibody concentration was measured by flow cytometry.

Simultaneously, an endocytosis plate was set up. Calu-3 cells were digested with trypsin and collected by centrifugation, and the cell density was adjusted with FACS buffer. The cells were then distributed into a 96-well U-bottom plate at 1 × 10⁵ cells per well. The plate was centrifuged at 1200 g for 5 minutes, and the supernatant was discarded. 100 µL of antibody solution serially diluted in FACS buffer (with 10-fold dilutions starting at a concentration of 100 nM across 8 concentration points, including a 0 nM point) was added to each well, followed by incubation at 37°C for 16 hours. The plate was centrifuged at 1200 g for 5 minutes, and the supernatant was discarded. The cells were washed twice with PBS, then resuspended in 100 µL/well of FITC-conjugated goat anti-human IgG H&L antibody prepared in FACS buffer, followed by incubation at 4°C for 1 hour. The plate was centrifuged at 1200 g for 5 minutes, and the supernatant was discarded. The cells were washed twice with PBS and resuspended in PBS, and the mean fluorescence intensity (MFI_{endocytosis well}) of the cells under each antibody concentration was measured by flow cytometry.

The internalization rate of the antibody at each concentration was calculated using the following formula: internalization rate % = (MFI_{binding well} - MFI_{endocytosis well}) / MFI_{binding well} × 100%, and a concentration-response curve was plotted to analyze the EC₅₀ concentration for antibody internalization. The results are shown in Table 13.

**Table 13: Internalization rate of antibodies**

| Antibody | EC₅₀ for internalization | Maximum internalization rate (%) |
|---|---|---|
| Ab10 | 0.16 nM | 81.28% |
| Ab15 | 0.17 nM | 81.79% |
| Ab16 | 0.12 nM | 83.35% |

The results demonstrated that the antibodies Ab10, Ab15, and Ab16 all exhibited high internalization rates for cells expressing ADAM9.

### Example 6: Synthesis of linker-payload

### Linker-payload X1:

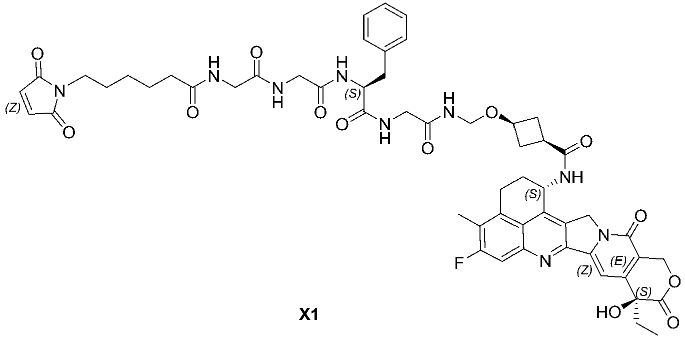

### Synthetic route:

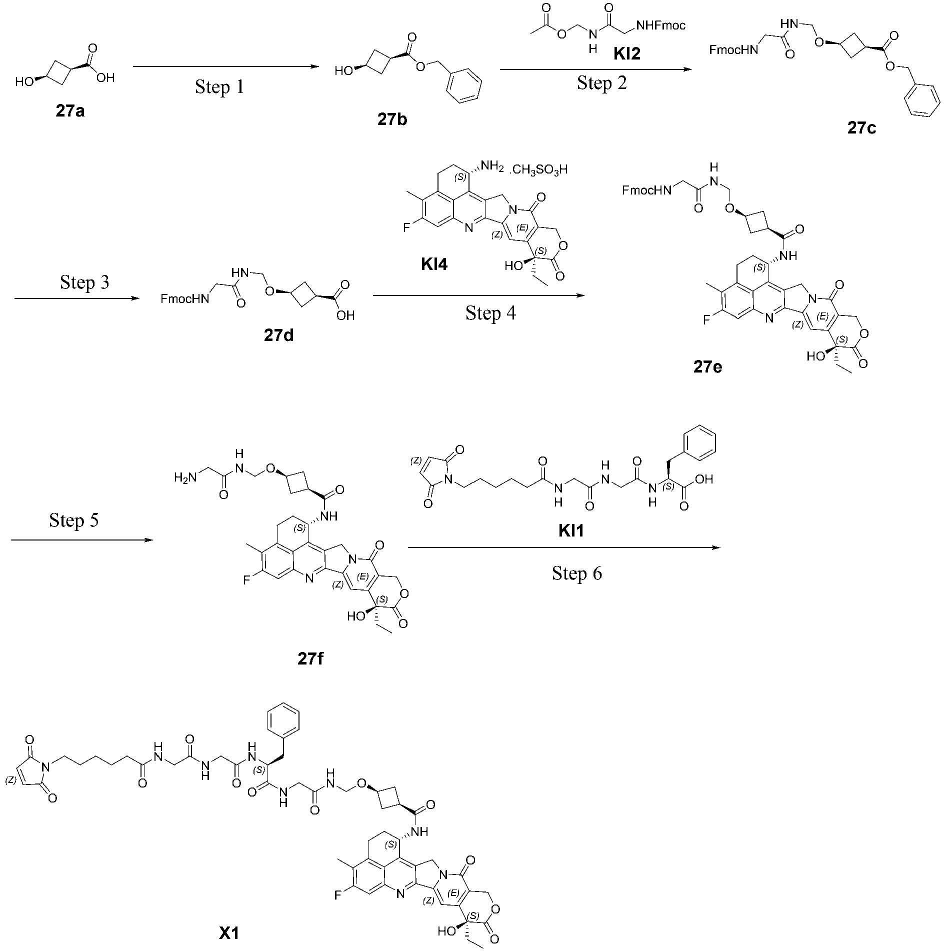

### Step 1:

To a solution of 27a (5.00 g, 43.0 mmol) and NaHCO₃ (10.9 g, 129 mmol) in DMF (50 mL) was added dropwise benzyl bromide (11.0 g, 64.6 mmol) under a nitrogen atmosphere, and the mixture was reacted at 25°C for 17 hours. TLC (PE/EA = 2/1) indicated that the reaction was complete. The reaction mixture was added to 500 mL of water and extracted twice with EA (250 mL). The phases were separated, and the organic phase was washed with saturated sodium chloride aqueous solution (500 mL), dried over anhydrous Na₂SO₄, and concentrated. The residue was purified by column chromatography (PE: EA = 3:2) to obtain 5.1 g of colorless liquid with a yield of 57.1%.

### Step 2:

To a solution of KI2 (4.00 g, 10.9 mmol) and TsOH (800 mg, 4.65 mmol) in THF (30 mL) was added dropwise a solution of 27b (4.50 g, 21.8 mmol) in THF (10 mL) at 0°C under a nitrogen atmosphere, and the mixture was reacted at 25°C for 2 hours. TLC (PE/EA = 1/2) indicated that the reaction was complete. The reaction mixture was added to 200 mL of water and extracted twice with EA (200 mL). The phases were separated, and the organic phase was dried over anhydrous Na₂SO₄ and concentrated. The residue was purified by column chromatography (PE/EA = 3/2) to obtain 1.56 g of white solid with a yield of 26%.

### Step 3:

To a mixed solution of 27c (800 mg, 1.55 mmol) in EtOH (8 mL) and EA (8 mL) was added Pd/C (80 mg) at 0°C under a hydrogen atmosphere, and the mixture was stirred at 0°C for 2.5 hours. LCMS indicated that the reaction was complete. The reaction mixture was filtered through diatomite, and the filter cake was washed with EA (200 mL). The resulting mixture was concentrated, and the residue was dissolved in THF (20 mL) and subjected to rotary evaporation to dryness to obtain 600 mg of white solid with a yield of 91%.

### Step 4:

To a solution of 27d (220 mg, 0.515 mmol), HY-13631A (250 mg, 0.47 mmol), and HATU (214 mg, 0.56 mmol) in DMF (6 mL) was added DIEA (152 mg, 1.18 mmol) at 0°C under a nitrogen atmosphere, and the mixture was reacted at 0°C for 2 hours. LCMS indicated that the reaction was complete. The reaction mixture was added to citric acid aqueous solution (pH = 4) (150 mL) and filtered, and the filter cake was washed with 175 mL of water. After filtration to dryness, the residue was dried under vacuum using an oil pump to obtain 260 mg of brown solid with a yield of 66%.

### Step 5:

To a solution of 27e (260 mg, 0.309 mmol) in DCM (30 mL) was added dropwise diethylamine (8 mL) at 0°C under a nitrogen atmosphere, and the mixture was reacted at 0°C for 3 hours. LCMS indicated that the reaction was complete. The reaction mixture was added to petroleum ether solution (600 mL) at 0°C, resulting in the precipitation of a solid. The mixture was allowed to stand until the solid settled at the bottom of the flask. The solution was decanted, and the residue was dried under vacuum using an oil pump to obtain 90 mg of brown solid with a yield of 47.1%.

### Step 6:

To a solution of 27f (90 mg, 0.13 mmol), KI-1 (92 mg, 0.19 mmol), and DIEA (50 mg, 0.39 mmol) in DMF (2.5 mL) was added HATU (74 mg, 0.19 mmol) at 0°C under a nitrogen atmosphere, and the mixture was reacted at 0°C for 2 hours. LCMS indicated that the reaction was substantially complete. The reaction mixture was added to citric acid aqueous solution (pH = 4) (30 mL) at 0°C, resulting in the precipitation of a flocculent solid. After filtration, the residue was purified by preparative TLC (DCM/MeOH = 10/1) to obtain 9.2 mg of light yellow solid X1 with a yield of 6%.

MS m/z (ESI): 1074 [M+1].

H-NMR (400 MHz, MeOD): 7.65 (d, 1H), 7.62 (s, 1H), 7.30 - 7.21(m, 5H), 6.79 (s, 2H), 5.69 - 5.65 (m, 1H), 5.57 (d, 1H), 5.43 - 5.10 (m, 3H), 4.70 (d, 2H), 4.48 - 4.39 (m, 2H), 4.10 - 4.05 (m, 1H), 4.01 - 3.75 (m, 5H), 3.46 (t, 2H), 3.22 - 3.15 (m, 2H), 3.07 - 3.00 (m, 1H), 2.75 (m, 1H), 2.62 (m, 1H), 2.45 (s, 3H), 2.37 - 2.20 (m, 6H), 2.10 - 2.02 (m, 2H), 2.00 - 1.92 (m, 2H) 1.68 - 1.57 (m, 6H), 1.01 (t, 3H).

### Linker-payload X2:

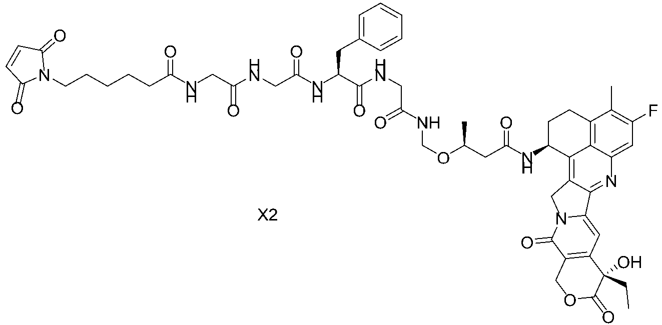

### Synthetic route:

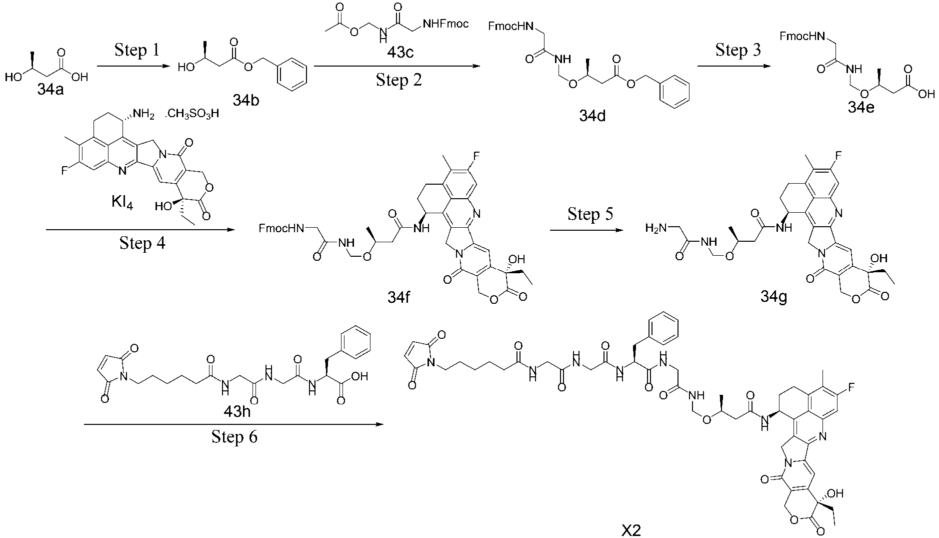

### Step 1

34a (5 g, 48.0 mmol) and K₂CO₃ (19.9 g, 144.0 mmol) were dissolved in DMF (20 mL). Benzyl bromide (12.3 g, 72.0 mmol) was added dropwise thereto, and the mixture was reacted at 25°C for 17 hours. TLC (PE/EA = 3/1) indicated that the starting material was completely consumed. The reaction mixture was added to water (200 mL) and extracted with EA (250 mL). The phases were separated, and the organic phase was washed with saturated NaCl, dried over anhydrous Na₂SO₄, and concentrated. The residue was purified by column chromatography (PE: EA = 2:1) to obtain 8.7 g of colorless liquid 34b with a yield of 93%. MS-ESI: m/z 195.1 [M+H]⁺.

### Step 2

34c (7.3 g, 19.8 mmol) and TsOH (1.46 g, 8.5 mmol) were dissolved in THF (20 mL). The mixture was cooled to 0°C under a nitrogen atmosphere, and a solution of 43b (7.7 g, 39.6 mmol) in THF (10 mL) was added dropwise thereto. After the addition was completed, the mixture was reacted at 0°C for 2 hours. TLC (PE/EA = 2/1) indicated that most of the starting material was reacted. The reaction mixture was poured into 100 mL of water and extracted with DCM (100 mL). The phases were separated, and the organic phase was washed with saturated NaCl, dried over anhydrous Na₂SO₄, and purified by column chromatography (PE/EA = 1/1) to obtain 3.9 g of colorless viscous oil 34d with a yield of 39%. MS-ESI: m/z 503.3 [M+H]⁺.

### Step 3

To a mixed solution of 34d (1.9 g, 3.78 mmol) in EtOH (100 mL) and EA (100 mL) was added Pd/C (1 g, 10 wt.%) at 0°C under a hydrogen atmosphere, and the mixture was reacted at 0°C for 3 hours. TLC (PE/EA = 2/1) indicated that the reaction was complete. The reaction mixture was filtered through diatomite, and the filter cake was washed with EA/EtOH (1:1, 100 mL × 3). The filtrate was concentrated, and the residue was dissolved in THF (50 mL × 3) and subjected to rotary evaporation to dryness. The process was repeated three times to obtain 1 g of gray solid 34e with a yield of 64%. MS-ESI: m/z 435.2 [M+Na]⁺.

### Step 4

To a solution of 34e (426 mg, 1.03 mmol), KI4 (500 mg, 0.94 mmol), and HATU (429 mg, 1.13 mmol) in DMF (20 mL) was added dropwise DIEA (303 mg, 2.35 mmol) at 0°C under a nitrogen atmosphere. After the addition was completed, the mixture was reacted at 0°C for 2 hours. LCMS indicated that the reaction was complete. The reaction mixture was added dropwise to 300 mL of water, stirred, allowed to stand for 5 minutes, and filtered. The filter cake was dissolved in DCM/MeOH (10:1, 100 mL), dried, and subjected to rotary evaporation to dryness. The residue was mixed with silica gel and purified by column chromatography (EA: MeOH = 30:1) to obtain 600 mg of yellow solid 34f with a yield of 77%. MS-ESI: m/z 830.3 [M+H]⁺.

### Step 5

To a solution of 34f (150 mg, 0.18 mmol) in DCM (5 mL) was added dropwise diethylamine (5 mL) at 0°C under a nitrogen atmosphere, and the mixture was reacted at 0°C for 2 hours. LCMS indicated that the reaction was complete. The reaction mixture was added with petroleum ether solution (100 mL × 6), resulting in the precipitation of a solid. The mixture was allowed to stand until the solid settled, and the solution was decanted. The residue was then dried under vacuum using an oil pump to obtain 120 mg of white powder 34g. LCMS indicated that the product had a content of 70% and a yield of 76%. MS-ESI: m/z 608.3 [M+H]⁺.

### Step 6

To a solution of 34g (60 mg, 0.099 mmol), 43h (51 mg, 0.108 mmol), and DIEA (32 mg, 0.25 mmol) in DMF (1 mL) was added a solution of HATU (45 mg, 0.118 mmol) in DMF (1 mL) at 0°C under a nitrogen atmosphere, and the mixture was reacted at 0°C for 2 hours. LCMS indicated that the starting material was completely consumed. The reaction mixture was directly purified by reverse-phase column chromatography (eluent: (MeCN/MeOH = 1/1): H₂O = 60%:40%) to obtain 14.8 mg of yellow solid X2 with a yield of 14%.

MS-ESI: m/z 1062.4 [M+H]⁺.

¹H NMR (400 MHz, Methanol-d₄) δ 7.69 - 7.61 (m, 2H), 7.22 - 7.16 (m, 2H), 7.16 - 7.09 (m, 3H), 6.76 (s, 2H), 5.70 - 5.64 (m, 1H), 5.60 (d, J = 16.4 Hz, 1H), 5.40 - 5.31 (m, 2H), 5.26 (d, J = 19.0 Hz, 1H), 4.65 - 4.50 (m, 7H), 4.25 - 4.16 (m, 1H), 3.87 (d, J = 16.7 Hz, 1H), 3.83 - 3.76 (m, 3H), 3.72 (d, J = 17.0 Hz, 2H), 3.44 (t, J = 7.1 Hz, 2H), 3.25 - 3.17 (m, 2H), 3.10 - 3.02 (m, 1H), 2.92 - 2.83 (m, 1H), 2.45 - 2.39 (m, 5H), 2.32 - 2.20 (m, 5H), 1.97 - 1.89 (m, 2H), 1.63 - 1.50 (m, 4H), 1.34 - 1.20 (m, 6H), 0.99 (t, J = 7.3 Hz, 3H).

### Linker-payload X3:

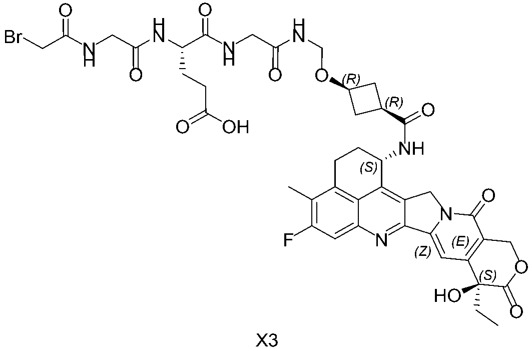

### Synthetic route:

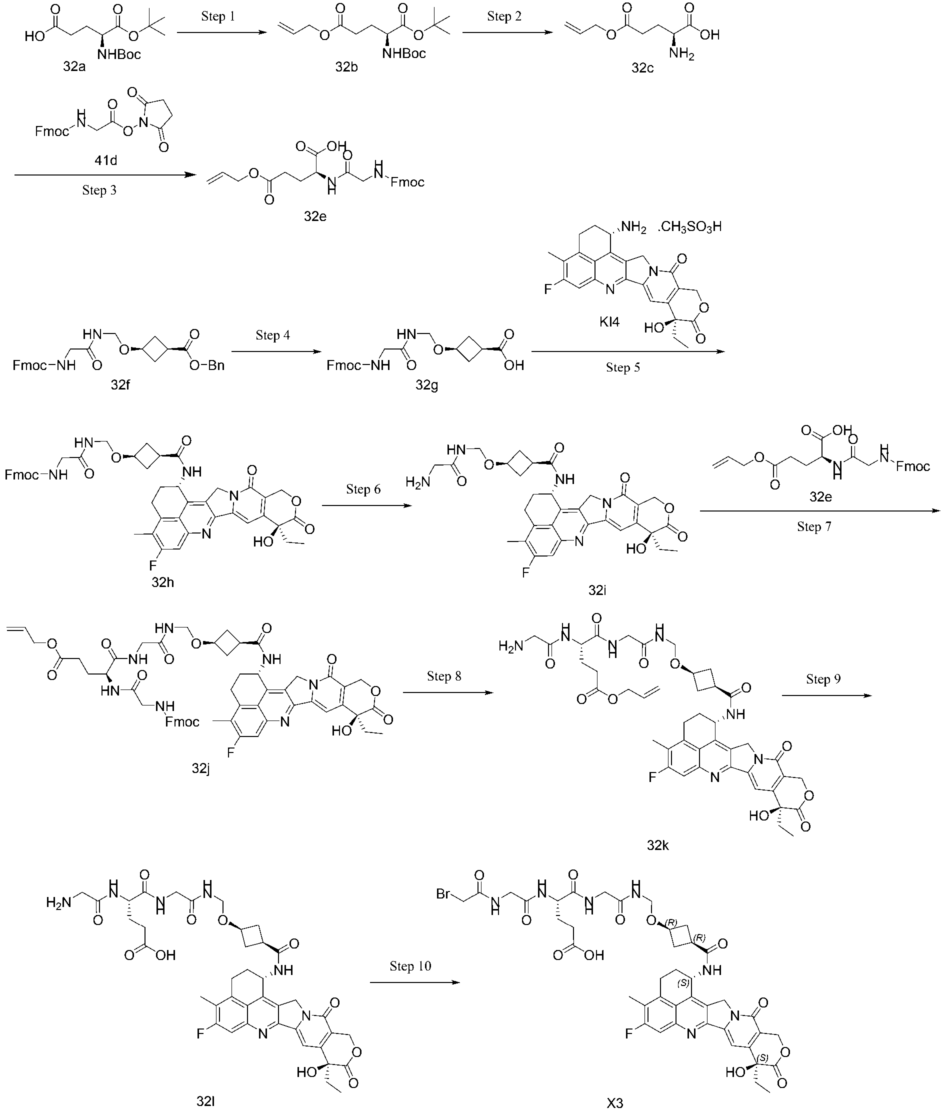

### Step 1:

To a solution of 32a (2.00 g, 6.6 mmol) and K₂CO₃ (1.82 g, 13.2 mmol) in MeCN (20 mL) was added bromopropene (960 mg, 7.92 mmol), and the mixture was reacted at 20°C for 5 hours. TLC (PE/EA = 1/2) indicated that the reaction was complete. The reaction mixture was poured into 100 mL of water to adjust the pH to 5, and extracted three times with EA (100 mL), dried over anhydrous sodium sulfate, and subjected to rotary evaporation to dryness. The residue was purified by column chromatography (PE/EA = 2/1) to obtain 1.83 g of white solid 32b with a yield of 81%.

### Step 2:

To a solution of 32b (1.38 g, 4.02 mmol) in DCM (10 mL) was added TFA (10 mL), and the mixture was stirred at 25°C for 17 hours. TLC (PE/EA = 1/3) indicated that the reaction was complete. The reaction mixture was subjected to rotary evaporation to dryness to obtain 0.91 g of yellow viscous oil 32c (yield not calculated).

### Step 3:

To a solution of 32c (910 mg, 4.87 mmol) and NaHCO₃ (613 mg, 7.3 mmol) in DME/H₂O (20 mL/10 mL) was added 41d (1.92 g, 4.87 mmol), and the mixture was stirred at 25°C for 3 hours. TLC (DCM/MeOH = 1/1) indicated that the reaction was complete. The reaction mixture was poured into 100 mL of water, added with aq. HCl (1 N) to adjust the pH to 5, extracted twice with EA (150 mL), dried over anhydrous sodium sulfate, and subjected to rotary evaporation to dryness. The residue was purified by column chromatography (DCM/MeOH = 20/1) to obtain 1.53 g of white solid 32e with a yield of 67%. MS-ESI: m/z 467.4 [M+H]⁺.

### Step 4:

To a solution of 32f (3 g, 5.83 mmol) in MeOH (50 mL) was added Pd/C (600 mg), and the mixture was stirred under a hydrogen balloon at 25°C for 5 hours. TLC (EA) indicated that the reaction was complete. The reaction mixture was filtered and subjected to rotary evaporation to dryness to obtain 1.9 g of white solid 32g with a yield of 77%.

### Step 5:

To a solution of 32g (789 mg, 1.86 mmol), KI4 (900 mg, 1.69 mmol), and triethylamine (342 mg, 3.38 mmol) in DMF (10 mL) was added HATU (707 mg, 1.86 mmol), and the mixture was stirred at 0°C for 3.5 hours. TLC (EA) indicated that the reaction was complete. The reaction mixture was poured into H₂O (80 mL) and extracted twice with EA (100 mL). The organic phase was dried over anhydrous sodium sulfate, subjected to rotary evaporation to dryness, and purified by column chromatography (EA) to obtain 1.186 g of white solid 32h with a yield of 83%. MS-ESI: m/z 842.3 [M+H]⁺.

### Step 6:

A solution of 32h (1.186 g, 1.41 mmol) in DCM/diethylamine (20 mL, 20/1) was stirred at 25°C for 17 hours. TLC (DCM/MeOH = 10/1) indicated that the reaction was complete. The reaction mixture was poured into petroleum ether (200 mL) and filtered to obtain 768 mg of white solid 32i with a yield of 88%. MS-ESI: m/z 620.3 [M+H]⁺.

### Step 7:

To a solution of 32i (676 mg, 1.09 mmol), 32e (508 mg, 1.09 mmol), and DIEA (423 mg, 3.27 mmol) in DMF (10 mL) was added HATU (414 mg, 1.09 mmol), and the mixture was stirred at 20°C for 17 hours. TLC (PE/EA = 1/5) indicated that the reaction was complete. The reaction mixture was poured into water (30 mL) and filtered. The filter cake was purified by column chromatography (DCM/MeOH = 50/1) to obtain 511 mg of white solid 32j with a yield of 44%. MS-ESI: m/z 1068.3 [M+H]⁺.

### Step 8:

A solution of 32j (482 mg, 0.451 mmol) in diethylamine/DCM (10 mL, 1/5) was stirred at 10°C for 17 hours. TLC (EA) indicated that the reaction was complete. The reaction mixture was poured into PE (300 mL) and filtered to obtain 301 mg of white solid 32k (yield not calculated).

### Step 9:

To a solution of 32k (301 mg, 0.356 mmol) and Pd(PPh₃)₄ (82 mg, 0.071 mmol) in THF (5 mL) was added morpholine (93 mg, 1.07 mmol), and the mixture was stirred at 25°C for 5 hours. LCMS indicated that the reaction was complete. The reaction mixture was purified by preparative TLC to obtain 108 mg of white solid 321 with a yield of 38%. MS-ESI: m/z 806.3 [M+H]⁺.

### Step 10:

To a solution of 321 (108 mg, 0.134 mmol) and triethylamine (41 mg, 0.402 mmol) in THF (2 mL) and DMF (2 mL) was added bromoacetyl bromide (27 mg, 0.134 mmol), and the mixture was stirred at 0°C for 1 hour. TLC (DCM/MeOH = 10/1) indicated that the reaction was complete. The reaction mixture was directly purified by preparative TLC to obtain 15 mg of white solid X3 with a yield of 12%.

MS-ESI: m/z 926.3 [M+H]⁺.

¹H NMR (400 MHz, DMSO-d₆) δ 12.11 (s, 1H), 8.54 - 8.42 (m, 3H), 8.27 - 8.16 (m, 2H), 7.78 (d, J = 11.0 Hz, 1H), 7.30 (s, 1H), 6.53 (s, 1H), 5.61 - 5.51 (m, 1H), 5.42 (s, 2H), 5.20 - 5.05 (m, 2H), 4.56 - 4.42 (m, 2H), 4.32 - 4.22 (m, 1H), 3.96 - 3.87 (m, 3H), 3.79 (d, J = 5.6 Hz, 2H), 3.70 (d, J = 5.9 Hz, 2H), 3.25 - 3.08 (m, 2H), 2.61 - 2.53 (m, 2H), 2.45 - 2.36 (m, 4H), 2.36 - 2.22 (m, 3H), 2.20 - 2.03 (m, 4H), 1.99 - 1.68 (m, 4H), 0.87 (t, J = 7.3 Hz, 3H).

### Linker-payload X4:

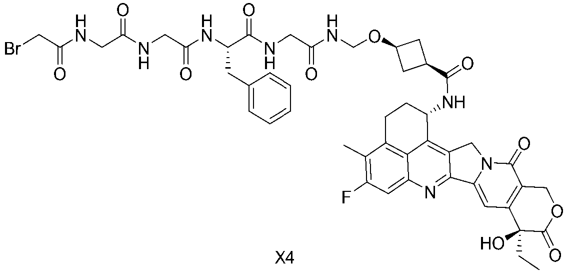

### Synthetic route:

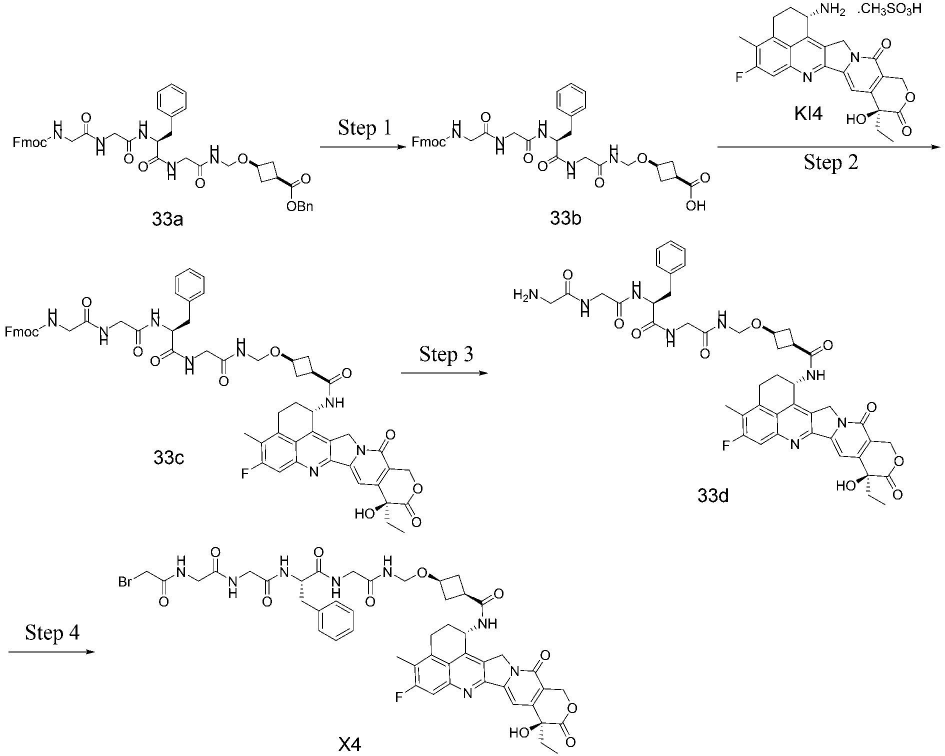

### Step 1:

To a solution of 33a (2.00 g, 2.58 mmol) in MeOH (20 mL) was added Pd/C (400 mg, 10 wt.%), and the mixture was stirred at 20°C for 5 hours. TLC (EA) indicated that the reaction was complete. The reaction mixture was filtered and subjected to rotary evaporation to dryness to obtain 1.3 g of white solid 33b with a yield of 74%.

### Step 2:

To a solution of 33b (0.55 g, 0.802 mmol), KI4 (427 mg, 0.802 mmol), and DIPEA (310 mg, 2.40 mmol) in DMF (5 mL) was added HATU (305 mg, 0.802 mmol), and the mixture was stirred at 0°C for 2 hours. TLC (DCM/MeOH = 1/10) indicated that the reaction was complete. The reaction mixture was poured into water (40 mL) and filtered to obtain a crude product, which was purified by column chromatography (DCM/MeOH = 20/1) to obtain 360 mg of yellow solid 33c with a yield of 41%.

### Step 3:

To a solution of 33c (360 mg, 0.326 mmol) in DCM (10 mL) was added diethylamine (2 mL), and the mixture was stirred at 25°C for 17 hours. TLC (DCM/MeOH = 5/1) indicated that the reaction was complete. The reaction mixture was poured into PE (100 mL) and filtered to obtain 205 mg of white solid 33d with a yield of 71%. MS-ESI: m/z 881.3 [M+H]⁺.

### Step 4:

To a solution of 33d (205 mg, 0.233 mmol) and triethylamine (118 mg, 1.17 mmol) in DMF (1 mL) and water (1 mL) was added a solution of bromoacetyl bromide (94 mg, 0.446 mmol) in THF (2 mL), and the mixture was stirred at 0°C for 1 hour. The reaction mixture was directly purified by preparative TLC to obtain 15 mg of white solid X4 with a yield of 6%.

MS-ESI: m/z 1001.2 [M+H]⁺.

¹H NMR (400 MHz, DMSO-d₆) δ 8.57 - 8.50 (m, 1H), 8.50 - 8.43 (m, 2H), 8.35 - 8.29 (m, 1H), 8.19 - 8.12 (m, 2H), 7.80 (d, J = 10.8 Hz, 1H), 7.27 - 7.14 (m, 7H), 6.53 (s, 1H), 5.59 - 5.51 (m, 1H), 5.44 - 5.39 (m, 2H), 5.20 - 5.07 (m, 2H), 4.56 - 4.44 (m, 3H), 3.92 (s, 3H), 3.80 - 3.68 (m, 5H), 3.41 (s, 1H), 3.21 - 3.12 (m, 2H), 2.83 - 2.74 (m, 1H), 2.58 - 2.55 (m, 3H), 2.39 (s, 4H), 2.18 - 2.03 (m, 4H), 1.93 - 1.78 (m, 2H), 0.87 (t, J = 7.3 Hz, 3H).

### Example 7: Preparation of anti-ADAM9 antibody conjugated with camptothecin linker-payload

### Preparation of antibody-drug conjugate Ab16-X1:

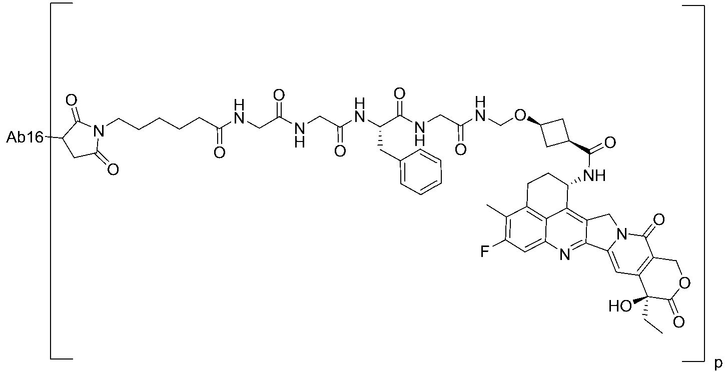

To the buffer of antibody Ab16 (PBS, pH 7.2; 40 mg, 8.7 mg/mL, 0.27 µmol) were added 2 mM EDTA solution (0.2 mL) and prepared tris(2-carboxyethyl)phosphine hydrochloride solution (7.0 mM, 0.424 mL, 2.97 µmol). The mixture was stirred in a temperature-controlled stirrer at 60 rpm for 18 hours at 22°C, after which the reaction was terminated. Linker-payload X1 (2.6 mg, 2.43 µmol) was then dissolved in 0.24 mL of DMA and added to the above reaction system. The mixture was stirred in a temperature-controlled stirrer at 60 rpm for 2 hours at 22°C, after which the reaction was terminated. The reaction mixture was desalted and purified using a G-25 gel column on an AKTA system (desalting column: HiPrep 26/10 desalting column; elution phase: 30 mM histidine-HCl, pH 5.5), followed by ultrafiltration concentration using a 30 KD ultrafiltration tube to obtain a solution of the exemplary product Ab16-X1 (30 mM histidine-HCl, pH 5.5; 38 mg, 5.6 mg/mL, yield: 95%), which was stored at -80°C. The DAR value was determined as p = 7.8 following HIC DAR analysis.

### Preparation of antibody-drug conjugate Ab16-X2:

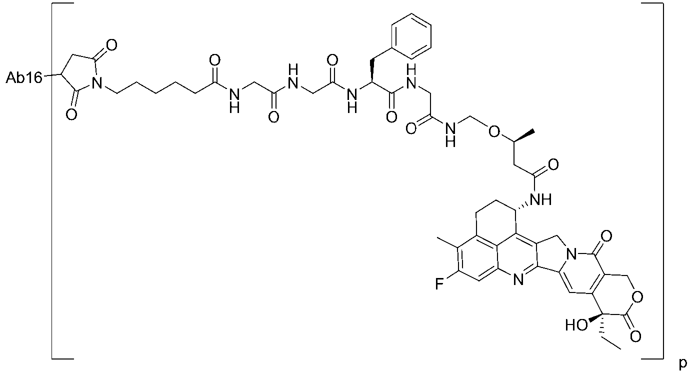

To the buffer of antibody Ab16 (PBS, pH 7.2; 40 mg, 8.7 mg/mL, 0.27 µmol) were added 2 mM EDTA solution (0.2 mL) and prepared tris(2-carboxyethyl)phosphine hydrochloride solution (7.0 mM, 0.424 mL, 2.97 µmol). The mixture was stirred in a temperature-controlled stirrer at 60 rpm for 18 hours at 22°C, after which the reaction was terminated. Linker-payload X2 (2.6 mg, 2.43 µmol) was then dissolved in 0.24 mL of DMA and added to the above reaction system. The mixture was stirred in a temperature-controlled stirrer at 60 rpm for 2 hours at 22°C, after which the reaction was terminated. The reaction mixture was desalted and purified using a G-25 gel column on an AKTA system (desalting column: HiPrep 26/10 desalting column; elution phase: 30 mM histidine-HCl, pH 5.5), followed by ultrafiltration concentration using a 30 KD ultrafiltration tube to obtain a solution of the exemplary product Ab16-X2 (30 mM histidine-HCl, pH 5.5; 37 mg, 5.6 mg/mL, yield: 92%), which was stored at -80°C. The DAR value was determined as p = 7.9 following HIC DAR analysis.

### Preparation of antibody-drug conjugate Ab15-X2:

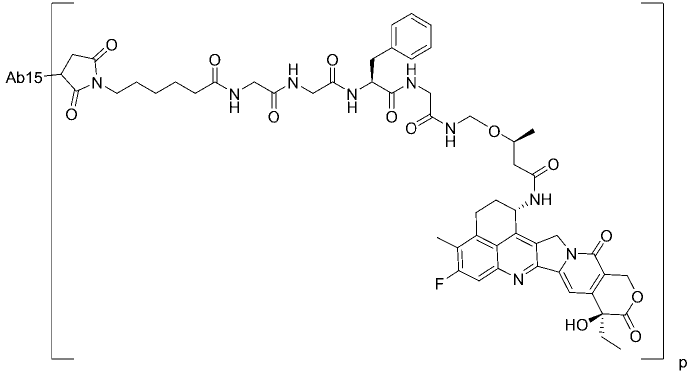

To the buffer of antibody Ab15 (PBS, pH 7.4; 40 mg, 6.8 mg/mL, 0.27 µmol) were added 2 mM EDTA solution (0.2 mL) and prepared tris(2-carboxyethyl)phosphine hydrochloride solution (7.0 mM, 0.471 mL, 3.29 µmol). The mixture was stirred in a temperature-controlled stirrer at 60 rpm for 18 hours at 22°C, after which the reaction was terminated. Linker-payload X2 (2.6 mg, 2.43 µmol) was then dissolved in 0.24 mL of DMA and added to the above reaction system. The mixture was stirred in a temperature-controlled stirrer at 60 rpm for 2 hours at 22°C, after which the reaction was terminated. The reaction mixture was desalted and purified using a G-25 gel column on an AKTA system (desalting column: HiPrep 26/10 desalting column; elution phase: 30 mM histidine-HCl, pH 5.5), followed by ultrafiltration concentration using a 30 KD ultrafiltration tube to obtain a solution of the exemplary product Ab15-X2 (30 mM histidine-HCl, pH 5.5; 32 mg, 5.2 mg/mL, yield: 80%), which was stored at -80°C. The DAR value was determined as p = 7.8 following HIC DAR analysis.

### Preparation of antibody-drug conjugate hMAB-A (21.2)-X2:

To the buffer of antibody hMAB-A (2I.2) (PBS, pH 7.4; 40 mg, 7.5 mg/mL, 0.27 µmol) were added 2 mM EDTA solution (0.2 mL) and prepared tris(2-carboxyethyl)phosphine hydrochloride solution (7.0 mM, 0.379 mL, 2.65 µmol). The mixture was stirred in a temperature-controlled stirrer at 60 rpm for 18 hours at 22°C, after which the reaction was terminated. Linker-payload X2 (2.6 mg, 2.43 µmol) was then dissolved in 0.24 mL of DMA and added to the above reaction system. The mixture was stirred in a temperature-controlled stirrer at 60 rpm for 2 hours at 22°C, after which the reaction was terminated. The reaction mixture was desalted and purified using a G-25 gel column on an AKTA system (desalting column: HiPrep 26/10 desalting column, 53 mL; elution phase: 30 mM histidine-HCl, pH 5.5), followed by ultrafiltration concentration using a 30 KD ultrafiltration tube to obtain a solution of the exemplary product hMAB-A (21.2)-X2 (30 mM histidine-HCl, pH 5.5; 29 mg, 3.2 mg/mL, yield: 72%), which was stored at -80°C. The DAR value was determined as p = 7.5 following HIC DAR analysis.

### Example 8: In vitro cell proliferation inhibition assay of anti-ADAM9 antibody-drug conjugate (ADAM9-ADC)

The CellTiter-Glo^{®} chemiluminescent cell viability assay (CTG method) was used to evaluate the inhibitory effect of anti-ADAM9 antibody-camptothecin payload ADC drugs on cell proliferation after 6 days of incubation in ADAM9-positive human colorectal cancer cells LS174T (purchased from the Cell Bank of the Chinese Academy of Sciences, Cat. No. TCHu32) or Colo205 (purchased from the Cell Bank of the Chinese Academy of Sciences, Cat. No. TCHu102) and human lung adenocarcinoma cells Calu-3.

Cells in the logarithmic growth phase were harvested and seeded at a density of 5000 cells/well. The cell plate was incubated overnight in an incubator at 37°C with 5% CO₂. On day 2, each ADC drug was diluted 3-fold in complete medium to generate 9 concentration gradients (starting from 300 nM as the highest concentration). 100 µL/well of each dilution was added to the cell culture plate with complete medium serving as a blank control, and three replicate wells were set up. The plate was incubated for another 6 days in an incubator at 37°C with 5% CO₂. After incubation, the cell culture plate was taken out and equilibrated to room temperature. 50 µL/well of CTG reagent (Promega, Cat. No. G7573) was added, and the mixture was vortexed and mixed. The plate was allowed to stand in the dark for 10 minutes, and luminescence was measured using a microplate reader. S-shaped dose-response curves were plotted using a nonlinear regression model in GraphPad Prism software, and IC₅₀ values were calculated. Cell viability was calculated using the formula: cell viability = (Lum_{test compound} - Lum_{blank control}) / (Lum_{vehicle control} - Lum_{blank control}) × 100%.

The experimental results are shown in Table 14 below, which demonstrated that Ab15-X2, Ab16-X1, and Ab16-X2 exhibited good inhibitory activity against the proliferation of human colorectal cancer or human lung adenocarcinoma cells.

**Table 14: Inhibitory activity of antibody-drug conjugates against proliferation of human colorectal cancer or human lung adenocarcinoma cells**

| Cell | Drug | IC₅₀ (nM) |
|---|---|---|
| LS174T | Ab15-X2 | 11.6 |
| | Ab16-X2 | 8.97 |
| | hMAB-A (2I.2)-X2 | 42.9 |
| Colo205 | Ab15-X2 | 28.5 |
| | Ab16-X2 | 29.8 |
| Calu-3 | Ab16-X1 | 8.60 |
| | Ab16-X2 | 3.83 |

### Example 9: In vivo tumor inhibition assay of anti-ADAM9 antibody-drug conjugate (ADAM9-ADC) in DLD-1 human colorectal cancer xenograft mice

To evaluate the inhibitory effect of ADAM9-ADC on tumorigenesis *in vivo,* the anti-tumor efficacy of each ADAM9-ADC was evaluated after establishment of xenografts in mice using ADAM9-positive human colorectal cancer cells DLD-1 (purchased from the Cell Bank of the Chinese Academy of Sciences, Cat. No. TCHul34).
(1) Test drugs and materials
   - Blank control (control group): normal saline, administered via tail vein injection, with a total of 1 dose
   - Ab15-X2 (treatment group): administered via tail vein injection at a dose of 5 mg/kg, with a total of 1 dose
   - Ab16-X2 (treatment group): administered via tail vein injection at a dose of 5 mg/kg, with a total of 1 dose
   - hMAB-A (21.2)-X2 (treatment group): administered via tail vein injection at a dose of 5 mg/kg, with a total of 1 dose
(2) Preparation method: All samples were diluted with normal saline.
(3) Experimental animals: 8-week-old female NOD SCID mice, purchased from Beijing Vital River Laboratory Animal Technology Co., Ltd.
(4) Experimental method:
   5 × 10⁶ DLD-1 cells were subcutaneously inoculated into the right anterior scapular region of 8-week-old female NOD SCID mice. When the tumor volume reached approximately 125 mm³, the tumor-bearing mice were randomly grouped using StudyDirector^{™}, and ADC drugs were administered intravenously (i.v.) on the same day (day 0) at a dose of 5 mg/kg for a total of 1 dose. Tumor volume and body weight were measured twice weekly, and data were recorded.

Each vehicle control or treatment group consisted of 6 mice. Tumor growth inhibition rate was calculated based on tumor volume measurements. Tumor growth inhibition rate (TGI%) = 100% - [(tumor volume of treatment group on the day of measurement - tumor volume of treatment group on day 0) / (tumor volume of control group on the day of measurement - tumor volume of control group on day 0)].

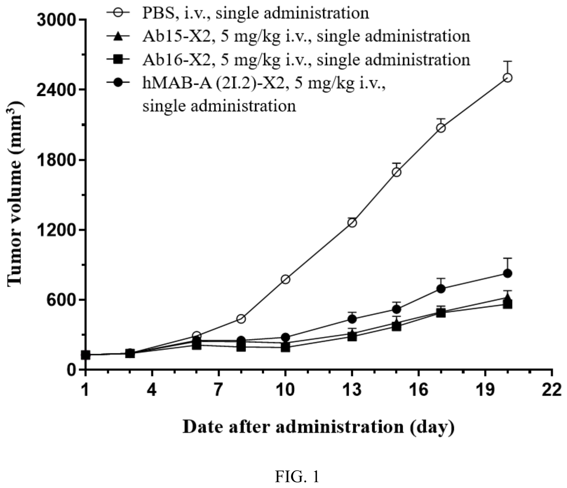

The experimental results are shown in FIG. 1 and Table 15, which demonstrated that the antibody-drug conjugates Ab15-X2 and Ab16-X2 exhibited significant tumor growth inhibition activity after a single administration and were significantly superior to hMAB-A (2I.2)-X2.

**Table 15: In vivo anti-tumor efficacy of antibody-drug conjugates in human colorectal cancer xenograft model**

| | D20 TGI% |
|---|---|
| Ab15-X2 | 79.2% |
| Ab16-X2 | 81.7% |
| hMAB-A (2I.2)-X2 | 70.6% |

### Example 10: In vivo tumor inhibition assay of anti-ADAM9 antibody-drug conjugate (ADAM9-ADC) in LS174T human colorectal cancer xenograft mice

To evaluate the inhibitory effect of ADAM9-ADC on tumorigenesis *in vivo,* the anti-tumor efficacy of each ADAM9-ADC was evaluated after establishment of xenografts in mice using ADAM9-positive human colorectal cancer cells LS174T.
(1) Test drugs and materials
   - Blank control (control group): normal saline, administered via tail vein injection (i.v.), with a total of 1 dose
   - Ab16-X2 (treatment group): administered via tail vein injection (i.v.) at a dose of 5 mg/kg, with a total of 1 dose
   - DS-8201 (treatment group): administered via tail vein injection (i.v.) at a dose of 5 mg/kg, with a total of 1 dose
   - DS-8201 (ENHERTU, purchased from Daiichi Sankyo Company Limited)
(2) Preparation method: All samples were diluted with normal saline.
(3) Experimental animals: 8-week-old female BALB/c-Nu mice, purchased from Beijing Vital River Laboratory Animal Technology Co., Ltd.
(4) Experimental method:
   5 × 10⁶ LS174T cells were subcutaneously inoculated into the right anterior scapular region of 8-week-old female BALB/c-Nu mice. When the tumor volume reached approximately 130 mm³, the tumor-bearing mice were randomly grouped using StudyDirector^{™}, and ADC drugs were administered intravenously (i.v.) on the same day (day 0) at a dose of 5 mg/kg for a total of 1 dose. Tumor volume and body weight were measured twice weekly, and data were recorded.

Each vehicle control or treatment group consisted of 6 mice. Tumor growth inhibition rate was calculated based on tumor volume measurements. Tumor growth inhibition rate (TGI%) = 100% - [(tumor volume of treatment group on the day of measurement - tumor volume of treatment group on day 0) / (tumor volume of control group on the day of measurement - tumor volume of control group on day 0)]. When the tumor volume of the control group exceeded 3000 mm³, the mice were euthanized, and the tumor growth inhibition rate (TGI) was calculated based on the tumor volume at the time of euthanasia.

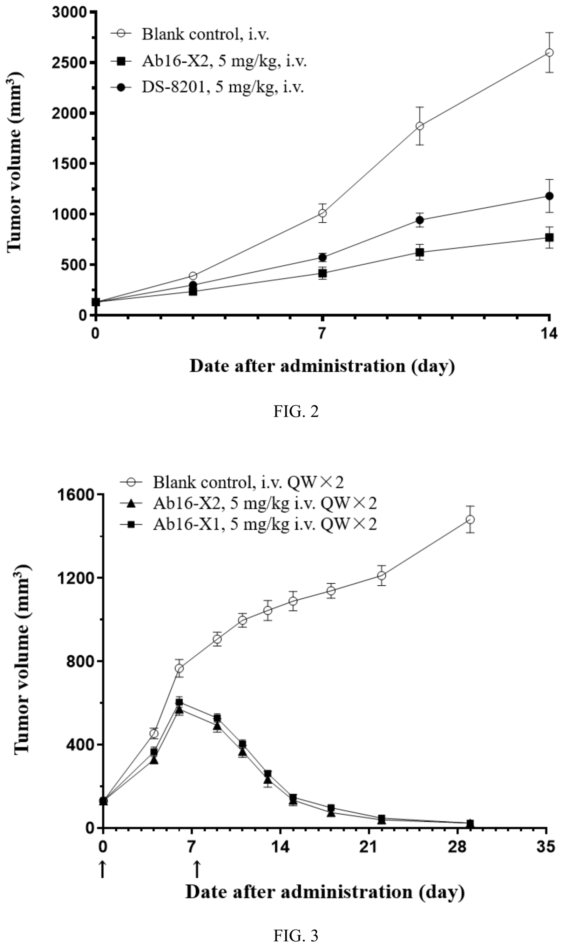

The experimental results are shown in FIG. 2 and Table 16, which demonstrated that the antibody-drug conjugate Ab16-X2 exhibited significant tumor growth inhibition activity after a single administration.

**Table 16: In vivo anti-tumor efficacy of antibody-drug conjugates in human colorectal cancer xenograft model**

| | D14 TGI% |
|---|---|
| Ab16-X2 | 74.12% |
| DS-8201 | 57.48% |

### Example 11: In vivo tumor inhibition assay of anti-ADAM9 antibody-drug conjugate (ADAM9-ADC) in Calu-3 human lung cancer xenograft mice

To evaluate the inhibitory effect of Ab16-X1 and Ab16-X2 on tumorigenesis *in vivo,* the anti-tumor efficacy was evaluated after establishment of xenografts in mice using ADAM9-positive human lung cancer cells Calu-3.
(1) Test drugs and materials
   - Blank control (control group): normal saline, administered via tail vein injection, once every 7 days, with a total of 2 doses
   - Ab16-X1 (treatment group): administered via tail vein injection at a dose of 5 mg/kg, once every 7 days, with a total of 2 doses
   - Ab16-X2 (treatment group): administered via tail vein injection at a dose of 5 mg/kg, once every 7 days, with a total of 2 doses
(2) Preparation method: All samples were diluted with normal saline.
(3) Experimental animals: 8-week-old female CB-17 SCID mice, purchased from Beijing Vital River Laboratory Animal Technology Co., Ltd.
(4) Experimental method:

1 × 10⁷ Calu-3 cells were subcutaneously inoculated into the right anterior scapular region of 8-week-old female CB-17 SCID mice. When the tumor volume reached approximately 130 mm³, the tumor-bearing mice were randomly grouped using StudyDirector^{™}, and ADC drugs were administered intravenously (i.v.) on the same day (day 0) at a dose of 5 mg/kg, once every 7 days for a total of 2 doses. Tumor volume and body weight were measured twice weekly, and data were recorded.

Each vehicle control or treatment group consisted of 5 mice. Tumor growth inhibition rate was calculated based on tumor volume measurements. Tumor growth inhibition rate (TGI%) = 100% - [(tumor volume of treatment group on the day of measurement - tumor volume of treatment group on day 0) / (tumor volume of control group on the day of measurement - tumor volume of control group on day 0)]. When tumor regression was observed (the tumor volume at measurement was smaller than the tumor volume at initial dosing), the tumor growth inhibition rate = {1 - [(tumor volume of treatment group on the day of measurement ÷ tumor volume of treatment group on day 0) ÷ (tumor volume of control group on the day of measurement ÷ tumor volume of control group on day 0)]} ÷ {1 - [(tumor volume of control group on day 0 ÷ tumor volume of control group on the day of measurement)]} × 100%.

The experimental results are shown in FIG. 3 and Table 17, which demonstrated that the antibody-drug conjugates Ab16-X1 and Ab16-X2 exhibited significant tumor growth inhibition activity after administration.

**Table 17: In vivo anti-tumor efficacy of antibody-drug conjugates in human colorectal cancer xenograft model**

| | D29 TGI% |
|---|---|
| Ab16-X1 | 107.90% |
| Ab16-X2 | 107.98% |

### Example 12: Cell proliferation inhibition of anti-ADAM9 antibody-drug conjugate (ADAM9-ADC) with low cell binding activity

### 12.1 In vitro cell proliferation inhibition assay in LS174T cells

The cell binding activity of Ab16-X2 was evaluated using tumor cells co-expressing ADAM9 and HerG2, with DS-8201 (ENHERTU, purchased from Daiichi Sankyo Company Limited) as the positive control and an anti-HIV human IgG antibody (iso-IgG1, produced by WuXi AppTec) as the negative control. HepG2 (purchased from the Cell Bank of the Chinese Academy of Sciences, Cat. No. TCHu72) and LS174T cells with medium and low ADAM9 expression were added to a 96-well round-bottom plate at 1E5 cells per well, followed by addition of 100 µL each of serially diluted Ab16-X2, DS-8201, or iso-IgG1, and incubated at 4°C for 1 hour. After incubation, the cells were washed with FACS buffer (PBS containing 2% FBS), followed by addition of PE-conjugated goat anti-human secondary antibody (Abcam, ab98596), and incubated at 4°C for 0.5 hours. After incubation, the cells were washed with FACS buffer, resuspended in FACS buffer, and analyzed by flow cytometry.

The CellTiter-Glo^{®} chemiluminescent cell viability assay (CTG method) was used to evaluate the cell killing effect of Ab16-X2 and DS-8201 on LS174T (3 days of incubation) and HepG2 (6 days of incubation) cells. Cells in the logarithmic growth phase were harvested and seeded at a density of 3000 cells/well. The cell plate was incubated overnight in an incubator at 37°C with 5% CO₂. On day 2, the drug was diluted 3-fold in complete medium to generate 9 concentration gradients (starting from 1000 nM as the highest concentration). 100 µL/well of each dilution was added to the cell culture plate with complete medium serving as a blank control, and three replicate wells were set up. The plate was incubated for another 3 or 6 days in an incubator at 37°C with 5% CO₂. After incubation, the cell culture plate was taken out and equilibrated to room temperature. 50 µL/well of CTG reagent was added, and the mixture was vortexed and mixed. The plate was allowed to stand in the dark for 10 minutes, and luminescence was measured using a microplate reader. S-shaped dose-response curves were plotted using a nonlinear regression model in GraphPad Prism software, and IC₅₀ values were calculated. Cell viability was calculated using the formula: cell viability = (Lum_{test compound} - Lum_{blank control}) / (Lum_{vehicle control} - Lum_{blank control}) × 100%. Proliferation inhibition rate = 100% - cell viability %.

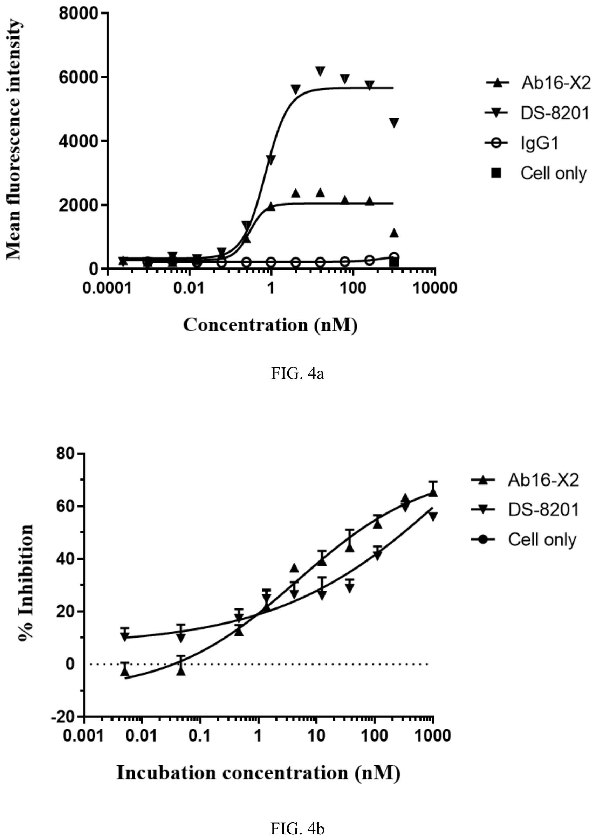

The results of FACS binding assay on LS174T cells are shown in Table 18 and FIG. 4a. Ab16-X2 exhibited low binding capability (mean fluorescence intensity) to LS174T cells, while DS-8201 exhibited high binding capability, which was approximately 2- to 3-fold higher than that of Ab16-X2 at maximum binding levels (within the concentration range of 0.976 nM to 1,000 nM), indicating that the affinity of DS-8201 for LS174T cells was significantly higher than that of Ab16-X2.

The results of *in vitro* proliferation inhibition by CTG are shown in Table 19 and FIG. 4b. The results demonstrated that when the incubation concentration reached 1.372 nM, the proliferation inhibition rate of Ab16-X2 was comparable to that of DS-8201. As the incubation concentration increased, within the range of 4.115 nM to 1,000 nM, the proliferation inhibition rate of Ab16-X2 was significantly higher than that of DS-8201. These results indicated that even with low cell binding activity, Ab16-X2 still exhibited strong cell proliferation inhibition, whereas DS-8201 did not show such effect.

**Table 18: Comparison of binding activity of Ab16-X2 and DS-8201 on LS174T cells**

| Incubation concentration (nM) | Mean fluorescence intensity | | |
|---|---|---|---|
| | Ab16-X2 | DS-8201 | iso-IgG 1 |
| 1000 | 1134 | 4566 | 368 |
| 250 | 2131 | 5739 | 269 |
| 62.5 | 2159 | 5937 | 229 |
| 15.625 | 2395 | 6180 | 209 |
| 3.906250 | 2379 | 5604 | 203 |
| 0.976563 | 1957 | 3402 | 212 |
| 0.244141 | 954 | 1345 | 208 |
| 0.061035 | 447 | 515 | 202 |
| 0.015259 | 268 | 301 | 213 |
| 0.003815 | 219 | 386 | 254 |
| 0.000954 | 223 | 213 | 220 |

**Table 19: Comparison of in vitro proliferation inhibition activity of Ab 16-X2 and DS-8201 on LS174T cells**

| | Inhibition rate | |
|---|---|---|
| Incubation concentration (nM) | Ab16-X2 | DS-8201 |
| 1000 | 65.38% | 55.91% |
| 333.333 | 63.34% | 59.43% |
| 111.111 | 53.32% | 41.01% |
| 37.037 | 44.37% | 28.74% |
| 12.346 | 39.14% | 26.02% |
| 4.115 | 36.73% | 26.43% |
| 1.372 | 21.80% | 24.91% |
| 0.457 | 12.48% | 17.34% |
| 0.046 | -2.54% | 9.74% |
| 0.005 | -2.73% | 10.26% |

### 12.2 In vitro cell proliferation inhibition assay in HepG2 cells

The binding activity and proliferation inhibition activity for HepG2 cells were evaluated with reference to Example 12.1, and the experimental procedure was the same as that for LS174T cells.

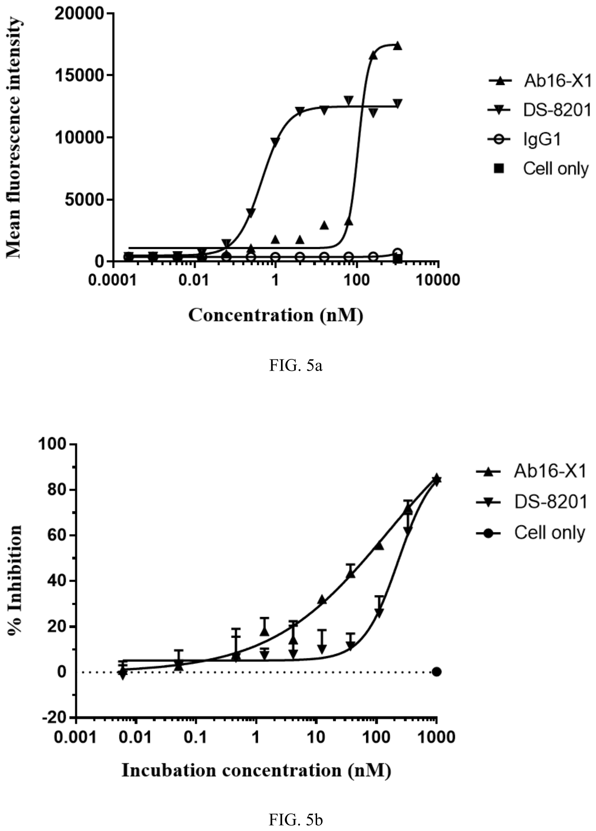

The results of FACS binding assay on HepG2 cells are shown in Table 20 and FIG. 5a. When the incubation concentration was 0.061035 nM to 62.5 nM, Ab16-X1 exhibited low binding capability (mean fluorescence intensity) to HepG2 cells, while at the same concentration, DS-8201 exhibited high binding capability to HepG2 cells. The experimental results indicated that at such incubation concentration, the affinity of DS-8201 for HepG2 cells was significantly higher than that of Ab16-X1.

The results of *in vitro* killing assay by CTG are shown in Table 21 and FIG. 5b. Within the concentration range of 0.051 nM to 333.33 nM, the proliferation inhibition rate of Ab16-X1 was superior to that of DS-8201. These results indicated that even with low cell binding activity, Ab16-X1 still exhibited strong cell proliferation inhibition, whereas DS-8201 did not show such effect.

**Table 20: Comparison of binding activity of Ab16-X1 and DS-8201 on HepG2 cells**

| Incubation concentration (nM) | Mean fluorescence intensity (MFI) | | |
|---|---|---|---|
| | Ab16-X1 | DS-8201 | iso-IgG1 |
| 1000 | 17416 | 12708 | 690 |
| 250 | 16644 | 11955 | 389 |
| 62.5 | 3287 | 12961 | 386 |
| 15.625 | 2956 | 12173 | 364 |
| 3.906250 | 1783 | 12075 | 361 |
| 0.976563 | 1793 | 9582 | 360 |
| 0.244141 | 1089 | 3889 | 363 |
| 0.061035 | 623 | 1423 | 377 |
| 0.015259 | 451 | 660 | 371 |
| 0.003815 | 396 | 441 | 349 |
| 0.000954 | 376 | 398 | 365 |
| 0.000238 | 370 | 366 | 361 |

**Table 21: Comparison of in vitro proliferation inhibition activity of Ab16-X1 and DS-8201 on HepG2 cells**

| Incubation concentration (nM) | Proliferation inhibition rate % | |
|---|---|---|
| | Ab16-X1 | DS-8201 |
| 1000 | 85.53% | 83.49% |
| 333.333 | 72.30% | 61.64% |
| 111.111 | 55.86% | 25.72% |
| 37.037 | 43.58% | 11.36% |
| 12.346 | 32.22% | 9.96% |
| 4.115 | 14.36% | 7.78% |
| 1.372 | 18.07% | 7.24% |
| 0.457 | 7.86% | 6.28% |
| 0.051 | 2.81% | 2.80% |
| 0.006 | 1.04% | -1.45% |

### Example 13: Preclinical toxicology study of anti-ADAM9 antibody-drug conjugate (ADAM9-ADC) in cynomolgus monkeys

AB16-X2 was administered to cynomolgus monkeys via intravenous infusion once every 3 weeks for a total of 2 doses. The nature, extent, and time-effect relationship of toxic reactions potentially induced by the antibody-drug conjugate were observed to preliminarily identify target organs or tissues of toxicity.

### Method

Two cynomolgus monkeys (sourced from Suzhou Xishan Zhongke Laboratory Animal Co., Ltd.), one male and one female, were intravenously administered with AB16-X2. The first dose was 45 mg/kg, and the second dose was increased to 80 mg/kg after 3 weeks. Necropsy was performed one week after the second dose. During the study, the following parameters were monitored and observed:
(1) General condition observation: During the trial, external signs, mental state, behavioral activity, excreta characteristics, and administration sites were observed 2 to 3 times daily. (2) Body weight: measured once during the acclimatization period and once weekly during the dosing period. (3) Food consumption: measured once daily. (4) Ophthalmic examination: measured once during the acclimatization period, and once on D14, before necropsy, or at the end of the dosing period. (5) Electrocardiogram: measured once during the acclimatization period, and once 6 hours and 24 hours after the first and second doses. (6) Clinical pathology: measured once during the acclimatization period, and collected once on D3, D7, D14, D21, and D28 during the dosing period. (7) Toxicokinetics. (8) Gross anatomical observation and histopathological examination: Routine histological processing and histopathological examination were performed on the sternum (including bone marrow), heart, lungs, kidneys, bladder, duodenum, colon, cecum, eyes, skin, and any macroscopically abnormal organs/tissues (spleen, rectum) from scheduled necropsy animals.

### Conclusion

Under the experimental conditions, cynomolgus monkeys were intravenously administered with 45 to 80 mg/kg of AB16-X2. During the trial, all animals survived until the end of the dosing period or the day of scheduled necropsy, with no animals found moribund or dead. Following AB16-X2 administration, only transient mild leukopenia and transient mild elevations of ALT and AST were observed in cynomolgus monkeys, with no other pathological changes observed.

IMGC-936, developed by MacroGenics, is an ADAM9-ADC conjugated with the maytansinoid tubulin inhibitor DM21-C and has entered clinical research. In monkey toxicology studies, corneal lesions and ocular toxicity were observed after administration of IMGC-936 at a dose of 22.5 mg/kg (Mol Cancer Ther 2022; 21: 1047-59). In monkey toxicology studies, no ocular toxicity was observed at a dose of 80 mg/kg for AB16-X2.

Although specific embodiments of the present disclosure have been described above, it should be understood by those skilled in the art that these are merely illustrative, and various changes or modifications may be made to these embodiments without departing from the principles and essence of the present disclosure. Therefore, the scope of protection of the present disclosure is defined by the appended claims.

## Claims

1. An ADAM9-targeting antibody or an antigen-binding fragment thereof, wherein the antibody or the antigen-binding fragment thereof comprises a heavy chain variable region and a light chain variable region, wherein the heavy chain variable region comprises heavy chain complementarity-determining regions HCDR1, HCDR2, and HCDR3, and the light chain variable region comprises light chain complementarity-determining regions LCDR1, LCDR2, and LCDR3;
the HCDR1 comprises the amino acid sequence shown in LYWMX₁, the HCDR2 comprises the amino acid sequence shown in X₂IIPIFGHTX₃YX₄EKFX₃X₆, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19;
wherein X₁ is N, D, H, or E; X₂ is R or D, X₃ is D or K, X₄ is N or E, X₅ is K or R, X₆ is D or N.

2. The antibody or the antigen-binding fragment thereof according to claim 1, wherein in the HCDR1, X₁ is N or H; in the HCDR2, X₂ is R, X₃ is K, X₄ is N, X₅ is K, X₆ is D or N; preferably,
when X₁ is N, then X₂ is R, X₃ is K, X₄ is N, X₅ is K, X₆ is D;
when X₁ is H, then X₂ is R, X₃ is K, X₄ is N, X₅ is K, X₆ is N.

3. The antibody or the antigen-binding fragment thereof according to claim 1 or 2, wherein the HCDR1 comprises the amino acid sequence shown in SEQ ID NO: 3, the HCDR2 comprises the amino acid sequence shown in SEQ ID NO: 8, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19; or,
the HCDR1 comprises the amino acid sequence shown in SEQ ID NO: 1, the HCDR2 comprises the amino acid sequence shown in SEQ ID NO: 7, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19; or,
the HCDR1 comprises the amino acid sequence shown in SEQ ID NO: 1, the HCDR2 comprises the amino acid sequence shown in SEQ ID NO: 5, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19; or,
the HCDR1 comprises the amino acid sequence shown in SEQ ID NO: 2, the HCDR2 comprises the amino acid sequence shown in SEQ ID NO: 6, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19; or,
the HCDR1 comprises the amino acid sequence shown in SEQ ID NO: 3, the HCDR2 comprises the amino acid sequence shown in SEQ ID NO: 7, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19; or,
the HCDR1 comprises the amino acid sequence shown in SEQ ID NO: 2, the HCDR2 comprises the amino acid sequence shown in SEQ ID NO: 9, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19; or,
the HCDR1 comprises the amino acid sequence shown in SEQ ID NO: 4, the HCDR2 comprises the amino acid sequence shown in SEQ ID NO: 10, the HCDR3 comprises the amino acid sequence shown in SEQ ID NO: 11, the LCDR1 comprises the amino acid sequence shown in SEQ ID NO: 12, the LCDR2 comprises the amino acid sequence shown in SEQ ID NO: 16, and the LCDR3 comprises the amino acid sequence shown in SEQ ID NO: 19.

4. The antibody or the antigen-binding fragment thereof according to claim 3, wherein the amino acid sequence of the HCDR1 is shown in SEQ ID NO: 3, the amino acid sequence of the HCDR2 is shown in SEQ ID NO: 8, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19; or,
the amino acid sequence of the HCDR1 is shown in SEQ ID NO: 1, the amino acid sequence of the HCDR2 is shown in SEQ ID NO: 7, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19; or,
the amino acid sequence of the HCDR1 is shown in SEQ ID NO: 1, the amino acid sequence of the HCDR2 is shown in SEQ ID NO: 5, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19; or,
the amino acid sequence of the HCDR1 is shown in SEQ ID NO: 2, the amino acid sequence of the HCDR2 is shown in SEQ ID NO: 6, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19; or,
the amino acid sequence of the HCDR1 is shown in SEQ ID NO: 3, the amino acid sequence of the HCDR2 is shown in SEQ ID NO: 7, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19; or,
the amino acid sequence of the HCDR1 is shown in SEQ ID NO: 2, the amino acid sequence of the HCDR2 is shown in SEQ ID NO: 9, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19; or,
the amino acid sequence of the HCDR1 is shown in SEQ ID NO: 4, the amino acid sequence of the HCDR2 is shown in SEQ ID NO: 10, the amino acid sequence of the HCDR3 is shown in SEQ ID NO: 11, the amino acid sequence of the LCDR1 is shown in SEQ ID NO: 12, the amino acid sequence of the LCDR2 is shown in SEQ ID NO: 16, and the amino acid sequence of the LCDR3 is shown in SEQ ID NO: 19.

5. The antibody or the antigen-binding fragment thereof according to any one of claims 1 to 4, wherein the heavy chain variable region and/or the light chain variable region further comprises a framework region, wherein the framework region is a humanized framework region; preferably,
the heavy chain variable region comprises an amino acid sequence having at least 90%, at least 95%, or at least 99% sequence identity with SEQ ID NO: 31, and/or the light chain variable region comprises an amino acid sequence having at least 90%, at least 95%, or at least 99% sequence identity with SEQ ID NO: 23; or,
the heavy chain variable region comprises an amino acid sequence having at least 90%, at least 95%, or at least 99% sequence identity with SEQ ID NO: 42, and/or the light chain variable region comprises an amino acid sequence having at least 90%, at least 95%, or at least 99% sequence identity with SEQ ID NO: 43; or,
the heavy chain variable region comprises an amino acid sequence having at least 90%, at least 95%, or at least 99% sequence identity with SEQ ID NO: 22, and/or the light chain variable region comprises an amino acid sequence having at least 90%, at least 95%, or at least 99% sequence identity with SEQ ID NO: 23; or,
the heavy chain variable region comprises an amino acid sequence having at least 90%, at least 95%, or at least 99% sequence identity with SEQ ID NO: 24, and/or the light chain variable region comprises an amino acid sequence having at least 90%, at least 95%, or at least 99% sequence identity with SEQ ID NO: 25; or,
the heavy chain variable region comprises an amino acid sequence having at least 90%, at least 95%, or at least 99% sequence identity with SEQ ID NO: 26, and/or the light chain variable region comprises an amino acid sequence having at least 90%, at least 95%, or at least 99% sequence identity with SEQ ID NO: 27; or,
the heavy chain variable region comprises an amino acid sequence having at least 90%, at least 95%, or at least 99% sequence identity with SEQ ID NO: 28, and/or the light chain variable region comprises an amino acid sequence having at least 90%, at least 95%, or at least 99% sequence identity with SEQ ID NO: 30; or,
the heavy chain variable region comprises an amino acid sequence having at least 90%, at least 95%, or at least 99% sequence identity with SEQ ID NO: 34, and/or the light chain variable region comprises an amino acid sequence having at least 90%, at least 95%, or at least 99% sequence identity with SEQ ID NO: 35; or,
the heavy chain variable region comprises an amino acid sequence having at least 90%, at least 95%, or at least 99% sequence identity with SEQ ID NO: 36, and/or the light chain variable region comprises an amino acid sequence having at least 90%, at least 95%, or at least 99% sequence identity with SEQ ID NO: 30; or,
the heavy chain variable region comprises an amino acid sequence having at least 90%, at least 95%, or at least 99% sequence identity with SEQ ID NO: 37, and/or the light chain variable region comprises an amino acid sequence having at least 90%, at least 95%, or at least 99% sequence identity with SEQ ID NO: 38; or,
the heavy chain variable region comprises an amino acid sequence having at least 90%, at least 95%, or at least 99% sequence identity with SEQ ID NO: 36, and/or the light chain variable region comprises an amino acid sequence having at least 90%, at least 95%, or at least 99% sequence identity with SEQ ID NO: 27; or,
the heavy chain variable region comprises an amino acid sequence having at least 90%, at least 95%, or at least 99% sequence identity with SEQ ID NO: 39, and/or the light chain variable region comprises an amino acid sequence having at least 90%, at least 95%, or at least 99% sequence identity with SEQ ID NO: 27; or,
the heavy chain variable region comprises an amino acid sequence having at least 90%, at least 95%, or at least 99% sequence identity with SEQ ID NO: 44, and/or the light chain variable region comprises an amino acid sequence having at least 90%, at least 95%, or at least 99% sequence identity with SEQ ID NO: 45;
the variable region comprising an amino acid sequence having at least 90%, at least 95%, or at least 99% sequence identity maintains antigen-binding functionality at least equivalent to that of the original sequence.

6. The antibody or the antigen-binding fragment thereof according to claim 5, wherein the amino acid sequence of the heavy chain variable region is shown in SEQ ID NO: 31, and/or the amino acid sequence of the light chain variable region is shown in SEQ ID NO: 23;
the amino acid sequence of the heavy chain variable region is shown in SEQ ID NO: 42, and/or the amino acid sequence of the light chain variable region is shown in SEQ ID NO: 43; or,
the amino acid sequence of the heavy chain variable region is shown in SEQ ID NO: 22, and/or the amino acid sequence of the light chain variable region is shown in SEQ ID NO: 23; or,
the amino acid sequence of the heavy chain variable region is shown in SEQ ID NO: 24, and/or the amino acid sequence of the light chain variable region is shown in SEQ ID NO: 25; or,
the amino acid sequence of the heavy chain variable region is shown in SEQ ID NO: 26, and/or the amino acid sequence of the light chain variable region is shown in SEQ ID NO: 27; or,
the amino acid sequence of the heavy chain variable region is shown in SEQ ID NO: 28, and/or the amino acid sequence of the light chain variable region is shown in SEQ ID NO: 30; or,
the amino acid sequence of the heavy chain variable region is shown in SEQ ID NO: 34, and/or the amino acid sequence of the light chain variable region is shown in SEQ ID NO: 35; or,
the amino acid sequence of the heavy chain variable region is shown in SEQ ID NO: 36, and/or the amino acid sequence of the light chain variable region is shown in SEQ ID NO: 30; or,
the amino acid sequence of the heavy chain variable region is shown in SEQ ID NO: 37, and/or the amino acid sequence of the light chain variable region is shown in SEQ ID NO: 38; or,
the amino acid sequence of the heavy chain variable region is shown in SEQ ID NO: 36, and/or the amino acid sequence of the light chain variable region is shown in SEQ ID NO: 27; or,
the amino acid sequence of the heavy chain variable region is shown in SEQ ID NO: 39, and/or the amino acid sequence of the light chain variable region is shown in SEQ ID NO: 27; or,
the amino acid sequence of the heavy chain variable region is shown in SEQ ID NO: 44; and/or, the amino acid sequence of the light chain variable region is shown in SEQ ID NO: 45.

7. The antibody or the antigen-binding fragment thereof according to any one of claims 1 to 6, wherein the antibody or the antigen-binding fragment thereof is:
(1) a full-length antibody, Fab, Fab', F(ab')₂, Fv, sdAb, or scFv; and/or
(2) a monoclonal antibody, bispecific antibody, or multispecific antibody;
and/or, the ADAM9 is human or monkey ADAM9.

8. The antibody or the antigen-binding fragment thereof according to claim 7, wherein when the antibody or the antigen-binding fragment thereof is a full-length antibody, it comprises a heavy chain constant region of a heavy chain of a human antibody, preferably a heavy chain constant region of a human antibody IgG1; and/or, it comprises a light chain constant region of a light chain of a human antibody, preferably a light chain constant region of a human antibody κ chain;
preferably, the amino acid sequence of the heavy chain constant region of the human antibody IgG1 is shown in SEQ ID NO: 48, or has at least 90%, at least 95%, or at least 99% sequence identity with SEQ ID NO: 48; and/or, the amino acid sequence of the light chain constant region of the human antibody κ chain is shown in SEQ ID NO: 49, or has at least 90%, at least 95%, or at least 99% sequence identity with SEQ ID NO: 49;
more preferably, the heavy chain of the antibody or the antigen-binding fragment thereof comprises an amino acid sequence having at least 90%, at least 95%, or at least 99% sequence identity with SEQ ID NO: 50; and/or, the light chain of the antibody or the antigen-binding fragment thereof comprises an amino acid sequence having at least 90%, at least 95%, or at least 99% sequence identity with SEQ ID NO: 51; or, the heavy chain of the antibody or the antigen-binding fragment thereof comprises an amino acid sequence having at least 90%, at least 95%, or at least 99% sequence identity with SEQ ID NO: 52; and/or, the light chain of the antibody or the antigen-binding fragment thereof comprises an amino acid sequence having at least 90%, at least 95%, or at least 99% sequence identity with SEQ ID NO: 53; the amino acid sequence having at least 90%, at least 95%, or at least 99% sequence identity maintains antigen-binding functionality at least equivalent to that of the original sequence;
even more preferably, the amino acid sequence of the heavy chain of the antibody or the antigen-binding fragment thereof is shown in SEQ ID NO: 50; and/or, the amino acid sequence of the light chain of the antibody or the antigen-binding fragment thereof is shown in SEQ ID NO: 51; or, the amino acid sequence of the heavy chain of the antibody or the antigen-binding fragment thereof is shown in SEQ ID NO: 52; and/or, the amino acid sequence of the light chain of the antibody or the antigen-binding fragment thereof is shown in SEQ ID NO: 53.

9. An isolated nucleic acid, wherein the nucleic acid encodes the antibody or the antigen-binding fragment thereof as defined in any one of claims 1 to 8.

10. A recombinant expression vector, wherein the recombinant expression vector comprises the isolated nucleic acid as defined in claim 9; preferably, the backbone plasmid of the recombinant expression vector is PTT5.

11. A transformant, wherein the transformant comprises the recombinant expression vector as defined in claim 10; preferably, the host cell of the transformant is a eukaryotic cell; more preferably, the eukaryotic cell is a CHO cell.

12. A method for preparing an ADAM9-targeting antibody or an antigen-binding fragment thereof, wherein the method comprises the steps of culturing the transformant as defined in claim 11, and isolating the ADAM9-targeting antibody or the antigen-binding fragment thereof from the culture.

13. A method for detecting ADAM9, wherein the method comprises the step of contacting a test sample with the antibody or the antigen-binding fragment thereof as defined in any one of claims 1 to 8; preferably, the detection is for non-diagnostic and/or non-therapeutic purposes.

14. An antibody-drug conjugate, wherein the antibody-drug conjugate comprises the antibody or the antigen-binding fragment thereof as defined in any one of claims 1 to 8, a linker unit L, and a cytotoxic drug.

15. The antibody-drug conjugate according to claim 14, wherein the cytotoxic drug is a structure represented by formula (A-1), a stereoisomer thereof, a pharmaceutically acceptable salt thereof, a solvate thereof, or a solvate of the salt thereof, 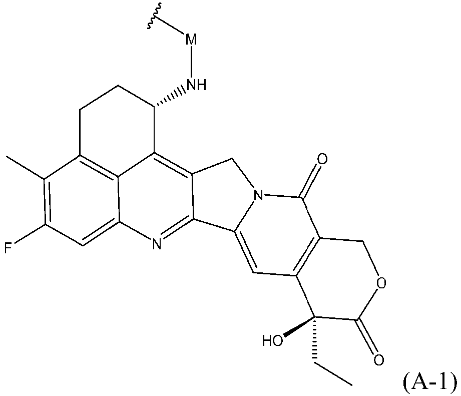 wherein
M is -L²-L¹-C(O)-;
L² is -O- or -S-, and L² is connected to the linker unit L;
L¹ is -(C(R^{1a})(R^{1b}))ₘ-CH₂-, C₃-C₆ saturated cycloalkyl, or 3- to 6-membered saturated heterocyclyl, wherein the C₃-C₆ saturated cycloalkyl and 3- to 6-membered saturated heterocyclyl are each independently and optionally substituted by one or more R^{2a};
m is 1, 2, 3, or 4; heteroatoms in the 3- to 6-membered saturated heterocyclyl are each independently N, O, or S, and the number of heteroatoms is 1, 2, or 3;
R^{1a}, R^{1b}, and R^{2a} are each independently hydrogen, halogen, hydroxyl, amino, or C₁-C₆ alkyl, wherein the C₁-C₆ alkyl is optionally substituted by one or more R;
each R is independently hydrogen or halogen.

16. The antibody-drug conjugate according to claim 15, wherein L¹ is -(C(R^{1a})(R^{1b}))ₘ-CH₂-; each R^{1a} is independently hydrogen, halogen, or C₁-C₆ alkyl; each R^{1b} is independently hydrogen, halogen, or C₁-C₆ alkyl.

17. The antibody-drug conjugate according to claim 16, wherein L¹ is

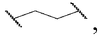

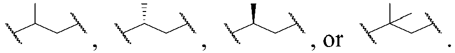

18. The antibody-drug conjugate according to claim 15, wherein L¹ is C₃-C₆ saturated cycloalkyl, wherein the C₃-C₆ saturated cycloalkyl is optionally substituted by one or more R^{2a}, and each R^{2a} is independently hydrogen, halogen, or C₁-C₆ alkyl.

19. The antibody-drug conjugate according to claim 18, wherein L¹ is

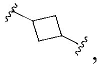

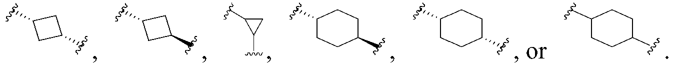

20. The antibody-drug conjugate according to claim 15, wherein the cytotoxic drug is any one of the following structures:

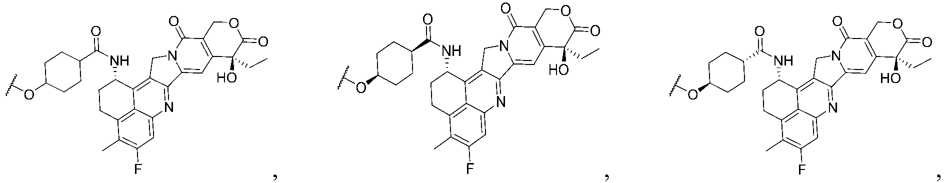

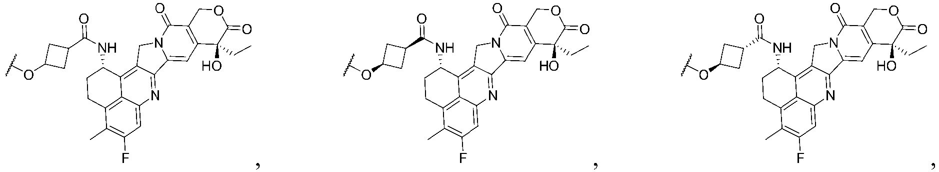

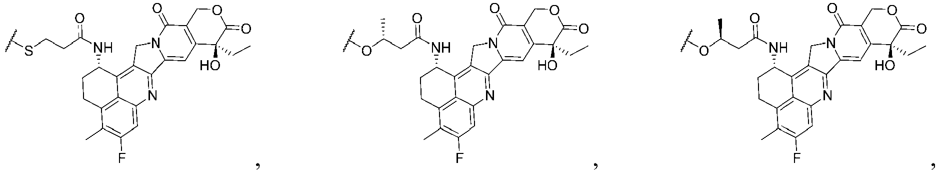

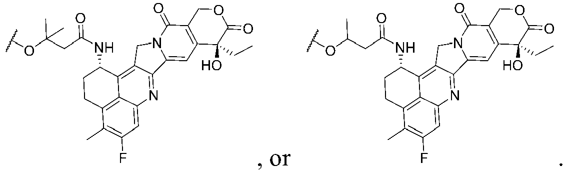

21. The antibody-drug conjugate according to any one of claims 14 to 20, an isomer thereof, a pharmaceutically acceptable salt thereof, or a mixture thereof, wherein the linker unit L is -Lₐ-L_{b}-L_{c}-; and the L_{c} is connected to the cytotoxic drug;
-Lₐ- is 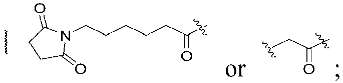 preferably 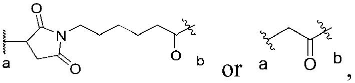 wherein the a-terminus is connected to Ab, and the b-terminus is connected to L_{b};
-L_{b}- is any one of the following structures: 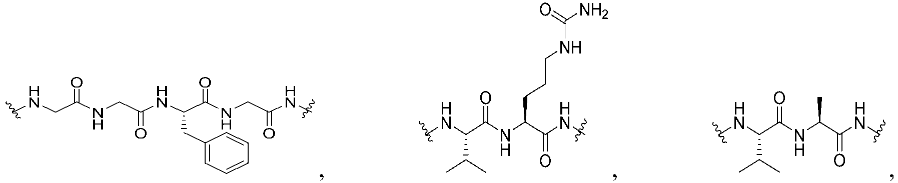 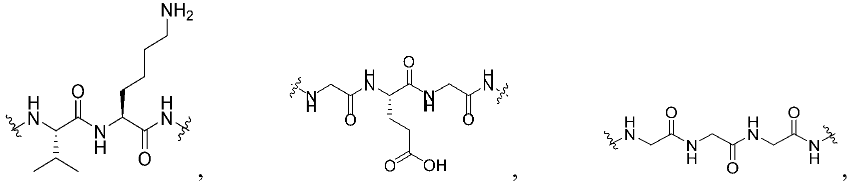 or 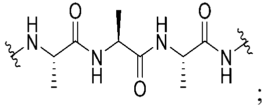 preferably 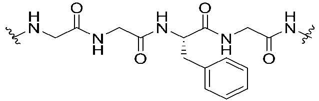 or 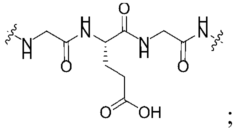 more preferably 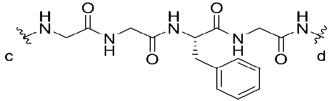 or 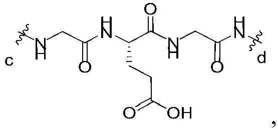 wherein the c-terminus is connected to Lₐ, and the d-terminus is connected to L_{c}; -L_{c}- is

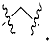

22. The antibody-drug conjugate according to claim 21, wherein the linker unit L is 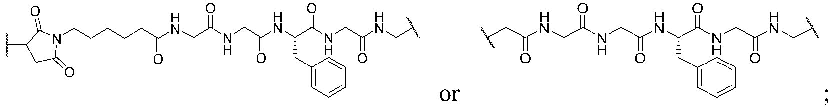 preferably

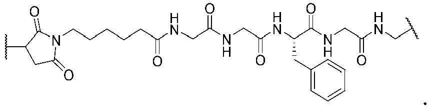

23. The antibody-drug conjugate according to any one of claims 14 to 22, wherein the antibody-drug conjugate has a structure represented by formula (A-2): 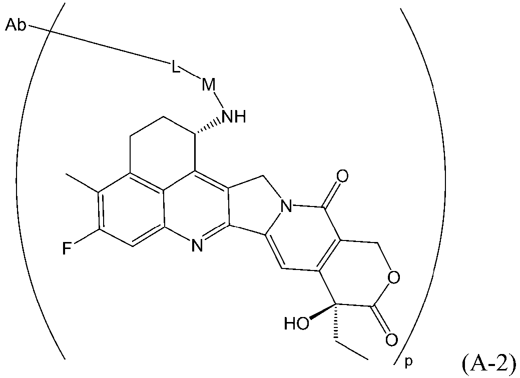
wherein p represents the average number of connections or the number of connections, and p is any integer or decimal from 1 to 10; preferably any integer or decimal from 3 to 9; more preferably an integer or decimal of 7 to 8, such as 7.8 or 7.9;
Ab is the antibody or the antigen-binding fragment thereof as defined in any one of claims 1 to 8;
M is as defined in any one of claims 14 to 20;
L is the linker unit as defined in claim 21 or 22.

24. The antibody-drug conjugate according to any one of claims 14 to 23, wherein the antibody-drug conjugate is selected from the group consisting of the following structural formulas: 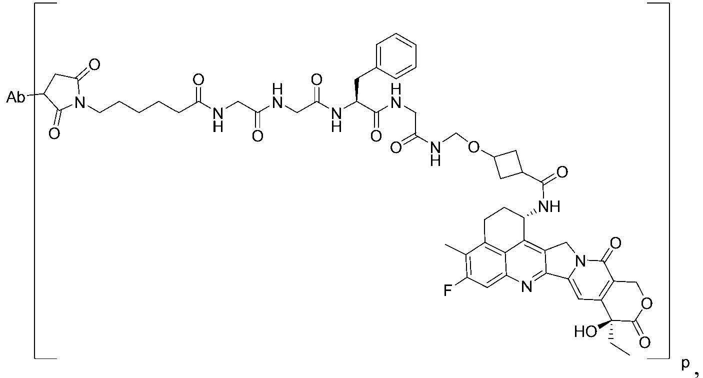 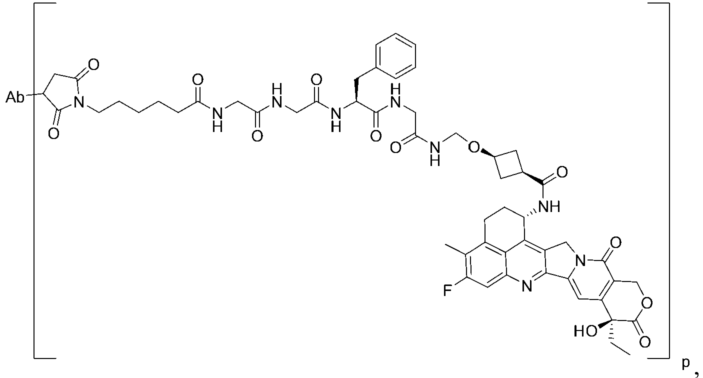 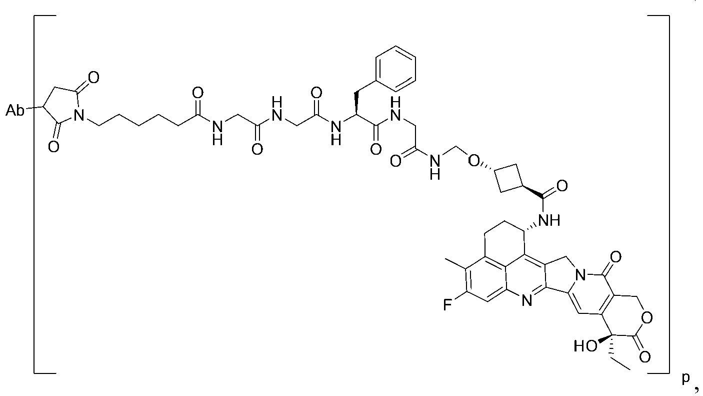 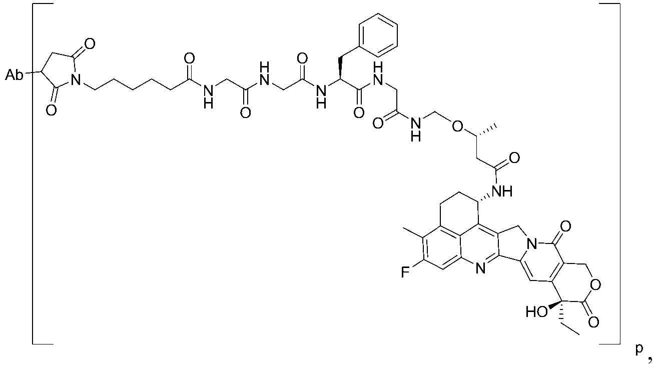 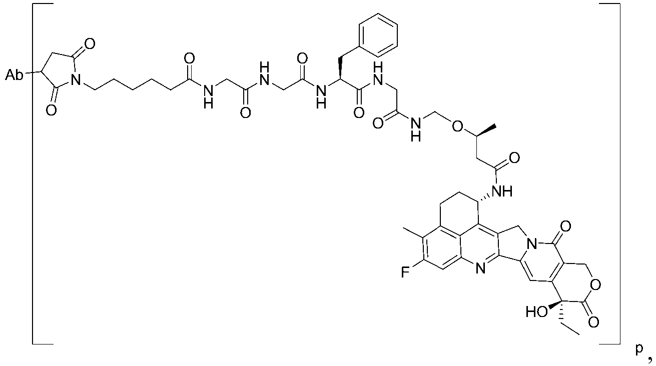 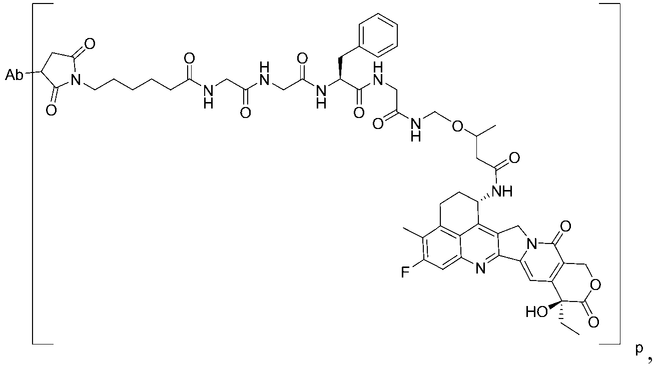 wherein
p represents the average number of connections or the number of connections, and p is any integer or decimal from 1 to 10, preferably any integer or decimal from 3 to 9;
Ab is the antibody or the antigen-binding fragment thereof as defined in any one of claims 1 to 8.

25. The antibody-drug conjugate according to any one of claims 14 to 24, wherein the antibody-drug conjugate is any one of the following conjugates: 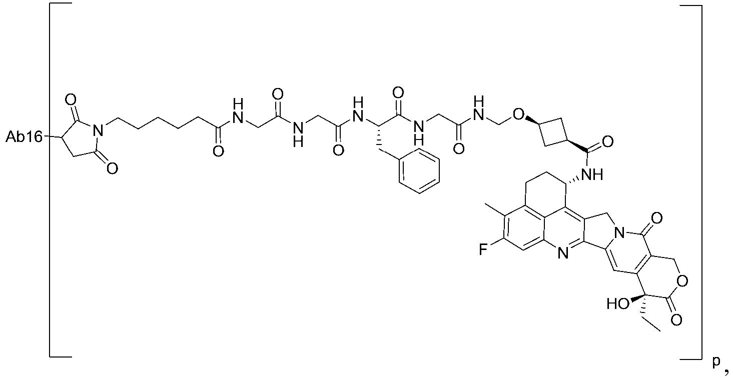 p is any integer or decimal from 1 to 10, preferably any integer or decimal from 3 to 9, more preferably any integer or decimal from 6 to 8; more preferably, p is 7.8; 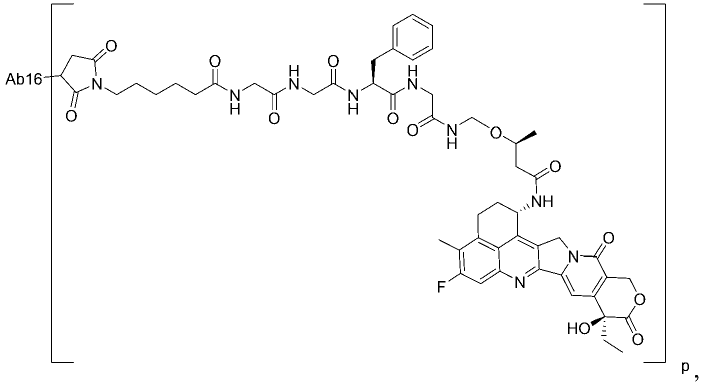 p is any integer or decimal from 1 to 10, preferably any integer or decimal from 3 to 9, more preferably any integer or decimal from 6 to 8; for example, p is 7.9; 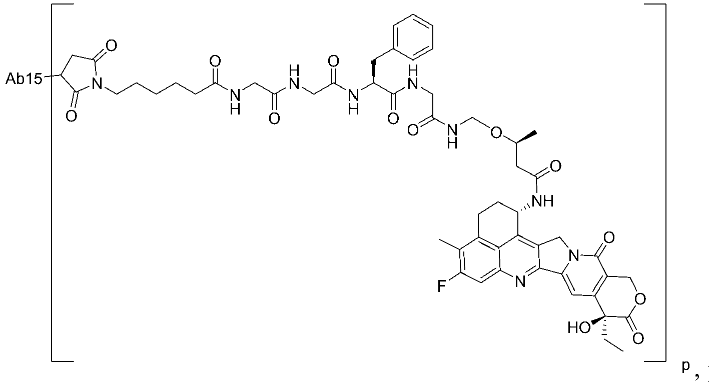 p is any integer or decimal from 1 to 10, preferably any integer or decimal from 3 to 9, more preferably any integer or decimal from 6 to 8; for example, p is 7.8;
Ab16 is an ADAM9-targeting antibody or an antigen-binding fragment thereof, wherein the amino acid sequence of the heavy chain of the Ab16 is shown in SEQ ID NO: 50, and the amino acid sequence of the light chain of the Ab16 is shown in SEQ ID NO: 51;
Ab15 is an ADAM9-targeting antibody or an antigen-binding fragment thereof, wherein the amino acid sequence of the heavy chain of the Ab15 is shown in SEQ ID NO: 52, and the amino acid sequence of the light chain of the Ab15 is shown in SEQ ID NO: 53.

26. The antibody-drug conjugate according to any one of claims 14 to 25, wherein the antibody-drug conjugate is the following conjugate: 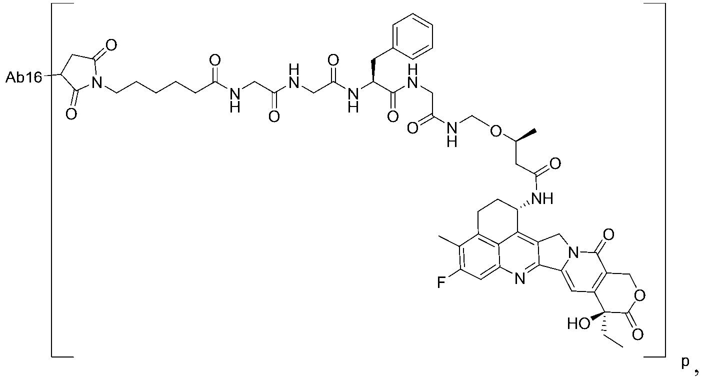 p represents the number of connections, and p is any integer from 1 to 10, preferably any integer from 3 to 9, more preferably any integer from 4 to 8; for example, p is 4, 5, 6, 7, or 8;
Ab16 is an ADAM9-targeting antibody or an antigen-binding fragment thereof, wherein the amino acid sequence of the heavy chain of the Ab16 is shown in SEQ ID NO: 50, and the amino acid sequence of the light chain of the Ab16 is shown in SEQ ID NO: 51.

27. A method for preparing the antibody-drug conjugate as defined in any one of claims 14 to 26, wherein the method comprises reacting the antibody or the antigen-binding fragment thereof as defined in any one of claims 1 to 8 with a compound represented by formula II to obtain the antibody-drug conjugate,
L'-cytotoxic drug II ;
L' forms the linker unit L as defined in any one of claims 14 to 26 with the antibody or the antigen-binding fragment thereof;
the cytotoxic drug is as defined in any one of claims 14 to 26;
preferably, the antibody-drug conjugate satisfies one or more of the following conditions:
(1) the compound represented by formula II is 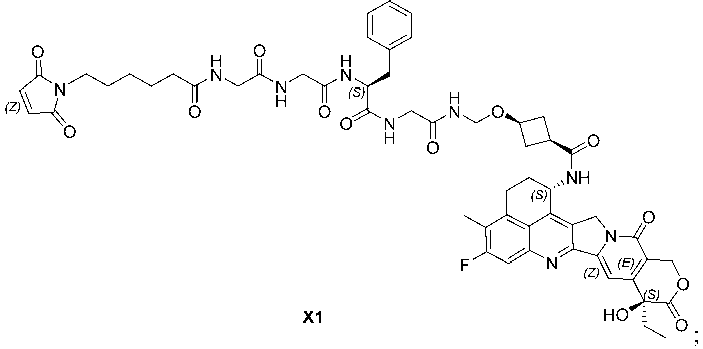 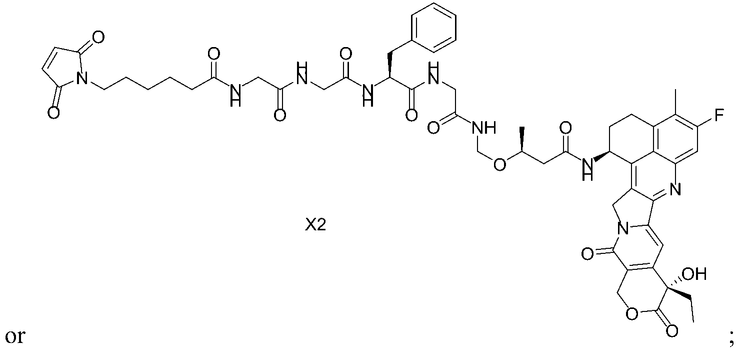
(2) the antibody or the antigen-binding fragment thereof is Ab16 or Ab15;
the amino acid sequence of the heavy chain of the Ab16 is preferably shown in SEQ ID NO: 50, and the amino acid sequence of the light chain of the Ab16 is preferably shown in SEQ ID NO: 51;
the amino acid sequence of the heavy chain of the Ab15 is preferably shown in SEQ ID NO: 52, and the amino acid sequence of the light chain of the Ab15 is preferably shown in SEQ ID NO: 53.

28. A pharmaceutical composition comprising the antibody or the antigen-binding fragment thereof as defined in any one of claims 1 to 8 and/or the antibody-drug conjugate as defined in any one of claims 14 to 26, and a pharmaceutically acceptable carrier.

29. Use of the antibody or the antigen-binding fragment thereof as defined in any one of claims 1 to 8, the antibody-drug conjugate as defined in any one of claims 14 to 26, and/or the pharmaceutical composition as defined in claim 28 in the manufacture of a medicament for diagnosing, preventing, and/or treating cancer with high expression of ADAM9;
preferably, the cancer with high expression of ADAM9 is intestinal cancer or lung adenocarcinoma.

30. Use of the antibody or the antigen-binding fragment thereof as defined in any one of claims 1 to 8, the antibody-drug conjugate as defined in any one of claims 14 to 26, and/or the pharmaceutical composition as defined in claim 28 in the manufacture of a medicament for diagnosing, preventing, and/or treating cancer;
preferably, the cancer is intestinal cancer or lung adenocarcinoma.

31. A method for diagnosing, preventing, and/or treating cancer with high expression of ADAM9, wherein the method comprises administering to a patient in need thereof a therapeutically effective amount of the antibody or the antigen-binding fragment thereof as defined in any one of claims 1 to 8, the antibody-drug conjugate as defined in any one of claims 14 to 26, and/or the pharmaceutical composition as defined in claim 28;
preferably, the cancer with high expression of ADAM9 is intestinal cancer or lung adenocarcinoma.

32. A method for diagnosing, preventing, and/or treating cancer, wherein the method comprises administering to a patient in need thereof a therapeutically effective amount of the antibody or the antigen-binding fragment thereof as defined in any one of claims 1 to 8, the antibody-drug conjugate as defined in any one of claims 14 to 26, and/or the pharmaceutical composition as defined in claim 28;
preferably, the cancer is intestinal cancer or lung adenocarcinoma.

33. The antibody or the antigen-binding fragment thereof as defined in any one of claims 1 to 8, the antibody-drug conjugate as defined in any one of claims 14 to 26, and/or the pharmaceutical composition as defined in claim 28 for use in diagnosing, preventing, and/or treating cancer with high expression of ADAM9;
preferably, the cancer with high expression of ADAM9 is intestinal cancer or lung adenocarcinoma.

34. The antibody or the antigen-binding fragment thereof as defined in any one of claims 1 to 8, the antibody-drug conjugate as defined in any one of claims 14 to 26, and/or the pharmaceutical composition as defined in claim 28 for use in diagnosing, preventing, and/or treating cancer;
preferably, the cancer is intestinal cancer or lung adenocarcinoma.

35. A combination therapy, comprising administering to a patient in need thereof the antibody or the antigen-binding fragment thereof as defined in any one of claims 1 to 8, the antibody-drug conjugate as defined in any one of claims 14 to 26, and/or the pharmaceutical composition as defined in claim 28, and a second therapeutic agent;
preferably, the second therapeutic agent comprises another anti-ADAM9 antibody or an antigen-binding fragment thereof, or an antibody-drug conjugate or pharmaceutical composition comprising the another anti-ADAM9 antibody or the antigen-binding fragment thereof, and/or another medicament for treating cancer with high expression of ADAM9;
more preferably, the cancer with high expression of ADAM9 is intestinal cancer or lung adenocarcinoma.

36. A combination therapy, comprising administering to a patient in need thereof the antibody or the antigen-binding fragment thereof as defined in any one of claims 1 to 8, the antibody-drug conjugate as defined in any one of claims 14 to 26, and/or the pharmaceutical composition as defined in claim 28, and a second therapeutic agent;
preferably, the second therapeutic agent comprises another anti-ADAM9 antibody or an antigen-binding fragment thereof, or an antibody-drug conjugate or pharmaceutical composition comprising the another anti-ADAM9 antibody or the antigen-binding fragment thereof, and/or another medicament for treating cancer;
more preferably, the cancer is intestinal cancer or lung adenocarcinoma.
